# FASCICULE DE BREVET EUROPEEN

(11) **EP 1 539 688 B1**
(45) Date de publication et mention de la délivrance du brevet: **10.08.2016**
(21) Numéro de dépôt: 03795042.5
(22) Date de dépôt: 11.09.2003
(51) Int. Cl.: C07C 231/12, C07C 233/13, C07C 233/18, C07C 233/31, C07C 233/36, C07C 327/36, C07D 207/27, C07D 209/14, C07D 317/36, C07D 209/48, C07C 329/16, C07C 329/18, C07D 339/06, C07F 9/40

(54) **NOUVEAUX COMPOSES COMPRENANT UN GROUPEMENT THIOCARBONYLSULFANYLE UTILES POUR LA SYNTHESE DE COMPOSES ALPHA-PERFLUOROALKYLAMINES PAR VOIE RADICALAIRE**
EINE THIOCARBONYLSULFANYL-GRUPPE BEGRIFTE VERBINDUNGEN VERWENDBAR ZUR RADICALISCHEN HERSTELLUNG VON ALFA-PERFLUORALKYLAMIN-VERBINDUNGEN
NOVEL COMPOUNDS COMPRISING A THIOCARBONYL-SULFANYL GROUP, WHICH CAN BE USED FOR THE RADICAL SYNTHESIS OF ALPHA-PERFLUOROALKYLAMINES

(30) Priorité: 11.09.2002 FR 0211261
(43) Date de publication de la demande: 15.06.2005
(73) Titulaire: Centre National de la Recherche Scientifique, 75794 Paris Cedex 16 (FR)
(72) Inventeur: ZARD, Samir, F-91190 Gif-sur-Yvette (FR); GAGOSZ, Fabien, F-92120 Montrouge (FR); TOURNIER, Lucie, F-75012 Paris (FR)
(74) Mandataire: Colombet, Alain André
(86) Numéro de dépôt international: PCT/FR2003/002697
(87) Numéro de publication internationale: WO 2004/024681

(56) Documents cités:
- Q.-Y. CHEN, ET AL.: "Perfluoro- and polyfluorosulphonic acids. 21. Synthesis of difluoromethyl esters using fluorosulphonyldifluoroacetic acid as a difluororcarbene precursor" JOURNAL OF ORGANIC CHEMISTRY, vol. 54, no. 13, 23 juin 1989 (1989-06-23), pages 3023-3027, XP002274706 AMERICAN CHEMICAL SOCIETY, WASHINGTON, DC, US ISSN: 0022-3263
- F. GAGOSZ, ET AL.: "A direct approach to alpha-trifluoroamines" ORGANIC LETTERS, vol. 5, no. 15, 1 juillet 2003 (2003-07-01), pages 2655-2657, XP002274506 AMERICAN CHEMICAL SOCIETY, WASHINGTON, DC, US ISSN: 1523-7060
- P. DELDUC, ET AL.: "A convenient source of alkyl and acyl radicals" JOURNAL OF THE CHEMICAL SOCIETY, CHEMICAL COMMUNICATIONS, no. 4, 15 février 1988 (1988-02-15), pages 308-310, XP002274695 ROYAL SOCIETY OF CHEMISTRY, LETCHWORTH., GB ISSN: 0022-4936
- J. BOIVIN, ET AL.: "A flexible, convergent approach to piperidines, pyridines, azepines, and related derivatives" TETRAHEDRON LETTERS, vol. 40, no. 19, 7 mai 1999 (1999-05-07), pages 3701-3704, XP004163809 ELSEVIER SCIENCE PUBLISHERS, AMSTERDAM, NL ISSN: 0040-4039
- A. LIARD, ET AL.: "A new synthesis of alpha-tetralones" TETRAHEDRON LETTERS, vol. 38, no. 10, 10 mars 1997 (1997-03-10), pages 1759-1762, XP004054986 ELSEVIER SCIENCE PUBLISHERS, AMSTERDAM, NL ISSN: 0040-4039
- B. QUICLET-SIRE, ET AL.: "An unusual route to deoxysugars by hydrogen atom transfer from cyclohexane. Possible manifestation of polar effects in a radical process" JOURNAL OF THE AMERICAN CHEMICAL SOCIETY, vol. 118, no. 38, 25 septembre 1996 (1996-09-25), pages 9190-9191, XP002244969 AMERICAN CHEMICAL SOCIETY, WASHINGTON, DC, US ISSN: 0002-7863
- A. LIARD, ET AL.: "A practical method for the reductive cleavage of the sulfide bond in xanthates" TETRAHEDRON LETTERS, vol. 37, no. 33, 12 août 1996 (1996-08-12), pages 5877-5880, XP004030563 ELSEVIER SCIENCE PUBLISHERS, AMSTERDAM, NL ISSN: 0040-4039
- T. NAKAI, ET AL.: "Potential synthetic utility of [3,3]-sigmatropic rearrangement of S-allyl dithiocarbamates. New routes to alpha,beta-unsaturated aldehydes" TETRAHEDRON LETTERS, no. 41, octobre 1974 (1974-10), pages 3625-3628, XP002244970 ELSEVIER SCIENCE PUBLISHERS, AMSTERDAM, NL ISSN: 0040-4039
- DATABASE CHEMABS CHEMICAL ABSTRACTS SERVICE, COLUMBUS, OHIO, US; XP002274701 extrait de STN Database accession no. 2003:396832 & WO 03/042153 A (KUMIAI CHEMICAL, ET AL.) 22 mai 2003 (2003-05-22)
- T. HIRAO, ET AL.: "Catalytic pinacol coupling in the presence of acylating agent" SYNLETT, no. 11, novembre 2000 (2000-11), pages 1658-1660, XP002274696 THIEME VERLAG, STUTTGART, DE ISSN: 0936-5214
- C.M. HU, ET AL.: "Synthesis of functionalised organic molecules containing a tetrafluoroethylene fragment by cobaloxime-promoted fluoroalkylation with substituted tetrafluoroethyl bromides" JOURNAL OF THE CHEMICAL SOCIETY, PERKIN TRANSACTIONS 1, no. 3, 7 février 1993 (1993-02-07), pages 331-334, XP002274697 ROYAL SOCIETY OF CHEMISTRY, LETCHWORTH, GB
- J. MÖNIG, ET AL.: "One-electron reduction of halothane (2-bromo-2-chloro-1,1,1-trifluoroethane) by free radicals. Radiation chemical model system for reductive metabolism" JOURNAL OF THE CHEMICAL SOCIETY, PERKIN TRANSACTIONS II, no. 12, 1984, pages 2057-2063, XP002274698 ROYAL SOCIETY OF CHEMISTRY, LETCHWORTH, GB ISSN: 0300-9580
- S.H. BROWN, ET AL.: "Making mercury-photosensitised dehydrodimerisation into an organic synthetic method: vapour pressure selectivity and the behaviour of functionalised substrates" JOURNAL OF THE AMERICAN CHEMICAL SOCIETY, vol. 111, no. 8, 12 avril 1989 (1989-04-12), pages 2935-2946, XP002274699 AMERICAN CHEMICAL SOCIETY, WASHINGTON, DC, US ISSN: 0002-7863
- H. DHIMANE, ET AL.: "Réduction duplicative d'acétals dérivés d'aldhéhydes aromatiques par la combinaison bimétallique aluminium/plomb: préparation de 1,2-diaryl-1,2-diméthoxyéthanes" BULLETIN DE LA SOCIETE CHIMIQUE DE FRANCE, no. 2, mars 1990 (1990-03), pages 283-291, XP002274700 SOCIETE FRANCAISE DE CHIMIE, PARIS, FR ISSN: 0037-8968
- J.L. DAVIDSON, ET AL.: "Reactions of nitrogen-containing heterocyclic compounds with hexafluoro-but-2-yne", JOURNAL OF CHEMICAL RESEARCH (MINIPRINT), no. 5, 1981, pages 1601-1627, XP008075622, Scientific Reviews, Northwood, GB ISSN: 0308-2350

## Description

La présente invention a trait à une nouvelle famille de composés utiles notamment pour la synthèse d'α-perfluoroalkylamines par voie radicalaire.

L'introduction d'atomes de fluor dans une molécule donnée modifie généralement de façon considérable ses propriétés chimiques. Dans le cas d'un composé biologiquement actif, l'introduction d'atomes de fluor peut notamment conduire à une modification du profil pharmacologique de la molécule.

Aussi, à l'heure actuelle, de nombreux efforts sont déployés pour développer des voies d'accès pratiques pour l'obtention de différentes classes de composés fluorés, notamment les α-perfluoroalkylamines et plus particulièrement les α-trifluorométhylamines.

Ainsi, à titre de composés trifluorométhylamines présentant une activité biologique intéressante, on peut par exemple citer les composés suivants :

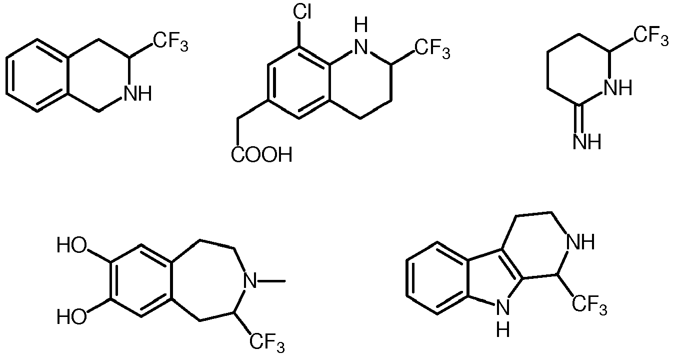

La plupart des procédés de synthèse développés jusqu'à présent pour accéder aux α-trifluorométhylamines consistent à réaliser une amination réductrice des composés trifluorométhyle cétones correspondants. Cependant, ces approches supposent généralement la mise en oeuvre d'un certain nombre d'étapes pour accéder aux composés trifluorométhyle cétones de départ et se traduisent par des rendements globaux relativement peu satisfaisants à cause de la linéarité du processus ; d'autre part, cette approche est souvent incompatible avec un nombre de groupes fonctionnels sensibles à l'action de l'amine ou à celle des réducteurs utilisés, ou bien encore aux réactifs nécessaires pour la synthèse des trifluorométhyle cétones de départ.

Une autre voie d'accès aux composés α-trifluorométhylamines importante consiste à faire réagir divers nucléophiles, de type énolates par exemple, avec des sels d'iminium. A ce sujet, on pourra notamment se reporter aux publications suivantes : *(a)* Blond, G. ; Billard, T. ; Langlois, B. J. Org. Chem. 2001, 66, 4826-4830*. (b)* Takaya ; J. H. ; Kagoshima, H. ; Akiyama, T. Org. Lett. 2000, 2, 1577-1579*. (c)* Dolbier, W. R. ; Xu, Y. J. Org. Chem. 2000, 65, 2134-2137*. (d)* Dolbier, W. R. ; Xu, Y. J. Tetrahedron Lett.. 1998, 39, 9151-9154*. (d)* Fuchigami, T. ; Nakagawa, Y. ; Nonaka, T. J. Org. Chem. 1987, 52, 5489-5491*).* Cependant, la formation d'un sel d'iminium au pied d'un groupe aussi électroattracteur qu'un trifluorométhyle nécessite en général des conditions relativement dures.

De façon inattendue, les inventeurs ont découvert un nouveau procédé permettant d'accéder aux composés α-trifluorométhylamine et de façon plus générale aux composés α-per-/α-poly- fluoroalkyl/fluoroaryl amine de façon directe, flexible et efficace avec des rendements satisfaisants, en partant de dérivés α-per-/α-poly- fluoroalkyl/fluoroaryl amine comprenant une fonction thiocarbonylsulfanyle en α, capables de réagir sur des oléfines.

La demande WO 03/042153 décrit l'ester de l'acide diméthyl-4-bromo-3,4,4-trifluorobutyl carbamodithioïque à titre d'agents de contrôle de la peste.

Delduc et al. (Journal of the Chemical Society, Chemical Communications, 1988, (4), 308-310) décrit des xanthates S-alkyles et S-acyles R-S(C=S)OR' et leurs utilisations comme source de radicaux qui se trouvent piégés par divers alcènes.

### COMPOSES DE FORMULE I

Ainsi, selon un premier aspect, la demande décrit des composés de formule générale (I) : 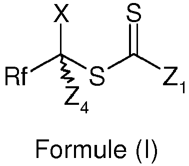 dans laquelle :
- X représente un groupe donneur par effet mésomère ;
- Z₁ représente un groupe choisi parmi :
   (i) les groupes alkyle, acyle, aryle, aralkyle, alcène ou alcyne, les cycles hydrocarbonés ou les hétérocycles,
   (ii) un groupe -OR^{a} ou -SR^{a} dans lequel R^{a} est un groupement choisi parmi:
      - un groupement alkyle, halogénoalkyle, alcényle, alcynyle, acyle, aryle, arylalkyle, arylalcényle, arylalcynyle, ou bien un cycle hydrocarboné ou un hétérocycle, ou bien une chaîne polymère ;
      - un groupement -CR^{b}R^{c}PO(OR^{d})(OR^{e}) dans lequel :
         - R^{b} et R^{c} représentent chacun, indépendamment l'un de l'autre, un atome d'hydrogène, un atome d'halogène, un groupe alkyle, perfluoroalkyle, un cycle hydrocarboné ou un hétérocycle, ou bien encore un groupement -NO₂, -NCO, CN, ou un groupement choisi parmi les groupements de type -R^{f}, -SO₃R^{f}, -OR^{f}, -SR^{f}, -NR^{f}R^{g}, -COOR^{f}, -O₂CR^{f}, -CONR^{f}R^{g}, -NCOR^{f}R^{g}, dans lesquels R^{f} et R^{g} désignent chacun, de façon indépendante, un groupement alkyle, alcényle, alcynyle, cycloalcényle, cycloalcynyle, aryle, éventuellement condensé à un hétérocycle, alkaryle, arylalkyle, hétéroaryle,
         - ou bien R^{b} et R^{c} forment ensemble avec l'atome de carbone auxquels ils sont rattachés un groupement C=O ou C=S ou bien un cycle hydrocarboné ou un hétérocycle ; et
         - R^{d} et R^{e} représentent chacun, indépendamment l'un de l'autre, un radical répondant à une des définitions données ci-dessus pour le groupement R^{f} ;
         - ou bien R^{d} et R^{e} forment ensemble une chaîne hydrocarbonée comportant de 2 à 4 atomes de carbone, éventuellement interrompue par un groupement choisi parmi -O-, -S- et -NR^{h}-; où R^{h} répond à l'une des définitions données ci-dessus pour le groupement R^{f} ;
   (iii) un groupement -NRⁱR^{j}, dans lequel :
      - Rⁱ et R^{j}, représentent indépendamment l'un de l'autre un radical choisi parmi un groupement alkyle, halogénoalkyle, alcényle, alcynyle, acyle, ester, aryle, arylalkyle, arylalcényle, arylalcynyle, ou bien encore un cycle hydrocarboné ou un hétérocycle ; ou
      - Rⁱ et R^{j} forment ensemble une chaîne hydrocarbonée, comportant de 2 à 4 atomes de carbone, éventuellement interrompue par un groupement -O-, -S-, ou -NR^{H}-, où R^{H} répond à l'une des définitions données ci-dessus pour le groupement R^{f}, ladite chaîne hydrocarbonée formant avantageusement un cycle à 5 chaînons avec l'atome d'azote auquel sont rattachés Rⁱ et R^{j},
- Z₄ représente un atome d'hydrogène, un groupement alkyle ou cycloalkyle, et
- Rf représente
   (i) un atome d'halogène, de préférence le fluor ;
   (ii) fluoroalkyle, de préférence perfluoroalkyle ;
   (iii) un radical aryle poly- ou per-halogéné, comportant au moins un, avantageusement deux atomes de fluor, ou
   (iv) un radical choisi parmi R_{A}-CF₂-, R_{A}-CF₂-CF₂-, R_{A}-CF₂-CF(CF₃)-, CF₃-C(R_{A})F- et (CF₃)R_{A}- avec R_{A} choisi parmi un groupe alkyle, acyle, aryle, aralkyle, alcène ou alcyne, les cycles hydrocarbonés ou les hétérocycles ;
et les sels de tels composés.

Avantageusement le groupe X est, ou comporte un atome métalloïde porteur d'un doublet tel que les halogènes, les chalcogènes et les métalloïdes de la colonne de l'azote.

Les « chalcogènes » désignent les atomes métalloïdes choisis parmi l'oxygène, le soufre, le sélénium et le tellure.

Les métalloïdes de la colonne de l'azote englobent notamment l'azote, le phosphore et l'arsenic.

Cet atome métalloïde X est porteur de la liaison avec le reste de la molécule, c'est-à-dire avec le carbone porteur de Rf.

Parmi les composés de formule (I), les composés dans lequels :
- X est choisi parmi -NZ₂Z₃, -OZ₅ ou Hal, où
- Z₂ et Z₃ représentent, indépendamment l'un de l'autre, un atome d'hydrogène, un groupe choisi parmi les alkyles, cycloalkyles, aryles et les groupements électroattracteurs, étant entendu qu'avantageusement l'un au moins des radicaux Z₂ et Z₃ présente un effet électroattracteur vis à vis de la densité électronique de l'atome d'azote auquel ils sont liés,
   - Z₅ représente un atome d'hydrogène, un groupe alkyle, cycloalkyle, aryle, ou un groupement électroattracteur vis-à-vis de l'atome d'oxygène auquel il est lié,
sont l'objet de l'invention.

De préférence, X représente -NZ₂Z₃.

Lorsque l'atome métalloïde est divalent (cas des chalcogènes) ou polyvalent (cas de l'azote) la disponibilité dudit doublet peut-être modulée par les substituants. Il est ainsi préféré qu'un substituant des dits métalloïdes soit électroattracteur. Par exemple, dans le cas où X représente un atome d'azote ou d'oxygène, le groupe électroattracteur permet de diminuer la conjugaison du doublet de l'azote ou de l'oxygène avec la fonction thiocarbonylsulfanyle en α.

Pour plus d'information à ce sujet, on pourra notamment se référer à l'ouvrage du professeur Jerry March intitulé « Advanced Organic Chemistry » (3^{e} édition), édité par John Wiley and Sons.

Un « groupement électroattracteur » au sens de la présente description, désigne un groupe en général au moins aussi électroattracteur que le groupe phényle, qui, dans l'échelle des constantes de Hammett σₚ, correspond à la valeur 0,05 ; rappelons que la valeur de l'hydrogène est par définition zéro et celle du trifluorométhyle est de 0,53. Selon la présente invention, le caractère électroattracteur du ou des substituants dudit métalloïde est assuré par la présence d'un, dans le cas des métalloïdes polyvalents, de préférence un seul, groupe carbonyle directement lié audit métalloïde.

A titre d'exemple de groupements électroattracteurs, on peut citer en particulier les groupes acyles (σₚ ∼ 0,47) lato sensu, y compris aroyle, carboxyle (σₚ ∼ 0,44), alkyloxycarbonyle (σₚ ∼ 0,44), aryloxycarbonyle, aralkyloxycarbonyle, carbamoyle, alkylcarbamoyle, arylcarbamoyle, les groupes cyano- (σₚ ∼ 0,70), sulfonyle, alkysulfonyle (σₚ ∼ 0,73), arylsulfonyle, de préférence acyle, carboxyle, alkyloxycarbonyle, aryloxycarbonyle, aralkyloxycarbonyle, carbamoyle, cyano-, sulfonyle.

A noter que les valeurs sigma p(ara), ((σₚ)) indiquées ci-dessus sont celles rapportées dans l'ouvrage du professeur Jerry March intitulé « Advanced Organic Chemistry » (3e édition), édité par John Wiley and Sons.

Les groupements Z₂ et Z₃ peuvent également être liés pour former avec l'atome d'azote auquel ils sont liés un hétérocycle hydrocarboné, saturé, insaturé ou aromatique, comportant de préférence de 5 à 6 chaînons, éventuellement interrompu par un ou deux atomes d'azote.

A titre d'exemple d'hétérocycle, on peut citer notamment, à titre préféré, les azoles, en particulier les diazoles tels que l'imidazole ou le pyrazole et les triazoles. Lorsque l'hétérocycle est saturé ou partiellement saturé, il peut comprendre des substituants de type oxo (O=) ou thioxo (S=). Comme exemple, on peut citer en particulier la pyrrolidone.

Dans l'ensemble de la présente description, on entend couvrir par le terme de groupement "alkyle" ou « Alk » un radical hydrocarboné saturé, linéaire ou ramifié, pouvant éventuellement inclure un ou plusieurs cycle(s) aliphatique(s) saturé(s). Au sens de l'invention, les groupes alkyles peuvent présenter jusqu'à 25 atomes de carbone, et ils contiennent de préférence de 1 à 12 atomes de carbone, et avantageusement de 1 à 6 atomes de carbone.

Parmi les radicaux alkyle envisageables, on peut notamment citer le radical méthyle, éthyle, propyle, butyle, pentyle, isopropyle, tert-butyle, (cyclo)pentyle, (cyclo)hexyle, octyle, decyle ou dodecyle.

De façon particulière, un groupe alkyle peut également désigner, au sens de la présente description, un groupe cycloalkyle, c'est-à-dire un radical hydrocarboné saturé cyclique, présentant de préférence de 3 à 10 atomes de carbone.

Une « chaîne polymère », au sens de la présente description, peut être issue d'une polymérisation radicalaire ou ionique ou encore d'une polycondensation.

Un groupement "alkoxy" désigne quant à lui, au sens de la présente description, un radical -OAlk, où Alk désigne un groupement alkyle tel que défini ci-dessus.

Par groupement "halogénoalkyle" au sens de la présente description, on entend un radical alkyle tel que défini précédemment et substitué par au moins un atome d'halogène, où le terme "atome d'halogène" désigne ici, comme dans l'ensemble de la description, un atome de fluor, de chlore, de brome ou d'iode, de préférence un atome de fluor ou de chlore, et avantageusement un atome de fluor. Les groupements "halogénoalkyle" de l'invention peuvent ainsi être par exemple des groupements "perfluoroalkyle", c'est à dire, au sens de l'invention, des groupements répondant à la formule CₙF₂ₙ₊₁, où n représente un entier allant de 1 à 20.

Par ailleurs, un groupement "alcényle", au sens où il est employé dans la présente description, désigne un radical hydrocarboné insaturé, linéaire ou ramifié, présentant au moins une double liaison C=C. Les groupes alcényles de l'invention peuvent présenter de 2 à 25 atomes de carbone et comprennent de préférence de 2 à 12 atomes de carbone, et avantageusement de 2 à 6 atomes de carbone.

A titre d'exemples de groupes alcényles, on peut notamment citer l'éthényle, le propényle, le n-butényle, l'i-butényle et l'allyle.

De même, on entend par groupe "alcynyle" un radical hydrocarboné insaturé, linéaire ou ramifié et présentant au moins une triple liaison C≡C. Les groupes alcynyles de l'invention présentent généralement de 2 à 25 atomes de carbone, et ils comprennent de préférence de 2 à 15 atomes de carbone, et avantageusement de 2 à 6 atomes de carbone.

Un groupement "aryle" ou « Ar » désigne quant à lui, au sens de la présente description, un groupe aromatique mono- ou polycyclique possédant généralement de 5 à 20 atomes de carbone, et de préférence de 6 à 10 atomes de carbone. Ainsi, il peut par exemple s'agir d'un groupe phényle, ou encore 1- ou 2- naphtyle. Selon une variante particulière un groupe "aryle" au sens de l'invention peut intégrer un ou plusieurs hétéroatomes tels que le soufre, l'oxygène, ou l'azote. Dans ce cas particulier, le groupe "aryle" désigne un groupement hétéroaromatique mono- ou polycyclique. Les groupes "arylalkyles", "aralcényles" et "aralcynyles" au sens de la présente description sont respectivement des chaînes alkyles, alcényles et alcynyles substituées par un groupement aryle tel que défini ci-dessus.

Un groupement "acyle" désigne quant à lui, au sens de la présente description, un groupement de formule -C(=O)-B, où B désigne un atome d'hydrogène ou une chaîne hydrocarbonée linéaire ou ramifiée, saturée ou insaturée, et comportant de 1 à 25 atomes de carbone, et qui peut notamment être un groupe alkyle, alcényle, alcynyle, le groupe alcényle pouvant notamment être un groupe aryle, tels que définis ci-dessus.

« Aroyle » désigne un groupe aryl-CO-, dans lequel le groupe aryle est tel que décrit dans le présent document. Des exemples types de groupes aroyles comprennent le benzoyle, le 1- et 2-naphtoyle.

Les groupes acyles préférés sont les groupes alkyl-CO- où le groupe alkyle désigne plus préférentiellement un alkyle en C₁-C₆. A titre d'exemples de groupes acyle, on peut citer notamment les groupes formyle, acétyle, propanoyle et pivaloyle.

Lorsque les groupes acyles doivent aussi jouer le rôle de groupe protecteur facilement libérable, les acyles sont avantageusement des groupes esters de l'acide carbonique (-CO-O-B) tels que les groupes terbutyloxycarbonyle et benzyloxycarbonyle.

Par groupement "ester" au sens de la présente description, on entend un groupement -C(=O)-OB, où B désigne une chaîne hydrocarbonée linéaire ou ramifiée, saturée ou insaturée, et comportant de 1 à 25 atomes de carbone, et qui peut notamment être un groupe alkyle, alcényle ou alcynyle tels que définis ci-dessus. Les groupes esters englobent notamment les groupes alkyloxycarbonyle, aryloxycarbonyle et aralkyloxycarbonyle.

« Alkyloxycarbonyle » ou « alkoxycarbonyle » désignent de préfé-rence un groupe alkyl-O-OC-, dans lequel le groupe alkyle est tel que défini dans la présente description. Des exemples de types de groupes alcoxycarbonyle comprennent le méthoxycarbonyle, l'éthoxycarbonyle ou le t-butoxycarbonyle (Boc).

« Aryloxycarbonyle » désigne de préférence un groupe aryl-O-CO-, dans lequel le groupe aryle est tel que défini dans la présente description. Des exemples en sont notamment le phénoxycarbonyle et le naphtoxycarbonyle.

« Aralkyloxycarbonyle » désigne de préférence un groupe aralkyl-O-CO-, dans lequel le groupe aralkyle est tel que défini dans la présente description. A titre d'exemple de groupe aralkoxycarbonyle, on peut citer notamment le benzyloxycarbonyle.

Au sens de la présente description, un radical de type "cycle hydrocarboné" désigne un groupement cyclique saturé, insaturé ou aromatique, notamment de type cycloalkyle, cycloalcényle ou cycloalcynyle, éventuellement substitué, et comportant de 3 à 20 atomes de carbone. Un radical de type "hétérocycle" désigne quant à lui un tel cycle carboné interrompu par au moins un hétéroatome choisi par exemple parmi N, O, S, P et Si, ledit cycle carboné pouvant être saturé ou insaturé.

« Carbamoyle » désigne de préférence un groupe NH₂-CO-.

« Carboxyle » désigne de préférence un groupe HO(O)C- (acide carboxylique).

« Alkylcarbamoyle » désigne de préférence un groupe alkyl-NH-CO, dans lequel le groupe alkyle est tel que défini dans la présente description.

« Sulfonyle » désigne de préférence un groupe -SO₃H.

« Alkylsulfonyle » désigne de préférence un groupe alkyl-SO₂- dans lequel le groupe alkyle est tel que défini dans la présente description.

« Arylsulfonyle » désigne de préférence un groupe aryl-SO₂-, dans lequel le groupe aryle est tel que tel que défini dans la présente description.

Les différents radicaux peuvent éventuellement être interrompus par un ou plusieurs hétéroatomes choisis notamment parmi O, S, N, P et Si, ou par des groupes -(C=O)-, -(C=S)-, -SO₂-, -SO-, ou amines secondaires ou tertiaires, et ils peuvent être substitués par tout type de groupements non susceptible d'interférer avec une réaction d'addition radiacalaire ou de mener à des réactions parasites entre les composés en présence, et notamment par un ou plusieurs groupements identiques ou différents choisis parmi les groupements alkoxycarbonyle ou aryloxycarbonyle (-COOR), carboxy (-COOH), acyloxy (-O₂CR), carbamoyle (-CONR₂), cyano (-CN), alkylcarbonyle, alkylarylcarbonyle, arylcarbonyle, arylalkylcarbonyle, phtalimido, maleïmido, succinimido, amidino, guanidino, hydroxy (-OH), amino (-NR₂) ou (-NH₂), halogène, perfluoroalkyle (CₙF₂ₙ₊₁), allyle, époxy, alkoxy (-OR), thioalkoxy ou thioaryloxy (-SR), sulfones, phosphonates, un groupement silylé, un atome d'halogène, des groupes présentant un caractère hydrophile ou ionique tels que les sels alcalins d'acides carboxyliques, les sels alcalins d'acides sulfoniques ou phosphoniques, les chaînes polyoxyde d'alkylène telles que le polyoxyéthylène POE et le polyoxypropylène POP, les substituants cationiques (sels d'ammonium quaternaires), R représentant un groupe alkyle ou aryle, ou une chaîne polymère, lesdits substituants pouvant éventuellement être interrompus par des hétéroatomes. Il est des compétences de l'homme du métier de choisir la nature des différents groupements et substituants présents dans les composés mis en oeuvre pour éviter toute réaction secondaire indésirable.

Selon une variante de l'invention, Z₁ représente un groupement alkyle ou aryle.

Selon un mode de réalisation particulièrement préféré, Z₁ représente un groupe -OR^{a}, où R^{a} est tel que défini précédemment. Dans ce cas, R^{a} est de préférence un groupe choisi parmi les alkyles, aralkyles ou cycloalkyles. Encore plus préférentiellement, R^{a} représente un groupe alkyle.

Selon une variante avantageuse de l'invention, Z₄ représente un atome d'hydrogène.

Avantageusement, Rf représente un groupe comprenant un chaînon difluorométhylène porteur de la liaison assurant le lien avec le reste de la molécule, notamment un groupe R_{A}-CF₂-.

Rf est de préférence fluoroalkyle, plus préférentiellement perfluoroalkyle, de préférence un radical trifluorométhyle.

Selon un mode de réalisation particulier, Rf est un radical aryle poly- ou perhalogéné comportant au moins un atome de fluor, de préférence deux atomes de fluor.

Selon un mode préférentiel de l'invention, les composés de formule (I) sont des composés de formule (la) dans laquelle X représente un groupement -NZ₂Z₃.

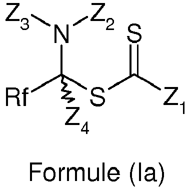

Selon une variante préférée de l'invention, l'un au moins des groupes Z₂ et Z₃ représente un groupement acyle, alkoxycarbonyle ou aralkyloxycarbonyle, de préférence acyle, par exemple un acétyle, alkoxycarbonyle. Sont également avantageux à titre de groupements électroattracteurs Z₂, Z₃, des groupements tels que le t-butoxycarbonyle (Boc), le benzyloxycarbonyle, souvent utilisés pour protéger les amines car faciles à enlever.

Dans ce cadre, il est particulièrement préféré que l'autre groupement Z₂ ou Z₃ représente un atome d'hydrogène ou un reste hydrocarboné avantageusement d'au plus 10 atomes de carbone, de préférence d'au plus 4. De préférence, le reste hydrocarboné est un groupe alkyle ou aryle.

Selon un autre aspect avantageux de l'invention, les composés sont des composés de formule (I) dans laquelle X représente -OZ₅, répondant à la formule (Ib) :

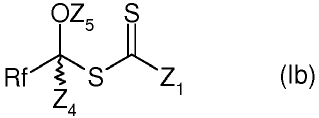

De préférence, Z₅ représente un atome d'hydrogène, un groupement acyle ou aroyle, et plus préférentiellement un groupement acétyle ou benzoyle.

Selon un autre mode préféré, les composés sont des composés de formule (I) dans laquelle X représente Hal, répondant à la formule (Ic) : 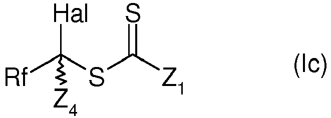
« Hal » désigne au sens de la présente invention un atome d'halogène. Hal représente de préférence un atome de chlore ou de brome, et plus préférentiellement un atome de chlore.
Notamment, lorsque Z₁ représente un groupe alkyle, aryle, -SR^{a}, ou -OR^{a} avec R^{a} choisi parmi les groupes alkyles, arylalkyles, cycloalkyles,
   - Z₄ représente avantageusement un atome d'hydrogène,
   - Rf représente avantageusement une chaîne perfluoroalkyle, de préférence un trifluorométhyle,
   - lorsque X = NZ₂Z₃ :
      - Z₂ représente avantageusement un groupe électroattracteur, tel que les groupements acyle, alkoxycarbonyle ou aralkyloxycarbonyle, de préférence acyle ou alkoxycarbonyle, dont notamment le t-butoxycarbonyle ou le benzyloxycarbonyle,
      - Z₃ représente, de manière avantageuse, soit un groupe électroattracteur identique ou différent de Z₂, tel que les groupements acyle, alkoxycarbonyle ou aralkyloxycarbonyle, de préférence acyle ou alkoxycarbonyle, dont notamment le t-butoxycarbonyle et le benzyloxycarbonyle, soit un atome d'hydrogène, un groupe alkyle, cycloalkyle ou aryle.
   - lorsque X = OZ₅, Z₅ représente avantageusement un groupement acyle, notamment aroyle, et plus préférentiellement un groupement acétyle ou benzoyle.
   - lorsque X = Hal, X représente de préférence un atome de chlore.

A titre illustratif des composés conformes à l'invention, on peut plus particulièrement citer les composés suivants: le dithiocarbonate S-[1-(N-acétylamino)-2,2,2-trifluoroéthyl]-O-éthyle, le diester de O-éthyle et de S-1-benzoylamino-2,2,2-trifluoro-éthyle de l'acide dithiocarbonique, l'ester d'O-éthyle et de S-(1-hydroxy-2,2,2 -trifluoro-éthyle) de l'acide dithio-carbonique, l'ester d'O-éthyle et de S-(1-acétyl-2,2,2-trifluoro-éthyle) de l'acide dithiocarbonique, l'ester de 1-éthoxythiocarbonylsulfanyl-2,2,2-trifluoro-éthyle de l'acide benzoïque, l'ester de O-éthyle et de S-1-chloro-2,2,2-trifluoro-éthyle de l'acide dithiocarbonique.

### PROCEDE DE PREPARATION DES COMPOSES DE FORMULE 1

Les composés utiles selon l'invention peuvent être préparés par l'application ou l'adaptation de procédés connus, par lesquels on entend des procédés utilisés jusque là ou décrits dans la littérature, par exemple ceux décrits par R. C. Laroche dans « Comprehensive Organic Transformations », VCH Publishers, 1989.

Selon un second aspect, l'invention a pour objet un procédé de préparation des composés de formule (Ia).

Ce procédé comprend les étapes successives suivantes :
a) une substitution nucléophile de la fonction alkoxyle de l'hémiacétal Rf-C(OAlk)(OH)Z₄ (A) par addition d'un dérivé Z₂Z₃NH de façon à obtenir un composé de formule Rf-C(NZ₂Z₃)(OH) Z₄, où Alk désigne un groupe alkyle, et où Rf, Z₂, Z₃ ont le sens précité ;
b) une halogénation de la fonction hydroxyle du composé obtenu à l'issu de l'étape (a),
c) une substitution du groupement halogène introduit dans l'étape (b) par un dérivé de thiocarbonylsulfanyle (Z₁-C(=S)-S-) sous forme de sel de métal alcalin, MS-(CS)-Z₁, où Z₁ a le sens précité et où M désigne un métal alcalin.

Ainsi, le procédé de préparation d'un composé de formule (Ia) selon l'invention peut être représenté par le schéma de synthèse général suivant : 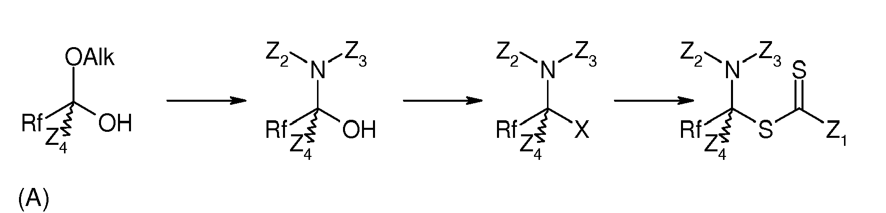 dans lequel Z₁, Z₂, Z₃, Z₄, Rf et M ont les définitions précitées, Alk désignant un groupe alkyle et X un halogène.

Sans vouloir se limiter à une quelconque théorie, ces réactions sont rendues possibles grâce à la présence du groupement Rf, lequel présente avantageusement un effet électroattracteur qui permet, de façon intéressante, de stabiliser les formes hydrate aminal et hémiacétal (A).

Quelle que soit leur structure exacte, les hémiacétals Rf-C(OAlk)(OH)Z₄ (A) et hydrates Rf-C(OH)₂Z₄ utilisables à titre de composés de départ dans l'étape (a) sont facilement accessibles. Si certains de ces composés sont disponibles commercialement, ils peuvent être également préparés selon différents modes d'obtention décrits dans les publications suivantes : *(a)* Gross, U. ; Rüdinger, S. in Organo-Fluorine Compounds ; Baasner. B.; Hagemann, H. ; Tatlow, J. C., Eds ; Houben-Weyl : Methods of Organic Chemistry ; Thieme : Stuttgart, 1999 ; Vol E10a*. (b)* Banks, R. E. ; Smart, B. E. ; Tatlow, J. C. Organofluorine Chemistry : Principles and Commercial Applications ; Plenum Press : New York, 1994*. (c)* Hudlicky, M. ; Pavlath, A. E. Chemistry of Organic Fluorine Compounds II. A Critical Review ; ACS Monograph 187 ; American Chemical Society : Washington DC, 1995*.*

Selon un mode de réalisation particulier, le dérivé amine Z₂Z₃NH mis en oeuvre dans l'étape (a) est un amide, de préférence, il s'agit de l'acétamide.

A titre illustratif et non limitatif des solvant convenant à l'étape (a) selon l'invention, on peut notamment citer le dioxane, le tétrahydrofuranne ou le diméthyle éther d'éthylène glycol (DME).

De préférence, l'halogénation réalisée dans l'étape (b) du procédé consiste en une chloration. Comme agents de chloration permettant de substituer une fonction hydroxyle par un atome de chlore dans l'étape (b), s'avèrent particulièrement intéressants dans le cadre de l'invention le chlorure de thionyle, l'oxychlorure de phosphore, le trichlorure de phosphore, le pentachlorure de phosphore et le phosgène.

L'invention a également pour objet un procédé de préparation de composés de formule (Ib). Les composés de formule (Ib) peuvent être préparés par toute méthode connue de l'homme du métier.

Les composés de formule (Ib) dans laquelle Z₅ est différent de H peuvent être préparés notamment par un procédé comprenant :
a) la mise en oeuvre d'un composé (Ib) dans lequel Z₅ = H et d'un composé Z₅-Y, où Z₅ est tel que défini ci-dessus et Y désigne un groupe partant ; et éventuellement
b) la récupération du produit obtenu.

Par «groupe partant », on entend une liaison Z₅-Y aisément labile, notamment sous l'action d'un nucléophile. Les groupements partants sont bien connus de l'homme du métier. A titre d'exemples de groupes partants, on pourra notamment se référer à l'ouvrage de T. H. Greene et P. G. Wuts, « Protective Groups », in Organic Chemistry, John Wiley and Sons, 1991.

De préférence, Y représente un groupement -O(C=O)Alk, -O(C=O)Ar, -O(SO₂)Alk, -O(SO₂)Ar, ou un atome d'halogène tel que le chlore ou le brome.

A titre de réactifs Z₅Y préférés, on peut citer notamment les anhydrides d'acide monocarboxyliques ou les halogénures d'acyles, les halogénures d'aroyles.

« Anhydride d'acide monocarboxylique » signifie un groupe (alkyl-(C=O))₂O ou (aryl(C=O))₂O dans lesquels alkyle et aryle sont tels que définis dans la présente description. Des exemples préférés en sont notamment l'anhydride acétique, l'anhydride butyrique et l'anhydride benzoïque.

« Halogénure d'acyle » ou « d'aroyle » se réfère à un groupe alkyl-C(=O)-Hal ou aryl-C(=O)-Hal. Des exemples d'halogénures d'acyles, y compris d'aroyles en sont notamment le chlorure d'acétyle et le chlorure de benzyle.

De préférence, l'étape a) de ce procédé comprend la mise en oeuvre d'un acide ou d'une base.

A titre d'exemple d'acide pouvant convenir à la mise en oeuvre de l'étape a), on peut citer notamment les acides minéraux tels que l'acide nitrique, l'acide phosphorique, l'acide sulfurique, l'acide chlorhydrique et des acides sulfoniques tels que l'acide méthanesulfonique, l'acide éthanesulfonique, l'acide benzènesulfonique et l'acide p-toluènesulfonique. De préférence, l'acide est mis en oeuvre dans des proportions catalytiques. Il a généralement pour but d'activer le départ du groupe partant.

Comme exemple de base, on peut notamment citer les pyridines, y compris picolines et quinoléine, les amines, avantageusement tertiaires, telles que la DABCO (diazabicyclooctane), la triéthylamine et la diisopropyléthylamine.

Selon un autre aspect, l'invention concerne également la préparation d'un composé de formule (Ib) dans laquelle Z₅ représente un atome d'hydrogène, comprenant :
a) la mise en oeuvre d'un composé de formule (A) : 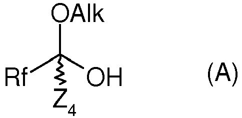 avec un acide et un composé MS-(C=S)-Z₁ où Z₁ est tel que défini ci-dessus et M désigne un métal alcalin et Alk désigne un groupe alkyle ; et le cas échéant
b) la récupération du produit obtenu.

Les acides pouvant être mis en oeuvre dans ce procédé sont tels que définis ci-dessus.

L'invention concerne également un procédé de préparation des composés de formule (Ic) comprenant :
a) la mise en oeuvre d'un composé de formule (Ib) dans laquelle Z₅ = H en présence d'un agent d'halogénation, et éventuellement
b) la récupération du produit obtenu.

A titre d'agent d'halogénation, on peut utiliser des réactifs conventionnels.

Comme agent de chloration, on peut citer notamment le phosgène, les réactifs phosphorés tels que le pentachlorure de phosphore (PCl₅), le trichlorure de phosphore (PCl₃) l'oxychlorure de phosphore (POCl₃), les réactifs soufrés, tels que le chlorure de thionyle (SOCl₂).

Comme exemple d'agent de bromation, on peut citer notamment les dérivés bromés du phosphore.

Les solvants pouvant convenir à la mise en oeuvre des procédés de préparation des composés (Ia), (Ib) et (Ic) selon l'invention peuvent être choisis parmi les cétones, les alcools, les solvants polaires non protiques, les hydrocarbures halogénés et les aromatiques.

Comme exemples de solvants de type cétone, on peut citer notamment l'acétone et la méthyléthylcétone.

Comme exemples de solvants de type alcool, on peut citer notamment le méthanol, l'éthanol, l'isopropanol.

Des exemples de solvants polaires non protiques englobent notamment l'acétonitrile, le N,N-diméthylformamide (DMF) et le diméthylsulfoxyde (DMSO).

A titre d'hydrocarbure halogéné, on peut citer notamment le dichlorométhane et le 1,2-dichloroéthane.

Comme exemple de solvant aromatique, on peut citer notamment le benzène, le toluène et les chlorobenzènes.

A titre représentatif des solvants pouvant convenir à la réalisation de l'étape (c) du procédé de préparation des composés de formule (Ia), on peut plus particulièrement citer l'acétone, l'acétonitrile, l'éthanol, l'isopropanol, le méthanol, la méthyle éthyle cétone, le *N,N*-diméthylformamide (DMF), et le diméthylsulfoxyde (DMSO).

A titre d'exemple de solvant particulièrement approprié au procédé de préparation des composés de formule (Ib), on peut notamment citer l'acétone et le dichlorométhane.

Parmi les dérivés comprenant une fonction thiocarbonylsulfanyle (Z₁-C(=S)-S-) pouvant être mis en oeuvre dans l'étape (c), on peut citer en particulier, les composés xanthates (Z₁=OR^{a}), dithiocarbamates (Z₁=NR^{b}R^{c}), trithiocarbonates (Z₁=SR^{a}). En ce qui concerne les groupements R^{a}, R^{b}, et R^{c}, ils répondent aux définitions soumises précédemment.

De préférence, R^{a}, R^{b} et R^{c} représentent un groupe alkyle, de préférence un alkyle en C₁-C₆.

A titre préférentiel, on peut citer les dérivés xanthate, par exemple l'*O-*éthylxanthate de potassium.

Les composés de formule (Ia), (Ib) et (Ic) peuvent être séparés et purifiés par des techniques de séparation et de purification conventionnelles, par exemple par filtration, concentration, extraction, cristallisation, recristallisation, chromatographie sur colonne ou une combinaison de telles méthodes

Les composés de formule (I), de préférence les composés de formule (Ia), se révèlent particulièrement intéressants dans le cadre de réactions de synthèse organique par voie radicalaire.

En effet, sous une activation thermique, chimique ou photochimique, de préférence chimique ou photochimique, les composés de formule (I) conduisent à des radicaux RfC^{•}(Z₄) (X).

De préférence, il s'agit des composés de formule (Ia) qui conduisent à des radicaux Rf-C^{•}(Z₄)(NZ₂Z₃).

Ces radicaux peuvent alors réagir avec des composés insaturés tels que les oléfines.

Par *activation,* on entend un processus permettant la création d'un radical Rf-C^{•}(Z₄)(X), de préférence Rf-C^{•}(Z₄)(NZ₂Z₃) à partir d'un composé de formule (I). Cette activation peut notamment être induite par les photons d'une source actinique, notamment lumineuse (activation photochimique), par la décomposition thermique d'un initiateur de radicaux libres, par exemple un peroxyde ou un composé diazo (activation chimique), ou par l'autoxydation d'un composé sensible à l'oxygène comme le triéthylborane.

Comme exemple d'initiateur préféré, il convient de citer les peroxydes, de préférence symétriques, et les composés azo. Parmi les peroxydes, on peut citer les peroxydes d'alkyle et notamment de tertioalkyle, les peroxydes d'acyle, notamment d'alcanoyle, de préférence symétriques.

Les peroxydes d'acyle utilisables sont de préférence des peroxydes dont les acyles sont de bas poids moléculaire, c'est-à-dire que leur nombre de carbones est au plus égal à 10, de préférence à 6 lorsqu'ils sont aliphatiques, mais il est préférable d'utiliser des peroxydes d'acyle de nature aromatique comme le peroxyde de benzoyle.

A titre d'exemples d'initiateur de type peroxyde, on peut notamment citer le peroxyde de benzoyle, l'hydroperoxyde de cumène, le peroxyde d'hydrogène, le peroxyde d'acétyle et le peroxyde de lauroyle.

A titre illustratif d'initiateurs de type azo (azobisnitrile), on peut citer notamment le 2,2'-azobis-isobutyronitrile, le 2,2'-azobis-(2-méthyl-propanenitrile), le 2,2'-azobis-(2,4-diméthylpentanenitrile), le 2,2'-azobis-(2,4-diméthyl-4-méthoxyvaléronitrile), le 2',2'-azobis-(2,4-diméthylvaléro-nitrile) et l'hydrochlorure de 2,2'-azobis-(2-amidinopropane).

A ce sujet, on pourra notamment se référer à l'ouvrage « Polymer Handbook », 4^{e} édition, éd. D. Bloch.

### UTILISATION DES COMPOSES DE FORMULE (I)

L'utilisation des composés de formule (I) en synthèse organique radicalaire mettant à profit ce type de processus constitue un autre aspect de l'invention.

Plus précisément, les composés de formule (I), dans laquelle
- X représente un groupe -NZ₂Z₃, -OZ₅ ou un atome d'halogène (Hal) choisi parmi CI, Br et I, où
   - Z₂ et Z₃ représentent, indépendamment l'un de l'autre, un atome d'hydrogène, un groupe choisi parmi les alkyles, cycloalkyles, aryles, et les groupements électroattracteurs, étant entendu que l'un au moins des radicaux Z₂ et Z₃ présente un effet électroattracteur vis-à-vis de la densité électronique de l'atome d'azote auquel ils sont liés,
   - Z₂ et Z₃ peuvent être liés pour former un hétérocycle avec l'atome d'azote,
   - Z₅ représente un atome d'hydrogène, un groupe choisi parmi les alkyles, cycloalkyles, aryles ou les groupements électroattracteurs vis-à-vis de la densité électronique de l'atome d'oxygène auquel il est lié,
- Z₁ représente un groupe choisi parmi :
   (i) les groupes alkyle, acyle, aryle, aralkyle, alcène ou alcyne, les cycles hydrocarbonés ou les hétérocycles,
   (ii) un groupe -OR^{a} ou -SR^{a} dans lequel R^{a} est un groupement choisi parmi :
      - un groupement alkyle, halogénoalkyle, alcényle, alcynyle, acyle, aryle, arylalkyle, arylalcényle, arylalcynyle, ou bien un cycle hydrocarboné ou un hétérocycle, ou bien une chaîne polymère ;
      - un groupement -CR^{b}R^{c}PO(OR^{d})(OR^{e}) dans lequel :
         - R^{b} et R^{c} représentent chacun, indépendamment l'un de l'autre, un atome d'hydrogène, un atome d'halogène, un groupe alkyle, perfluoroalkyle, un cycle hydrocarboné ou un hétérocycle, ou bien encore un groupement -NO₂, -NCO, CN, ou un groupement choisi parmi les groupements de type -R^{f}, -SO₃R^{f}, -OR^{f}, -SR^{f}, -NR^{f}R^{g}, -COOR^{f}, -O₂CR^{f}, -CONR^{f}R^{g}, -NCOR^{f}R^{g}, dans lesquels R^{f} et R^{g} désignent chacuns, de façon indépendante, un groupement alkyle, alcényle, alcynyle, cycloalcényle, cycloalcynyle, aryle, éventuellement condensé à un hétérocycle, alkaryle, arylalkyle, hétéroaryle,
         - ou bien R^{b} et R^{c} forment ensemble avec l'atome de carbone auxquels ils sont rattachés un groupement C=O ou C=S ou bien un cycle hydrocarboné ou un hétérocycle ; et
         - R^{d} et R^{e} représentent chacun, indépendamment l'un de l'autre, un radical répondant à une des définitions données ci-dessus pour le groupement R^{f} ;
         - ou bien R^{d} et R^{e} forment ensemble une chaîne hydrocarbonée comportant de 2 à 4 atomes de carbone, éventuellement interrompue par un groupement choisi parmi -O-, -S- et -NR^{h}-; où R^{h} répond à l'une des définitions données ci-dessus pour le groupement R^{f} ;
   (iii) un groupement -NRⁱR^{j}, dans lequel :
      - Rⁱ et R^{j}, représentent indépendamment l'un de l'autre un radical choisi parmi un groupement alkyle, halogénoalkyle, alcényle, alcynyle, acyle, ester, aryle, arylalkyle, arylalcényle, arylalcynyle, ou bien encore un cycle hydrocarboné ou un hétérocycle ; ou
      - Rⁱ et R^{j} forment ensemble une chaîne hydrocarbonée, comportant de 2 à 4 atomes de carbone, éventuellement interrompue par un groupement -O-, -S-, ou -NR^{H}- où R^{H} répond à l'une des définitions données ci-dessus pour le groupement R^{f},
- Z₄ représente un atome d'hydrogène, un groupement alkyle ou cycloalkyle, et
- Rf représente
   (i) un atome d'halogène, de préférence le fluor ;
   (ii) fluoroalkyle ;
   (iii) un radical aryle poly- ou per-halogéné, ou
   (iv) un radical choisi parmi R_{A}-CF₂, R_{A}-CF₂-CF₂-, R_{A}-CF₂-CF(CF₃)-, CF₃-C(R_{A})F- et (CF₃)R_{A}- avec R_{A} choisi parmi un groupe alkyle, acyle, aryle, aralkyle, alcène ou alcyne, les cycles hydrocarbonés ou les hétérocycles,
   et les sels de tels composés,
   dont notamment les composés de formule (Ia),
   sont particulièrement utiles en synthèse organique radicalaire, à titre de source de radicaux Rf-C^{•}(Z₄)(X), dont notamment de radicaux (Rf-C^{•}(Z₄)(NZ₂Z₃)), activable photochimiquement ou chimiquement.

Dans ce cadre, les composés de formule (I), respectivement (Ia), peuvent être utilisés pour introduire un groupement Rf(Z₄)(X)C-, respectivement Rf(Z₄)(NZ₂Z₃)C-, notamment perfluoroalkylamine, en particulier le radical 2,2,2-trifluoroéthylamine, sur une oléfine.

Les composés de formule (I) sont particulièrement utiles, pour introduire sur une oléfine, un des groupements (1a), (1 b) ou (1 c) : 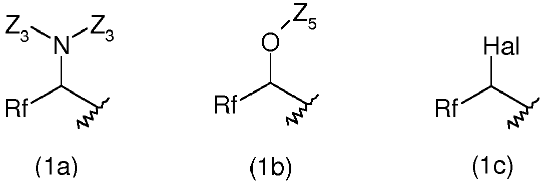 et en particulier un des groupements (1'a), (1'b) ou (1'c) :

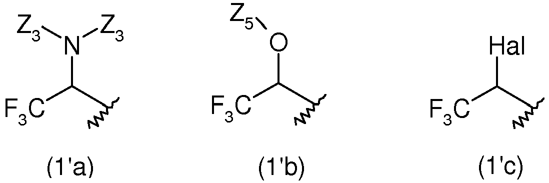

### PROCEDE DE PREPARATION DES COMPOSES DE FORMULE (II)

Ainsi, selon un autre aspect, l'invention a pour objet un procédé de préparation des composés de formule (II) : 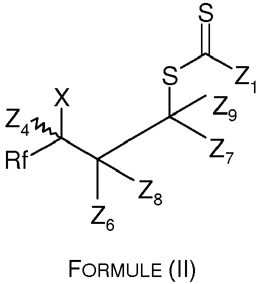 dans laquelle :
- X représente un groupe -NZ₂Z₃, -OZ₅ ou un atome d'halogène (Hal) choisi parmi CI, Br et I, où
   - Z₂ et Z₃ représentent, indépendamment l'un de l'autre, un atome d'hydrogène, un groupe choisi parmi les alkyles, cycloalkyles, aryles, et les groupements électroattracteurs, étant entendu que l'un au moins des radicaux Z₂ et Z₃ présente un effet électroattracteur vis-à-vis de la densité électronique de l'atome d'azote auquel ils sont liés,
   - Z₂ et Z₃ peuvent être liés pour former un hétérocycle avec l'atome d'azote,
   - Z₅ représente un atome d'hydrogène, un groupe choisi parmi les alkyles, cycloalkyles, aryles ou les groupements électroattracteurs vis-à-vis de la densité électronique de l'atome d'oxygène auquel il est lié, ,
   - Rf représente
      (i) un atome d'halogène, de préférence le fluor ;
      (ii) fluoroalkyle, plus préférentiellement un groupe perfluoroalkyle (CₙF₂ₙ₊₁) ;
      (iii) un radical aryle poly- ou per-halogéné, ou
      (iv) un radical choisi parmi R_{A}-CF₂, R_{A}-CF₂-CF₂-, R_{A}-CF₂-CF(CF₃)-, CF₃-C(R_{A})F- et (CF₃)R_{A}- avec R_{A} choisi parmi un groupe alkyle, acyle, aryle, aralkyle, alcène ou alcyne, les cycles hydrocarbonés ou les hétérocycles,
   - Z₆, Z₇, Z₈ et Z₉ représentent indépendamment un atome d'hydrogène, un atome d'halogène, un groupe alkyle, halogénoalkyle, alcényle, alcynyle, acyle, aryle, arylalkyle, arylalcényle, arylalcynyle, ou bien un cycle hydrocarboné ou un hétérocycle, une chaîne polymère, un groupe -(CH₂)ₘ-OR^{k}, -(CH₂)ₘ-CH(OR^{k})(OR^{l}), CH(OR^{k})(OR^{l})-, -(CH₂)ₘ-SR^{k}, -(CH₂)ₘ-SO₃R^{k}, -(CH₂)ₘ-NO₂, -(CH₂)ₘ-CN, -(CH₂)ₘ-R^{k}, -[(CH₂)ₘ-P(O)(OR^{k})(OR^{l})], (CH₂)ₘ-SiR^{k}R^{l}R^{m}, -(CH₂)ₘ-COOR^{k}, -(CH₂)ₘ-NCOR^{k}, -(CH₂)ₘ-NR^{k}R^{l}, dans lesquels :
      - R^{k}, R^{l} et R^{m} désignent chacun de façon indépendante un groupe alkyle, acyle, aryle, alcényle, alcynyle, aralkyle, alkaryle, alkylsulfonyle, arylsulfonyle, un cycle hydrocarboné ou un hétérocycle,
      - ou bien R^{k} et R^{l} forment ensemble avec l'atome auquel ils sont rattachés, un cycle hydrocarboné ou un hétérocycle;
      - m désignant un nombre entier supérieur ou égal à 1, de préférence allant de 1 à 100, et de manière avantageuse de 1 à 20, et mieux encore de 1 à 4,
   ou bien Z₆, Z₇, Z₈ et Z₉ forment deux à deux un ou plusieurs cycle(s) hydrocarboné(s) ou hétérocycle(s), les groupes Z₆, Z₇, Z₈ et Z₉ ne formant pas de cycle étant choisis parmi les radicaux précités,
   lesdits groupes alkyle, halogénoalkyle, alkoxy, halogénoalkyle, alcényle, alcynyle, acyle, ester, cycle carboné, aryle, arylalkyle, alkaryle, aralcényle, aralcynyle, alkylsulfonyle, arylsulfonyle étant tels que définis précédemment,
   ledit procédé comprenant la mise en réaction d'un composé de formule (I), avec au moins une oléfine de formule (III) : 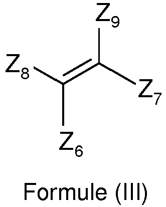 dans laquelle Z₆, Z₇, Z₈ et Z₉ sont tels que définis ci-dessus, en présence d'une source de radicaux libres, dans un solvant organique inerte vis-à-vis des radicaux, et la récupération dudit composé de formule générale (II).

De préférence, X représente -NZ₂Z₃, -OZ₅ ou Hal, plus préférentiellement NZ₂Z₃, Z₂, Z₃, Z₅ et Hal ayant les définitions précitées pour les composés de formule (I).

L'invention concerne également les composés de formule (II) susceptibles d'être obtenus selon ce procédé.

De préférence, les composés de formule (II) sont des composés dans lesquels X = NZ₂Z₃, de formule (IIa) :

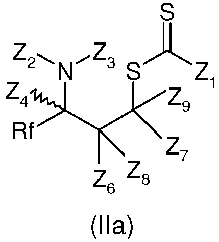

Plus précisément, la préparation des composés de formule (II) consiste en une addition radicalaire d'un composé de formule (I) sur une oléfine de formule (III).

De préférence, l'oléfine de formule (III) est une oléfine monosubstituée dans laquelle l'un des groupes Z₆, Z₇, Z₈ et Z₉ représente un substituant différent de H, les autres étant H.

De façon particulièrement intéressante, il a été montré que, de façon générale, le groupement -S-C(=S)-Z₁ s'additionne sélectivement sur le carbone portant un substituant différent de H de l'oléfine monosubstituée.

Par « sélectivement », on entend un taux de sélectivité de préférence supérieur à 80%, plus préférentiellement supérieur à 90% et mieux encore supérieur à 95%.

Généralement, l'oléfine mise en oeuvre est introduite à raison de 1 à 3 équivalents par rapport au composé de formule (I).

A titre d'exemple de cycles hydrocarbonés ou hétérocycles formés par deux des groupements Z₆, Z₇, Z₈ ou Z₉, on peut citer notamment les cycloalkyles tels que le cyclopropane ou bien des hétérocycles tels que le 1,3-dioxolane, la 1,3-dioxolan-4-one.

De préférence, -(CH₂)ₘ-OR^{k} représente un groupe -(CH₂)ₘ-OCOAlk, -(CH₂)ₘ-O-CO-Ar, -(CH₂)ₘ-CO-Alk ou -(CH₂)ₘ-CO-Ar.

A titre d'exemples préférés, on peut citer notamment les groupes -O-CO-CH₃, -CH₂-O-(CO)-CH₃, -(CH₂)₂-O-CO-CH₃, -(CH₂)₂-CO-CH₃.

De préférence, -(CH₂)ₘ-CH(OR^{k})(OR^{l}) se réfère à un groupe -(CH₂)ₘ-CH(OAlk₁)(OAlk₂) où Alk₁ et Alk₂ sont des groupes alkyles identiques ou différents, tels que définis ci-dessus. A titre d'exemple, on peut citer notamment -CH₂-CH(OEt)₂ et -CH(OEt)₂.

De préférence, -(CH₂)ₘ-P(O)(OR^{k})(OR^{l}) se réfère à un groupe -(CH₂)ₘ-P(O)(OAlk₁)(OAlk₂) où Alk₁ et Alk₂ désignent un groupement alkyle tel que défini ci-dessus. A titre d'exemple, on peut citer notamment -CH₂-P(O)(OEt)₂ et le -CH₂-P(O)(OMe)₂.

De préférence, -(CH₂)ₘ-SiR^{k}R^{l}R^{m} se réfère à un groupe -(CH₂)ₘ-Si(Alk₁) (Alk₂)(Alk₃) où Alk₁, Alk₂, Alk₃ désignent des groupes alkyles identiques ou différents, tels que définis ci-dessus. A titre d'exemple, on peut citer notamment -CH₂-SiMe₃.

De préférence, -(CH₂)ₘ-NR^{k}R^{l} désigne un groupement dans lequel R^{k} et R^{l} représentent indépendamment de préférence un atome d'hydrogène, un groupe alkyle, aryle, aralkyle, hétéroaralkyle, alkylsulfonyle ou forment avec l'atome d'azote auquel ils sont attachés un hétérocycle tel que défini ci-dessus, comprenant de préférence de 4 à 8 atomes de carbone.

Lorsque l'hétérocycle est saturé ou partiellement saturé, il peut comprendre des substituants de type oxo (O=) ou thioxo (S=).

A titre d'exemple de groupement préféré NR^{k}R^{l}, on peut citer notamment les groupes N-arylalkylsulfonamide tel que le N(4-bromo-phényl)méthane sulfonamide.

A titre d'exemple de groupement -NR^{k}R^{l} hétérocyclique, on peut citer notamment les hétérocycles pyrrolidin-2-one, isoindolyle-1,3-dione, phtalimide, aziridinyle, pyrrolidinyle, pyrazolidinyle, imidazolidinyle, piperidyle et piperazinyle.

Selon un mode de réalisation spécifique, l'oléfine de formule (III) est un composé dans lequel les groupements Z₆, Z₇, Z₈ et Z₉ sont choisis parmi les groupements suivants :
- hydrogène,
- -OAc,
- -CH₂-OAc,
- -(CH₂)₂-OAc,
- -CH₂-SiMe₃,
- -CH₂-CN,
- -CH(OEt)₂, -CH₂-CH(OEt)₂,
- -CH₂-P(O)(OEt)₂, -CH₂-P(O)(OMe)₂,
- -(CH₂)₂-COMe,
- pyrrolidin-2-one,
- 2-méthyl-isoindole-1,3-dione,
- (4-bromo-phényl)-diméthylamine,
ou bien Z₆, Z₇, Z₈ et Z₉ forment ensemble deux à deux un cycle 1,3-dioxol-2-one, où le symbole « Ac » représente un groupe acétyle, « Et » représente un groupe éthyle, et « Me » représente un méthyle.

A titre illustratif et non limitatif, l'oléfine de formule (III) mise en oeuvre peut être choisie parmi les composés suivants : acétate de vinyle, hex-5-èn-2-one, acétate d'allyle, vinyltriméthylsilane, but-3-ènenitrile, 3,3-diéthoxypropène, diéthyle allylphosphonate.

La source de radicaux libres désigne, au sens de la présente description, une source capable d'activer les composés de formule (I), et donc d'amorcer la réaction radicalaire. Ceux-ci peuvent, en effet, subir une activation de nature photo-chimique, par exposition à la lumière notamment, ou chimique, par la décomposition d'un peroxyde, par exemple.

De préférence, l'activation résulte de la décomposition d'un amorceur chimique tel qu'un peroxyde ou un composé diazo (décomposition thermique) ou décomposition, par autoxydation avec l'oxygène, d'un composé organométallique tel que le triéthylborane, le diéthylzinc, un trialkylaluminium.

Ainsi, comme exemples de peroxydes particulièrement adaptés à titre de source de radicaux libres dans le procédé de l'invention, on peut notamment citer le diisobutyryle peroxyde, cumyle peroxyneodecanoate, ter-amyle peroxyne-odécanoate, di(2-éthylhexyl) peroxydicarbonate, tert-butyle peroxyneodecanoate, le dibutyle peroxydicarbonate, dicétyle peroxydicarbonate, dimyristyle peroxydicarbonate, tert-butyle peroxyneoheptanoate, tert-amyle peroxypivalate, didecanoyle peroxyde, tert-amyle peroxy-2-éthylhexanoate, tert-butyle peroxyisobutyrate, 1,4-di(tert-butylperoxycarbo)cyclohexane, tert-butyle peroxy-acétate, tert-butyle peroxybenzoate, di-tert-amyle peroxyde, tert-butyle cumyle peroxyde, le peroxyde de bis-tertiobutyle, le peroxyde de dicumyle, le peroxyde de dilauroyle ou le di(4-tert-butylcyclohexyl)peroxydicarbonate.

Quelle que soit sa nature exacte, la source de radicaux libres mise en oeuvre selon le procédé de l'invention est utilisée dans des conditions permettant la production de radicaux libres, ce qui est généralement réalisé par activation thermique, c'est à dire en élevant la température du milieu réactionnel, généralement à une température de l'ordre de l'ambiante (environ 20°C) à 200 °C, de préférence de 40°C à 180°C, avantageusement de 80°C à 160 °C. La production de radicaux libres peut également être réalisée à basse température, généralement à une température inférieure à l'ambiante, de préférence de 10 °C à -78°C, en utilisant des sources de radicaux libres sensibles au processus d'autoxydation avec l'oxygène. De façon générale, le choix de la source de radicaux libres dépend de la température à laquelle on souhaite réaliser la réaction.

La quantité de la source de radicaux libres à introduire dans la milieu dépend de plusieurs paramètres dont notamment de son efficacité, de son mode d'introduction, de la pureté des réactifs, de la concentration du milieu réactionnel, de l'efficacité de l'oléfine comme piège à radicaux. Il est des compétences de l'homme de l'art d'ajuster la quantité de source de radicaux libres à introduire dans le milieu en fonction de ces différents paramètres. Généralement, la source de radicaux libres utilisée est introduite en une quantité telle que la quantité de radicaux libres qu'elle est susceptible de libérer est comprise entre 50 % et 200 % en mole, et de préférence comprise entre 2 % et 30 % en mole, par rapport à la quantité molaire totale de fonctions thiocarbonylsulfanyles portées par les composés de formule (I) présents dans le milieu.

Le solvant mis en oeuvre dans le procédé de préparation des composés de formule (II) est choisi parmi les solvants utilisés classiquement en synthèse radicalaire, comme le 1,2-dichloroéthane, le dichlorométhane, le benzène, le toluène, le trifluorométhylbenzène (trifluorotoluène), le chlorobenzène, l'hexane, le cyclohexane, l'heptane, l'octane, l'acétate d'éthyle, l'alcool tertiobutylique.

La réaction est généralement réalisée sous pression atmosphérique, à la température d'ébullition du solvant choisi.

### COMPOSES DE FORMULE (II)

Les composés de formule (II) obtenus par le procédé défini précédemment sont nouveaux et constituent également, un objet de la présente invention.

Parmi les composés de formule (II), on peut particulièrement citer :
- l'ester de l'acide S-[1-(2-acétylamino-3,3,3-trifluoro-propyl)-4-oxo-pentyl] dithiocarbonique ester *O*-éthylique,
- l'ester de l'acide *S*-[5-(1-acétylamino-2,2,2-trifluoro-éthyl)-2-oxo-[1,3]dioxolan-4-yl] dithiocarbonique ester *O*-éthylique,
- l'ester de l'acide 3-acétylamino-1-éthoxythiocarbonylsulfanyl-4,4,4-trifluoro-butyle acétique,
- l'ester de l'acide *S*-(3-acétylamino-4,4,4-trifluoro-1-triméthyl-silanylméthyl-butyl) dithiocarbonique ester *O*-éthylique,
- l'ester de l'acide *S*-(3-acétylamino-1-cyanométhyl-4,4,4-trifluoro-butyl) dithiocarbonique ester *O*-éthylique,
- l'ester de l'acide *S*-(3-acétylamino-1-diéthoxyméthyl-4,4,4-trifluoro-butyl) dithiocarbonique ester *O*-éthylique,
- l'ester de l'acide *S*-[3-acétylamino-1-(1,3-dioxo-1,3-dihydro-isoindol-2-ylméthyl)-4,4,4-trifluoro-butyl] dithiocarbonique ester *O*-éthylique,
- l'ester de l'acide (4-acétylamino-2-éthoxythiocarbonylsulfanyl-5,5,5-trifluoro-pentyl)-phosphonique diéthylique,
- l'ester de l'acide 4-acétylamino-2-éthoxythiocarbonylsulfanyl-5,5,5-trifluoro-pentyle acétique,
- l'ester de l'acide *S*-[3-acétylamino-4,4,4-trifluoro-1-(2-oxo-pyrrolidin-1 yl)-butyl] dithiocarbonique ester *O*-éthylique,
- l'ester de l'acide *S*-[3-acétylamino-1-{[(4-bromophényl)-méthane-sulfonyl-amino]-méthyl}-4,4,4-trifluoro-butyl) dithiocarbonique ester *O*-éthylique,
- l'ester de l'acide dithiocarbonique S-[1-(2-acétylamino-3,3,3-trifluoro-propyl)-2-phényl-cyclopropane] O-éthyle,
- l'ester de l'acide acétique 4-benzoylamino-2-éthoxythio-carbonyl-sulfanyl-5,5,5-trifluoro-butyle,
- l'ester 4-tertbutyloxycarbamate-2-éthoxythiocarbonyl-sulfanyl-5,5,5-trifluoro-pentylique de l'acide acétique,
- l'ester O-éthylique *S*-(3-tertbutyloxycarbamate-1-diéthoxy-méthyl-4,4,4-trifluoro-butyle de l'acide dithiocarbonique,
- le diester de O-éthyle et de *S*-(3-tertbutyl-oxycarbamate-1-diéthoxy-méthyl-4,4,4-trifluoro-pentyle) de l'acide dithiocarbonique,
- le diester de O-éthyle et de S-[3-acétoxy-1-(1,3-dioxo-1,3-dihydro-isoindol-2-ylméthyl)-4,4,4-trifluoro-butyle] de l'acide dithiocarbonique,
- l'ester de O-éthyle et de S-(3-acétoxy-4,4,4-trifluoro-1-triméthyl-silanylméthyl-butyle) de l'acide dithiocarbonique,
- l'ester de 3-acétoxy-1-éthoxythiocarbonylsulfanyl-4,4,4-trifluoro-butyle de l'acide acétique,
- le diester de O-éthyle et de *S*-(3-acétoxy-1-diéthoxyméthyl-4,4,4-trifluoro-pentyle) de l'acide dithiocarbonique,
- l'ester de O-éthyle et de *S*-(3-acétoxy-1-cyanométhyl-4,4,4-trifluoro)butyle de l'acide dithiocarbonique,
- le diester de O-éthyle et de *S*-1-(2-acétoxy-3,3,3-trifluoro-propyl)-4-oxo-pentyle de l'acide dithiocarbonique,
- l'ester de 4-[4-bromo-phényl)-méthanesulfonyl-amino]-3-éthoxy-carbonylsulfanyl-1-trifluorométhyl-butyle de l'acide acétique,
- le diester de O-éthyle et de *S*-3-chloro-4,4,4-trifluoro-1-triméthylsilanylméthylbutyle de l'acide dithiocarbonique,
- l'ester de 4-chloro-2-éthoxythiocarbonylsulfanyl-5,5,5-trifluoro-pentyle de l'acide acétique,
- l'ester de O-éthyle et de *S*-3-chloro-1-(1,3-dioxo-1,3-dihydro-isoindol-2-ylméthyl)-4,4,4-trifluoro-butyle de l'acide dithiocarbonique,
- le diester de O-éthyle et de *S*-1-(2-chloro-3,3,3-trifluoro-propyl)-4-oxo-pentyle de l'acide dithiocarbonique,
- l'ester de diméthyle et de 4-Chloro-2-éthoxythiocarbonyl-sulfanyl-5,5,5-trifluoro-pentyle de l'acide phosphonique,
- le diester de O-éthyle et de *S*-3-chloro-1-cyanométhyl-4,4,4-trifluoro-butyle de l'acide dithiocarbonique,
- le diester de O-éthyle et de *S*-3-chloro-1-diéthoxyméthyl-4,4,4-trifluoro-pentyle de l'acide dithiocarbonique,
- le diester de O-éthyle et de S-3-chloro-1-(4-chloro-phénoxyméthyl)-4,4,4-trifluoro-butyle de l'acide dithiocarbonique,
- le diester de O-éthyle et de *S*-3-chloro-4,4,4-trifluoro-1-(2-oxo-pyrrolidin-1-yl)-butyle de l'acide dithiocarbonique.

Les composés de formule (II) sont particulièrement intéressants notamment à titre d'intermédiaire de synthèse organique, en particulier en chimie radicalaire. En effet, comme les composés de formule (I), ces composés possèdent de façon surprenante une grande réactivité, notamment en chimie radicalaire, en particulier vis-à-vis des oléfines et tout particulièrement vis-à-vis des oléfines monosubstituées.

Les composés de formule (II) constituent ainsi des intermédiaires clés pour la synthèse organique de composés fonctionnalisés tels que les dérivés α-perfluoroalkylamines, les α-perfluoroalcools ou les halogénures de α-perfluoroalkyles, généralement difficiles d'accès.

### PROCEDES DE TRANSFORMATION DES COMPOSES DE FORMULE (II)

Le procédé de transformation des composés de formule (II) constitue également un objet de la présente invention.

En l'occurrence, le procédé selon l'invention comprend la mise en oeuvre d'un composé de formule (II) dans l'une des réactions suivantes :
- réduction,
- élimination,
- addition sur une oléfine,
- oxydation du carbone portant la fonction thiocarbonylsulfanyle en aldéhyde,
lesdites réactions aboutissant à la transformation ou au déplacement de la fonction thiocarbonylsulfanyle.

Par réaction de « réduction » au sens de la présente description, on entend toute réaction impliquant l'apport d'électrons par un réactif réducteur riche en électrons à la fonction thiocarbonylsulfanyle du composé de formule (II). Cette réaction se traduit par la substitution de la fonction thiocarbonylsulfanyle par un atome d'hydrogène tel que représenté dans la formule générale (IV) : 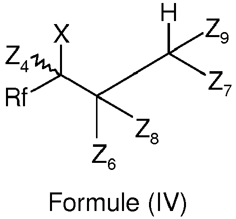 dans laquelle les groupes Rf, X, Z₄, Z₆, Z₇, Z₈ et Z₉, ont le sens précité pour les composés de formule (II).

On préfère particulièrement les composés de formule (IV) dans laquelle X = -NZ₂Z₃ où Z₂ et Z₃ ont le sens précité.

La réaction « d'élimination », au sens où elle est employée dans la présente description, désigne une réaction résultant de deux départs successifs ou concertés de deux entités de nature différente, à savoir d'un proton H⁺ provoqué par l'attaque d'une base, d'une part, et le départ de l'anion -S(CS)Z₁ provoqué par le carbanion voisin (en α). Cette réaction conduit à l'obtention d'un produit comprenant une double liaison entre le carbone portant initialement la fonction thiocarbonylsulfanyle et le carbone en α, répondant à la formule générale (V):

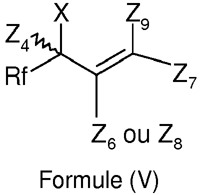

Le procédé de préparation des composés de formule (IV) selon l'invention comprend donc la mise en oeuvre d'un composé de formule (II) dans lequel les groupes X, Z₄, Z₆, Z₇, Z₈ et Z₉ et Rf ont le sens précité, étant entendu que l'un au moins des groupes Z₆ et Z₈ représente un atome d'hydrogène, avec une base.

A titre d'exemple de base, on peut citer notamment le fluorure de tétrabutylammonium.

De préférence, X représente NZ₂Z₃ où Z₂ et Z₃ ont le sens précité.

Une réaction « d'addition radicalaire » d'un composé (II) sur une oléfine Z₁₀Z₁₁(C=C)Z₁₂Z₁₃, en présence d'une source de radicaux libres, répondant aux définitions soumises précédemment, conduit à l'obtention d'un composé répondant à la formule générale (VI) : 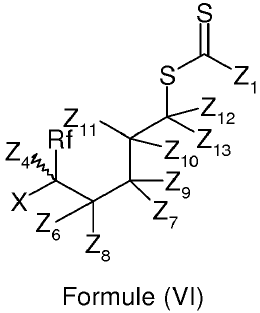 dans laquelle Rf, X, Z₄, Z₆, Z₇, Z₈ et Z₉ sont tels que définis précédemment et Z₁₀, Z₁₁, Z₁₂ et Z₁₃ répondent aux définitions précitées pour Z₆, Z₇, Z₈ et Z₉.

Par « oxydation du carbone portant la fonction thiocarbonylsulfanyle en aldéhyde», on entend toute réaction, en présence d'un acide organique ou minéral, d'un composé de formule (II) dans laquelle Z₇, Z₉ représentent chacun un radical acyloxy- et un atome d'hydrogène, conduisant aux composés de formule générale (VII) : 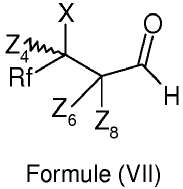 dans laquelle Rf, X, Z₄, Z₆ et Z₈ sont tels que définis précédemment.

De préférence, X représente -NZ₂Z₃ où Z₂ et Z₃ ont le sens précité.

A titre illustratif des composés obtenus par un des procédés de transformation des composés de formule (II) ci-dessus, on peut citer en particulier :
- le *N-*[3-(2-Oxo-pyrrolidin-1-yl)-1-trifluorométhyl-allyl] acétamide,
- le *N-*[4-(1,3-dioxo-1,3-dihydro-isoindol-2-yl)-1-trifluorométhyl-butyl] acétamide,
- l'ester de l'acide dithiocarbonique S-{1-[5-(1-acétylamino-2,2,2-trifluoroéthyl)-2-oxo-[1,3]dioxolan-4-ylméthyl]-2,2-diéthoxy-éthyl} ester *O-*éthylique,
- le *N*-[1-(5-Bromo-1-méthanesulfonyl-2,3-dihydro-1*H-*indol-3-ylméthyl)-2,2,2-trifluoro-éthyl]-acétamide,
- le *N*-(3,3-diméthoxy-1-trifluorométhyl-propyl)-acétamide,
- 4-acétoxy-5,5,5-trifluoro-pent-1-ène,
- l'ester de 1-[5-bromo-1-méthanesulfonyl-2,3-dihydro-1 H-indol-3-ylméthyl)-2,2,2 -trifluoro-éthyle] de l'acide acétique,
- 1-(3-chloro-4,4,4-trifluoro-but-1-ènyl)-pyrrolidin-2-one,
- 2-(4-chloro-5,5,5-trifluoro-pentyl)-isoindole-1,3-dione.

### PROCEDE DE PREPARATION DES COMPOSES DE FORMULE (VIII)

Après activation photochimique ou chimique en l'absence de composés réactifs, les composés de formule (I) peuvent conduire à la formation d'un composé de formule générale (VIII) : 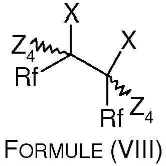
dans laquelle Z₄ est tel que défini ci-dessus,
   - X représente un groupe -NZ₂Z₃, où
   - Z₂ et Z₃ représentent, indépendamment l'un de l'autre, un atome d'hydrogène, un groupe choisi parmi les alkyles, cycloalkyles, aryles, et les groupements électroattracteurs, étant entendu que l'un au moins des radicaux Z₂ et Z₃ présente avantageusement un effet électroattracteur vis-à-vis de la densité électronique de l'atome d'azote auquel ils sont liés,
et Rf représente :
   (i) un atome de fluor ;
   (ii) fluoroalkyle, plus préférentiellement perfluoroalkyle ;
   (iii) un radical aryle poly- ou per-halogéné, ou
   (iv) un radical choisi parmi R_{A}-CF₂, R_{A}-CF₂-CF₂-, R_{A}-CF₂-CF(CF₃)-, CF₃-C(R_{A})F- et (CF₃)R_{A}- avec R_{A} choisi parmi un groupe alkyle, acyle, aryle, aralkyle, alcène ou alcyne, les cycles hydrocarbonés ou les hétérocycles.

De préférence, X représente -NZ₂Z₃ ou -OZ₅, plus préférentiellement -NZ₂Z₃, Z₂, Z₃ et Z₅ étant tels que définis précédemment.

A titre illustratif des composés de formule (VIII) préférés, on peut notamment citer l'ester de 2-benzoyloxy-3,3,3-trifluoro-1-trifluorométhylpropyle de l'acide benzoïque et le N-(2-acétylamino-3,3,3-trifluoro-méthylpropyl)acétamide.

Le procédé de préparation de tels composés de formule (VIII) constitue, selon un autre aspect, un objet de l'invention.

Plus précisément, le procédé selon l'invention comprend une étape de dimérisation radicalaire d'un composé de formule générale (I) et une étape de récupération dudit composé de formule (VIII).

Au sens de la présente invention, on entend par *dimérisation radicalaire,* la formation d'une liaison carbone-carbone entre deux radicaux (X) (Rf)(Z₄)C• identiques.

Cette réaction comprend la mise en oeuvre d'un composé de formule (I), de préférence (Ia) ou (Ib), avec une source de radicaux libres.

Dans ce cadre, il est particulièrement préféré que l'un au moins des groupes Z₂ et Z₃ représente un groupe acyle.

De préférence, Z₅ représente un groupement acyle, y compris aroyle, plus préférentiellement un groupe benzoyle.

En ce qui concerne les conditions de température et de pression ainsi que la nature du solvant et de la source de radicaux libres convenant particulièrement au procédé de dimérisation radicalaire selon l'invention, elles répondent aux définitions précédemment soumises pour le procédé de préparation des composés de formule (II).

En général, le composé (VIII) est obtenu dans ce cadre par dimérisation du radical issu du composé (I) sur lui-même, en présence d'une quantité au moins stoechiométrique d'une source de radicaux libres.

Comme il a été précédemment souligné, un avantage des composés (I) selon l'invention est qu'ils possèdent une grande réactivité en synthèse radicalaire, notamment vis-à-vis des oléfines.

Ces composés s'avèrent particulièrement utiles dans ce cadre pour introduire un groupement (X)(Rf)(Z₄)C-, notamment sur une grande variété d'oléfines, fonctionnalisées ou non.

Un autre avantage est que le procédé de préparation des composés de formule (II) constitue une voie d'accès aux dérivés α-perfluoroalkylamine particulièrement flexible. En effet, la fonction thiocarbonylsulfanyle (Z₁-C(=S)-S-) présente sur le composé (II) peut être aisément réduite, éliminée, ou bien encore donner lieu à plusieurs réactions radicalaires successives. Les produits résultants peuvent ainsi représenter un squelette particulièrement avantageux pour accéder à des structures trifluorométhylées complexes.

De manière générale, les produits de départs et réactifs mis en oeuvre dans les procédés de préparation des composés de formule (I), (II), et (VIII) sont peu coûteux.

De plus, le procédé de préparation des composés de formule (II) selon l'invention requiert avantageusement des conditions expérimentales neutres d'où une compatibilité avec un grand nombre de fonctions chimiques qui peuvent être présentes sur le partenaire oléfinique.

De façon particulièrement intéressante, les composés de formule (II) au sens de l'invention, constituent un accès convergent et rapide à des structures élaborées contenant une grande diversité de fonctions.

Enfin, la présence du groupe thiocarbonylsulfanyle, en particulier d'un groupe xanthate, sur le produit d'arrivée (II) fournit avantageusement une entrée à la chimie extrêmement riche du soufre (via les thiols, les sulfures, les sulfones, les acides sulfoniques, les sulfonamides, les sels de sulfonium, les ylures de soufre, etc.)

Les exemples suivants sont présentés à titre illustratif et non limitatif de la présente invention.

### EXEMPLES

### EXEMPLE DE PROCEDE DE PREPARATION DE COMPOSES DE FORMULE (IA)

### Exemple 1 : Préparation du dithiocarbonate S-[1-(N-acétylamino)-2,2,2-trifluoroéthyl]-O-éthyle

### a) N-(2,2,2-trifluoro-1-hydroxy-éthyl)-acétamide

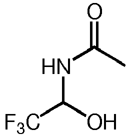

C₄H₆F₃NO₂ M= 157,09 g.mol⁻¹

### Réaction :

Une solution de 2,2,2-trifluoro-1-méthoxy-éthanol (6,50 g, 50 mmol) et d'acétamide (2,95 g, 50 mmol) dans 75 ml de 1,4-dioxane est portée au reflux pendant une heure. Après retour à température ambiante, le mélange réactionnel est concentré sous pression réduite avant d'être purifié.

### Purification :

Chromatographie sur gel de silice (acétate d'éthyle-éther de pétrole 4/6).

### Produit :

Cristallin blanc.

### Rendement :

65%

### PF (°C)

117-119 (acétate d'éthyle-heptane)

### b) N-(1-Chloro-2,2,2-trifluoro-éthyl)-acétamide

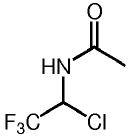

C₄H₅ClF₃NO M= 175,54 g.mol⁻¹

### Réaction :

Une solution de l'alcool a (2,00 g, 12,73 mmol) et de pentachlorure de phosphore (3,05 g, 14,64 mmol) est agitée à température ambiante pendant 30 minutes puis à 70°C pendant 15 minutes. Après évaporation sous pression réduite, le résidu est purifié.

### Purification :

Cristallisation.

### Produit :

Cristallin blanc.

### Rendement :

59%

PF (°C)

78-81 (éther de pétrole)

RMN¹H (δ, ppm) (CDCl₃, 400 MHz)

2,14 (s, 3H, COCH₃); 6,34 (qd, J= 5,3 Hz, 11,1 Hz, 1 H, CF₃CH); 6,47 (d, J= 10,0 Hz, 1 H, NH).

IR (v, cm⁻¹)(CCl₄)

3436 (NH); 3315 (NH); 2995; 1726 (C=O); 1497; 1370; 1345; 1282; 1253; 1221; 1203; 1140.

### c) Ester de l'acide dithiocarbonique S-(1-acétylamino-2,2,2-trifluoro-éthyl) ester O-éthylique

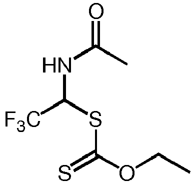

C₇H₁₀F₃NO₂S₂ M= 261,29 g.mol⁻¹

### Réaction :

A une solution du composé chloré b (208 mg, 1,18 mmol) dans 5 ml d'acétone est ajouté le sel d'éthylxanthogénate de potassium (208 mg, 1,29 mmol). Après 15 minutes à température ambiante le mélange réactionnel est concentré sous pression réduite. Le résidu est repris dans l'éther, filtré et concentré à nouveau sous pression réduite.

### Purification :

chromatographie sur gel de silice (acétate d'éthyle-éther de pétrole 2/8).

### Produit :

Cristallin blanc.

### Rendement :

100%

RMN¹H (δ, ppm) (CDCl₃, 400 MHz)

1,44 (t, J= 7,0 Hz, 3H, CH₂CH₃); 2,09 (s, 3H, COCH₃); 4,69 (q, J= 7,0 Hz, 2H, CH₃CH₂); 6,59 (qd, J= 7,6 Hz, 10,0 Hz, 1 H, CF₃CH); 6,89 (d, J= 10,0 Hz, 1 H, NH).

RMN¹³C (δ, ppm) (CDCl₃, 100 MHz)

13,68 (CH₃CH₂); 22,96 (CH₃CO); 57,83 (q, J= 38 Hz, CF₃CH);71,42 (CH₂CH₃); 123,48 (q, J= 280 Hz, CF₃); 169,63 (C=O); 206,87 (C=S).

IR (ν, cm⁻¹)(CCl₄)

3441 (NH); 2983; 1714 (C=O); 1489; 1368; 1331; 1270; 1234; 1196; 1122; 1047.

PF (°C)

84-86 (acétate d'éthyle-heptane)

Masse (IC, NH₃)

262 (MH⁺), 279 (MNH₄⁺).

| Microanalyse | Elément: | Carbone | Hydrogène |
|---|---|---|---|
| | Calculé (%) | 32,18 | 3,86 |
| | Trouvé (%) | 32,57 | 3,91 |

### Exemple 2 : Préparation du diester O-éthyle et de S-1-tert-butyloxycarbonylamino-2,2,2-trifluoro-éthyle de l'acide dithiocarbonique

### a) N-(2,2,2-trifluoro-1-hydroxy-éthyl)tert-butylcarbamate

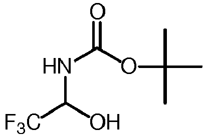

C₇H₁₂F₃NO₃ M= 215,17 g.mol⁻¹

### Réaction :

Une solution de 2,2,2-trifluoro-1-méthoxy-ethanol (1,63 g, 12,5 mmol) et de *tert-*butylcarbamate (1,46 g, 12,5 mmol) dans le 1,4-dioxane (20 mL) est portée au reflux pendant une heure. Après retour à température ambiante, le mélange réactionnel est concentré sous pression réduite avant d'être purifié.

### Purification :

Chromatographie sur gel de silice (acétate d'éthyle-éther de pétrole 4/6).

### Produit :

Cristaux blancs.

### Rendement :

55 %.

Tf

118 °C (acétate d'éthyle-heptane)

### b) N-(1-Chloro-2,2,2-trifluoro-éthyl)- tert-butylcarbamate

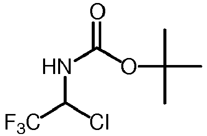

C₇H₁₁ClF₃NO₂ M= 233,04 g.mol⁻¹

### Réaction :

A une solution de l'alcool A8 (250 mg, 1,16 mmol) dans le dichlorométhane (10mL) sont ajoutés du chlorure de thionyle (85 µL, 1,16 mmol) et de la pyridine (95 µL, 1,4 mmol). Après 1h30 au reflux, le mélange réactionnel est refroidi et concentré sous pression réduite.

### Produit :

### Cristaux jaunes.

### Rendement :

61%

Tf

125 °C (acétate d'éthyle-heptane)

### c) Diester de O-éthyle et de S-1-tert-butyloxycarbonyl-amino-2,2,2-trifluoro-éthyle de l'acide dithiocarbonique

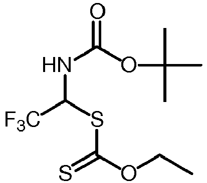

C₁₀H₁₆F₃NO₃S₂ M= 319,05 g.mol⁻¹

### Réaction :

A une solution du composé chloré A10 (165 mg, 0,71 mmol) dans l'acétone (8 mL) est ajouté le sel d'éthylxanthogénate de potassium (230 mg, 1,42 mmol). Après 30 min à température ambiante le mélange réactionnel est concentré sous pression réduite. Le résidu est repris dans l'éther, filtré et concentré à nouveau sous pression réduite.

### Purification :

Chromatographie sur gel de silice (acétate d'éthyle-éther de pétrole 2/98).

### Produit :

Cristaux blancs.

### Rendement :

100%

RMN¹H (δ, ppm) (CDCl₃, 400 MHz)

1,52 (s, 9H, 3xC*H*₃) ; 1,64 (t, *J =* 6,8 Hz, 3H, CH₂C*H*₃); 4,69 (m, 2H, CH₃C*H*₂); 5,21 (d, J= 10,0 Hz, 1 H, N*H*) ; 6,32 (m, 1 H, CF₃C*H*).

RMN¹³C (δ, ppm) (CDCl₃, 100 MHz)

13,6 (*C*H₃CH₂); 28,1 (3x*C*H₃); 57,8 (q, *J=* 38 Hz, CF₃*C*H); 69,5 (C(CH₃)₃) ; 71,4 (*C*H₂CH₃); 120,4 (q, *J=* 278 Hz, *C*F₃); 167,6 (*C*=O); 205,6 (C=S).

IR (v, cm⁻¹) (CCl₄)

3441 (NH); 2983; 2358; 1737 (C=O); 1489; 1368; 1335; 1236; 1193; 1153; 1123; 1048.

Tf

80 °C (acétate d'éthyle-heptane)

Masse (IC, NH3)

320 (MH⁺), 337 (MNH₄⁺).

| Microanalyse : | Elément | Carbone | Hydrogène |
|---|---|---|---|
| | Calculé (%) | 37,61 | 5,05 |
| | Trouvé (%) | 37,42 | 5,23 |

### ADDITIONS RADICALAIRES

### Mode opératoire général :

Une solution de xanthate (n mmol) et d'oléfine (2n mmol) dans le 1,2-dichloroéthane (2n mL) est portée au reflux sous argon pendant quelques minutes avant d'y ajouter du péroxyde de lauroyle (DLP) à raison de 2 à 5 mol% / n toutes les 90 min. Une fois le xanthate de départ entièrement consommé, le milieu réactionnel est ramené à température ambiante puis concentré sous pression réduite avant d'être purifié.

### PROCEDE DE PREPARATION DES COMPOSES FORMULE DE (IIA) ADDITIONS RADICALAIRES

### Exemple 3 :

Ester de l'acide S-[1-(2-acétylamino-3,3,3-trifluoro-propyl)-4-oxo-pentyl] dithiocarbonique ester *O*-éthylique

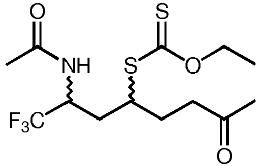

C₁₃H₂₀F₃NO₃S₂ M= 359,43 g.mol⁻¹

### Réaction :

Effectuée selon le mode opératoire général avec 160 mg ( 0,61 mmol) de xanthate de l'exemple 1 et 142 µl (1,23 mmol) de hex-5-en-2-one dans 2 ml de 1,2-dichloroéthane. La réaction est terminée après addition de 5% de DLP et 1 heure 30 minutes de reflux.

### Purification :

Chromatographie sur gel de silice (acétate d'éthyle-éther de pétrole 1/1).

### Produit :

Huile jaune pâle.

### Rendement :

88% (2 diastéréoisomères dans un rapport 1/1)

RMN¹H (δ, ppm) (CDCl₃, 400 MHz)

1,37 (t, J= 7,0 Hz, 3H, CH₂CH₃); 1,39 (t, J= 7,0 Hz, 3H, CH₂CH₃); 1,67-1,86 (m, 2H); 1,90-2,16 (m, 6H); 1,98 (s, 3H, COCH₃); 2,10 (s, 3H, COCH₃); 2,11 (s, 3H, COCH₃); 2,12 (s, 3H, COCH₃); 2,51-2,70 (m, 4H); 3,64 (m, 1 H, CHS); 3,87 (m, 1 H, CHS); 4,58 (q, J= 7,0 Hz, 2H, CH₃CH₂); 4,61 (q, J= 7,0 Hz, 2H, CH₃CH₂); 4,75 (m, 1 H, CF₃CH); 4,81 (m, 1 H, CF₃CH); 6,35 (d, J= 9,4 Hz, 1 H, NH); 6,57 (d, J= 10,0 Hz, 1H, NH).

RMN¹³C (δ, ppm) (CDCl₃, 100 MHz)

13,78 (2 CH₃CH₂); 23,07 (2 CH₃CON); 25,59 (CH₂); ,29,16(CH₂); 29,97 (CH₃CO); 30,06 (CH₃CO); 32,74 (CH₂); 34,19 (CH₂); 40,24 (CH₂); 40,61 (CH₂); 46,51 (CHS); 47,07 (CHS); 48,33 (q, J= 32 Hz, CF₃CH); 48,56 (q, J= 30 Hz, CF₃CH); 70,49 (CH₂CH₃); 70,65 (CH₂CH₃); 124,88 (q, J= 281 Hz, CF₃); 125,10 (q, J= 281 Hz, CF₃); 170,39 (NC=O); 170,77 (NC=O); 207,32 (C=O); 208,15 (C=O); 213,69 (C=S); 214,09 (C=S).

IR (v, cm⁻¹)

3442 (NH); 2983; 1714 (C=O); 1703 (C=O); 1504; 1443; 1369; (CCl₄) 1238; 1185; 1133; 1112; 1050.

Masse (IC, NH₃)

360 (MH⁺), 377 (MNH₄⁺).

| Microanalyse | Elément: | Carbone | Hydrogène |
|---|---|---|---|
| | Calculé (%) | 43,44 | 5,61 |
| | Trouvé (%) | 43,65 | 5,77 |

### Exemple 4 :

### Ester de l'acide S-[5-(1-acétylamino-2,2,2-trifluoro-éthyl)-2-oxo-[1,3]dioxolan-4-yl] dithiocarbonique ester O-éthylique

### Réaction :

Effectuée selon le mode opératoire général avec 200 mg (0,77 mmol) de xanthate de l'exemple 1 et 200 mg (2,31 mmol) de 1,3-dioxol-2-one dans 1,5 ml de 1,2-dichloroéthane. La réaction est terminée après addition de 20% de DLP et 6 heures de reflux.

### Purification :

Chromatographie sur gel de silice (éther-éther de pétrole 4/6 à 6/4).

### Produit :

### Premier diastéréoisomère :

Rf (éther-éther de pétrole 6/4) = 0,30, huile jaune pâle qui cristallise lentement avec le temps.

### Second diastéréoisomère :

Rf (éther-éther de pétrole 6/4) = 0,16, cristallin incolore.

### Rendement :

72% (2 diastéréoisomères dans un rapport 1/1)

### Premier diastéréoisomère

RMN¹H (δ, ppm) (CDCl₃, 400 MHz)

1,46 (t, J= 7,0 Hz, 3H, CH₂CH₃); 2,16 (s, 3H, COCH₃); 4,69 (q, J= 7,0 Hz, 2H, CH₃CH₂); 5,12 (d, J= 5,3 Hz, 1 H, CF3CH(NAc)CH); 5,17 (qd, J= 7,6 Hz, 10,0 Hz, 1 H, CF₃CH); 6,04 (d, J= 5,3 Hz, 1 H, CHS); 7,38 (d, J= 10,0 Hz, 1 H, NH).

RMN¹³C (δ, ppm) (CDCl₃, 100 MHz)

13,56 (CH₃CH₂); 22,61 (CH₃CO); 51,18 (q, J= 29 Hz, CF₃CH); 71,55 (CH₂CH₃); 77,68(CF3CH(NAc)CH); 83,55 (CHS); 123,16 (q, J= 283 Hz, CF₃); 152, 95 (OC=O); 171,92 (NC=O); 205,72 (C=S).

IR (v, cm⁻¹) (CCl₄)

3432 (NH); 3343 (NH); 2959; 1842; 1816 (C=O); 1741 (C=O); 1709; 1500; 1709; 1500; 1371; 1273; 1236; 1192; 1141; 1047.

Masse (IC, NH₃)

348 (MH⁺); 365 (MNH₄⁺).

| Microanalyse | Elément: | Carbone | Hydrogène |
|---|---|---|---|
| | Calculé (%) | 34,58 | 3,48 |
| | Trouvé (%) | 34,28 | 3,47 |

### Second diastéréoisomère

RMN¹H (δ, ppm) (CDCl₃, 400 MHz) 1,48 (t, J= 7,0 Hz, 3H, CH₂CH₃); 2,13 (s, 3H, COCH₃); 4,72 (q, J= 7,0 Hz, 2H, CH₃CH₂); 4,85 (dd, J= 5,3 Hz, 5,3 Hz, 1 H, CF₃CH(NAc)CH); 5,19 (qdd, J= 7,6 Hz, 5,3 Hz, 10,0 Hz, 1 H, CF₃CH); 6,06 (d, J= 10,0 Hz, 1 H, NH); 6,35 (d, J= 5,3 Hz, 1 H, CHS).

RMN¹³C (δ, ppm) (CD₃OD,100 MHz)

13,86 (CH₃CH₂); 22,40 (CH₃CO); 52,86 (q, J= 30 Hz, CF₃CH);72,75 (CH₂CH₃); 77,29 (CF₃CH(NAc)CH); 85,58 (CHS); 125,03 (q, J= 283 Hz, CF₃); 153, 75 (OC=O); 173,66 (NC=O); 208,71 (C=S).

IR (ν, cm⁻¹)(CCl₄)

3258 (NH); 3064 (NH); 2985; 1818 (C=O); 1686; 1549; 1442; 1360; 1298; 1255; 1135; 1076; 1047.

Masse (IC, NH₃)

348 (MH⁺); 365 (MNH₄⁺).

| Microanalyse | Elément: | Carbone | Hydrogène |
|---|---|---|---|
| | Calculé (%) | 34,58 | 3,48 |
| | Trouvé (%) | 34,81 | 3,44 |

### Exemple 5 :

### Ester de l'acide 3-acétylamino-1-éthoxythiocarbonylsulfanyl-4,4,4-trifluoro-butyl acétique

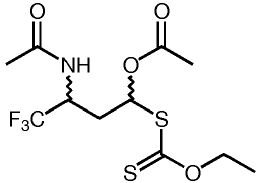

C₁₁H₁₆F₃NO₄S₂ M= 347,38 g.mol⁻¹

### Réaction :

Effectuée selon le mode opératoire général avec 200 mg (0,77 mmol) de xanthate de l'exemple 1 et 85 µL (0,92 mmol) d'acétate de vinyl dans 1,5 ml de

1,2-dichloroéthane. La réaction est terminée après addition de 2,5% de DLP et 1 heures et 30 minutes de reflux.

### Purification :

Chromatographie sur gel de silice (acétate d'éthyle-éther de pétrole 2/8 à 3/7).

### Produit :

Huile épaisse jaune pâle qui cristallise avec le temps

### Rendement :

95% (2 diastéréoisomères dans un rapport 4/6)

RMN¹H (δ, ppm) (CDCl₃, 400 MHz)

1,38 (t, J= 7,6 Hz, 3H, CH₂CH₃); 1,39 (t, J= 7,0 Hz, 3H, CH₂CH₃); 2,03 (s, 6H, COCH₃); 2,06 (s, 3H, COCH₃); 2,07 (s, 3H, COCH₃); 2,12-2,22 (m, 2H, CF₃CH(NAc)CH₂); 2,38-2,51 (m, 2H, CF₃CH(NAc)CH₂); 4,60 (q, J= 7,0 Hz, 2H, CH₃CH₂); 4,61 (q, J= 7,6 Hz, 2H, CH₃CH₂); 4,70-4,83 (m, 2H, CF₃CH); 6,53 (dd, J= 10,0 Hz, 2,9 Hz, 1 H, CHS); 6,62 (d, J= 9,4 Hz, 1 H, NH); 6,64 (dd, J= 8,2 Hz, 4,7 Hz, 1 H, CHS); 6,78 (d, J= 10,0 Hz, 1 H, NH).

RMN¹³C (δ, ppm) (CDCl₃, 100 MHz)

13,60 (CH₃CH₂); 20,66 (CH₃CO); 20,77 (CH₃CO); 22,80 (CH₃CO); 22,96 (CH₃CO); 32,66 (CH₂CHS); 33,33 (CH₂CHS); 47,36 (q, J= 32 Hz, CF₃CH); 47,65 (q, J= 32 Hz, CF₃CH); 70,45 (CH₂CH₃); 70,73 (CH₂CH₃); 76,00 (CHS); 78,30 (CHS); 124,63 (q, J= 281 Hz, CF₃); 124,71 (q, J= 281 Hz, CF₃); 168,95 (C=O); 169,67 (C=O); 170,48 (C=O); 170,72 (C=O); 209,33 (C=S); 209,86 (C=S).

IR (v, cm⁻¹)(CCl₄)

3429 (NH); 2982; 1767 (C=O); 1706 (C=O); 1503; 1369; 1235; 1188; 1137; 1049.

Masse (IC, NH₃)

288 (M-AcOH+H⁺); 348 (MH⁺); 365 (MNH₄⁺).

| Microanalyse | Elément: | Carbone | Hydrogène |
|---|---|---|---|
| | Calculé (%) | 38,03 | 4,64 |
| | Trouvé (%) | 37,79 | 4,51 |

### Exemple 6 :

Ester de l'acide S-(3-acétylamino-4,4,4-trifluoro-1-triméthylsilanylméthyl-butyl) dithiocarbonique ester *0*-éthylique

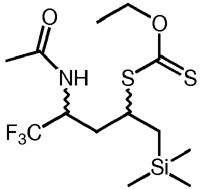

C₁₃H₂₄F₃NO₂S₂Si M= 375,55 g.mol⁻¹

### Réaction :

Effectuée selon le mode opératoire général avec 200 mg ( 0,77 mmol) de xanthate de l'exemple 1 et 364 µl (2,29 mmol) d'allyl-trimethyl-silane dans 1,5 ml de 1,2-dichloroéthane. La réaction est terminée après addition de 5% de DLP et 1 heure de reflux.

### Purification :

Chromatographie sur gel de silice (acétate d'éthyle-éther de pétrole 1/9 à 2/8).

### Produit :

### Premier diastéréoisomère :

Rf (acétate d'éthyle-éther de pétrole 1/9) = 0,38, cristallin incolore.

### Second diastéréoisomère :

Rf (acétate d'éthyle-éther de pétrole 1/9) = 0,19, cristallin incolore.

### Rendement :

95% (2 diastéréoisomères dans un rapport 40/60)

### Premier diastéréoisomére (majoritaire)

### RMN¹H (δ, ppm) (CDCl₃, 400 MHz)

0,03 (s, 9H, Si(CH₃)₃); 0,85-1,16 (m, 2H, CH₂Si(CH₃)₃); 1,42 (t, J= 7,0 Hz, 3H, CH₂CH₃); 1,93-2,16 (m, 2H, CF3CH(NAc)CH₂); 2,11 (s, 3H, COCH₃); 3,64 (m, 1 H, CHS); 4,63 (q, J= 7,0 Hz, 2H, CH₃CH₂); 4,71 (m, 1 H, CF₃CH); 6,00 (d, J= 9,4 Hz, 1H, NH).

RMN¹³C (δ, ppm) (CDCl₃, 100 MHz)

-0,82 (Si(CH₃)₃); 13,87 (CH₃CH₂); 19,48 (CH₂); 23,12 (CH₃CO); 37,37 (CH₂); 43,79 (CHS); 48,44 (q, J= 32 Hz, CF₃CH); 70,29 (CH₂CH₃); 124,99 (q, J= 281 Hz, CF₃); 170,34 (NC=O); 215,11 (C=S).

IR (v, cm⁻¹) (CCl₄)

3432 (NH); 2956; 1701 (C=O); 1507; 1250; 1218; 1184; 1130; 1112; 1051.

Masse (IC, NH₃)

254 (M-HSCSOEt+H+); 376 (MH⁺);393 (MNH₄⁺).

| Microanalyse | Elément: | Carbone | Hydrogène |
|---|---|---|---|
| | Calculé (%) | 41,58 | 6,44 |
| | Trouvé (%) | 41,48 | 6,41 |

### Second diastéréoisomère (minoritaire)

RMN¹H (δ, ppm) (CDCl₃, 400 MHz)

0,07 (s, 9H, Si(CH₃)₃); 1,04-1,19 (m, 2H, CH₂Si(CH₃)₃); 1,40 (t, J= 7,0 Hz, 3H, CH₂CH₃); 1,94-2,21 (m, 2H, CF₃CH(NAc)CH₂); 2,03 (s, 3H, COCH₃); 4,02 (m, 1 H, CHS); 4,63 (q, J= 7,0 Hz, 2H, CH₃CH₂); 4,84 (m, 1 H, CF₃CH); 6,03 (d, J= 10,0 Hz, 1 H, NH).

RMN¹³C (δ, ppm) (CDCl₃, 100 MHz)

-0,68 (Si(CH₃)3); 13,86 (CH₃CH₂); 23,18 (CH₂); 23,26 (CH₃CO); 35,07 (CH₂); 44,98 (CHS); 48,50 (q, J= 28 Hz, CF₃CH); 70,11 (CH₂CH₃); 125,13 (q, J= 281 Hz, CF₃); 170,09 (NC=O); 213,53 (C=S).

IR (v, cm⁻¹) (CCl₄)

3441 (NH); 2955; 1704 (C=O); 1441; 1367; 1251; 1217; 1183; 1129; 1112; 1048.

Masse (IC, NH₃)

254 (M-HSCSOEt+H⁺); 376 (MH⁺); 393 (MNH₄⁺).

| Microanalyse | Elément: | Carbone | Hydrogène |
|---|---|---|---|
| | Calculé (%) | 41,58 | 6,44 |
| | Trouvé (%) | 41,49 | 6,45 |

### Exemple 7 :

Ester de l'acide dithiocarbonique *S*-(3-acétylamino-1-cyanométhyl-4,4,4-trifluorobutyl) ester *O*-éthylique

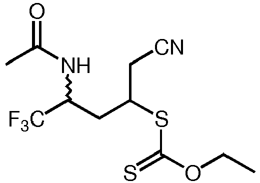

C₁₁H₁₅F₃N₂O₂S₂ M= 328,38 g.mol⁻¹

### Réaction :

Effectuée selon le mode opératoire général avec 200 mg ( 0,77 mmol) de xanthate de l'exemple 1 et 184 µL (2,29 mmol) de but-3-enenitrile dans 1,5 ml de 1,2-dichloroéthane. La réaction est terminée après addition de 15% de DLP et 4 heures et 30 minutes de reflux.

### Purification :

Chromatographie sur gel de silice (acétate d'éthyle-éther de pétrole 3/7).

### Produit :

Huile épaisse jaune pâle.

### Rendement :

87% (2 diastéréoisomères dans un rapport 4/6).

RMN¹H (δ, ppm) (CDCl₃, 400 MHz)

1,39 (t, J= 7,0 Hz, 3H, CH₂CH₃); 1,40 (t, J= 7,0 Hz, 3H, CH₂CH₃); 1,99 (s, 3H, COCH₃); 2,06 (s, 3H, COCH₃); 2,02-2,31 (m, 4H, CF₃CH(NAc)CH₂); 2,82-3,02 (m, 4H, CH₂CN); 3,92 (m, 1 H, CHS); 4,01 (m, 1 H, CHS); 4,61 (q, J= 7,0 Hz, 2H, CH₃CH₂); 4,63 (q, J= 7,0 Hz, 2H, CH₃CH₂); 4,73 (m, 1 H, CF₃CH); 4,82 (m, 1 H, CF₃CH); 6,90 (d, J= 10,0 Hz, 1 H, NH); 7,01 (d, J= 9,4 Hz, 1 H, NH).

RMN¹³C (δ, ppm) (CDCl₃, 100 MHz)

13,66 (CH₃CH₂); 22,28 (CH₂); 22,79 (CH₃CO); 24,21 (CH₂); 30,40 (CH₂); 31,39 (CH₂); 42,48 (CHS); 43,11 (CHS); 48,20 (q, J= 30 Hz, CF₃CH); 48,27 (q, J= 30 Hz, CF₃CH); 70,98 (CH₂CH₃); 71,14 (CH₂CH₃); 116,53 (CN); 116,90 (CN); 124,52 (q, J= 281 Hz, CF₃); 124,71 (q, J= 281 Hz, CF₃); 170,93 (C=O); 171,24 (C=O); 211,24 (C=S); 211,34 (C=S).

IR (v, cm⁻¹)(CCl₄)

3438 (NH); 2984; 1701 (C=O); 1505; 1442; 1369; 1237; 1191; 1139; 1112; 1049.

Masse (IC, NH₃)

329 (MH⁺); 346 (MNH₄⁺).

| Microanalyse | Elément: | Carbone | Hydrogène |
|---|---|---|---|
| | Calculé (%) | 40,23 | 4,60 |
| | Trouvé (%) | 40,39 | 4,53 |

### Exemple 8 :

### Ester de l'acide S-(3-acétylamino-1-diéthoxyméthyl-4,4,4-trifluoro-butyl) dithiocarbonique ester O-éthylique

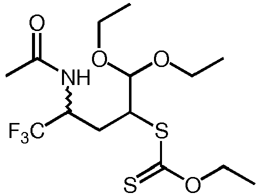

C₁₄H₂₄F₃NO₄S₂ M= 391,47 g.mol⁻¹

### Réaction :

Effectuée selon le mode opératoire général avec 200 mg (0,77 mmol) de xanthate de l'exemple 1 et 351 µL (2,29 mmol) de 3,3-diéthoxy-propène dans 1,5 ml de 1,2-dichloroéthane. La réaction est terminée après addition de 5% de DLP et 1 heure 30 minutes de reflux.

### Purification :

Chromatographie sur gel de silice (acétate d'éthyle-éther de pétrole 3/7).

### Produit :

Huile épaisse jaune pâle.

### Rendement :

95% (2 diastéréoisomères dans un rapport 1/1).

RMN¹H (δ, ppm) (CDCl₃, 400 MHz)

1,16 (t, J= 7,0 Hz, 3H, CH₂CH₃); 1,17 (t, J= 7,0 Hz, 3H, CH₂CH₃); 1,20 (t, J= 7,0 Hz, 3H, CH₂CH₃); 1,21 (t, J= 7,0 Hz, 3H, CH₂CH₃); 1,38 (t, J= 7,0 Hz, 6H, CH₂CH₃); 1,80-2,19 (m, 3H, CF₃CH(NAc)CH₂); 1,98 (s, 3H, COCH₃); 2,05 (s, 3H, COCH₃); 2,49 (m, 1 H, CF₃CH(NAc)CH₂); 3,43-3,76 (m, 8H, CH₃CH₂); 3,95-4,05 (m, 2H, CHS); 4,52 (d, J= 3,0 Hz, 1 H, CH(OEt)₂); 4,59 (d, J= 3,0 Hz, 1 H, CH(OEt)₂); 4,61 (q, J= 7,0 Hz, 4H, CH₃CH₂); 4,73-4,91 (m, 2H, CF₃CH); 6,19 (d, J= 9,4 Hz, 1 H, NH); 6,31 (d, J= 9,4 Hz, 1 H, NH).

RMN¹³C (δ, ppm) (CDCl₃, 100 MHz)

13,78 (2 x CH₃CH₂); 15,08 (2 x CH₃CH₂); 15,15 (CH₃CH₂); 15,34 (CH₃CH₂); 23,04 (CH₃CO); 23,13 (CH₃CO); 25,80 (CH₂CHS); 28,46 (CH₂CHS); 48,42 (q, J= 30 Hz, CF₃CH); 49,08 (q, J= 30 Hz, CF₃CH); 49,88 (CHS); 50,44 (CHS); 64,09 (CH₂CH₃); 64,31 (CH₂CH₃); 64,76 (CH₂CH₃); 65,54 (CH₂CH₃); 70,54 (CH₂CH₃); 70,63 (CH₂CH₃);102,75 (CH(OEt)₂); 103,95 (CH(OEt)₂); 124,94 (q, J= 281 Hz, CF₃); 125,17 (q, J= 283 Hz, CF₃); 170,14 (C=O); 171,32 (C=O); 213,91 (C=S); 214,79 (C=S).

IR (v, cm⁻¹) (CCl₄)

3443 (NH); 2979; 2930; 2875; 1741 (C=O); 1703 (C=O); 1508; 1443; 1370; 1341; 1284; 1218; 1185; 1135; 1112; 1054.

Masse (IC, NH₃)

346 (M-EtOH+H⁺).

### Exemple 9 :

### Ester de l'acide S-[3-acétylamino-1-(1,3-dioxo-1,3-dihydro-isoindol-2-ylméthyl)-4,4,4-trifluoro-butyl] dithiocarbonique ester O-éthylique

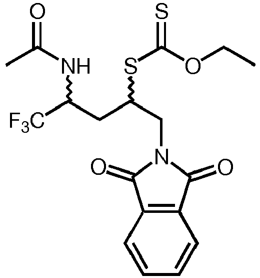

C₁₈H₁₉F₃N₂O₄S₂ M= 448,48 g.mol⁻¹

### Réaction :

Effectuée selon le mode opératoire général avec 200 mg ( 0,77 mmol) de xanthate de l'exemple 1 et 286 mg (1,53 mmol) d'allyl phtalimide dans 1,5 ml de 1,2-dichloroéthane. La réaction est terminée après addition de 10% de DLP et 3 heures de reflux.

### Purification :

Chromatographie sur gel de silice (acétate d'éthyle-éther de pétrole 4/6).

### Produit :

Cristallin incolore.

### Rendement :

77% (2 diastéréoisomères dans un rapport 6/4)

RMN¹H (δ, ppm) (CDCl₃, 400 MHz)

1,33 (t, J= 7,0 Hz, 3H, CH₂CH₃); 1,37 (t, J= 7,0 Hz, 3H, CH₂CH₃); 2,02-2,24 (m, 4H, CF₃CH(NAc)CH₂); 2,02 (s, 3H, COCH₃); 2,19 (s, 3H, COCH₃); 3,93-4,06 (m, 4H, CH₂N); 4,16-4,23 (m, 1 H, CHS); 4,23-4,30 (m, 1 H, CHS); 4,51 (q, J= 7,0 Hz, 2H, CH₃CH₂); 4,57 (q, J= 7,0 Hz, 2H, CH₃CH₂); 4,90-5,11 (m, 2H, CF₃CH); 6,44 (d, J= 10,0 Hz, 1 H, NH); 6,53 (d, J= 10,0 Hz, 1 H, NH); 7,70-7,73 (m, 4H, H_{Ar}.); 7,79-7,84 (m, 4H, H_{Ar.}).

RMN¹³C (δ, ppm) (CDCl₃, 100 MHz)

13,62 (CH₃CH₂); 13,71 (CH₃CH₂); 23,13 (CH₃CO); 23,19 (CH₃CO); 23,96 (CF₃CH(NAc)CH₂); 30,42 (CF₃CH(NAc)CH₂); 39,03 (CH₂N); 41,56 (CH₂N); 45,89 (CHS); 46,34 (CHS); 48,24 (q, J= 31 Hz, CF₃CH); 48,62 (q, J= 31 Hz, CF₃CH); 70,68 (CH₂CH₃); 70,71 (CH₂CH₃); 123,52 (CH_{Ar.}); 123,61 (CH_{Ar.}); 124,85 (q, J= 279 Hz, CF₃); 124,99 (q, J= 280 Hz, CF₃); 131,67 (Cq_{Ar.}); 131,72 (Cq_{Ar.}); 134,36 (2 x CH_{Ar}); 168,17 (2 x C=O_{Ar}.); 168,24 (2 x C=O_{Ar}.); 170,34 (C=O); 170,75 (C=O); 211,90 (C=S); 212,75 (C=S).

IR (v, cm⁻¹) (CCl₄)

3441 (NH); 2983; 1776 (C=O); 1722 (C=O); 1504; 1468; 1441; 1392; 1366; 1227; 1187; 1134; 1112; 1050.

Masse (IC, NH₃)

449 (MH⁺); 366 (MNH₄⁺).

| Microanalyse | Elément: | Carbone | Hydrogène |
|---|---|---|---|
| | Calculé (%) | 48,21 | 4,27 |
| | Trouvé (%) | 48,61 | 4,42 |

### Exemple 10 :

### Ester de l'acide (4-acétylamino-2-éthoxythiocarbonylsulfanyl-5,5,5-trifluoropentyl)-phosphonique diéthylique

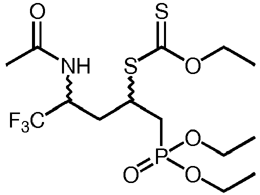

C₁₄H₂₅F₃NO₅PS₂ M= 439,45 g.mol⁻¹

### Réaction :

Effectuée selon le mode opératoire général avec 200 mg (0,77 mmol) de xanthate de l'exemple 1 et 407 mg (2,29 mmol) de diéthyle ester d'allyl phosphonique dans 1,5 ml de 1,2-dichloroéthane. La réaction est terminée après addition de 5% de DLP et 1 heure 30 minutes de reflux.

### Purification :

Chromatographie sur gel de silice (dichlorométhane-méthanol 99/1).

### Produit :

Huile jaune pâle.

### Rendement :

86% (2 diastéréoisomères dans un rapport 6/4)

RMN¹H (δ, ppm) (CDCl₃, 400 MHz)

1,29 (t, J= 7,6 Hz, 6H, CH₂CH₃); 1,30 (t, J= 7,0 Hz, 6H, CH₂CH₃); 1,25 (t, J= 7,0 Hz, 3H, CH₂CH₃); 1,36 (t, J= 7,0 Hz, 3H, CH₂CH₃); 1,99 (s, 3H, COCH₃); 2,02 (s, 3H, COCH₃); 2,06-2,45 (m, 8H, CH₂P + CF₃CH(NAc)CH₂); 3,92-4,16 (m, 10 H, CH₃CH₂ + CHS); 4,53-4,61 (m, 4H, CH₃CH₂); 4,65-4,79 (m, 2H, CF₃CH); 7,05 (d, J= 8,8 Hz, 1 H, NH); 7,10 (d, J= 9,4 Hz, 1 H, NH).

RMN¹³C (δ, ppm) (CDCl₃, 100 MHz)

13,68 (2 x CH₃CH₂); 16,34 (m, CH₃CH₂OP); 22,90 (2 x CH₃CO); 29,02 (d, J= 138 Hz, CH₂P); 31,26 (CF₃CH (NAc)CH₂); 31,45 (CF₃CH(NAc)CH₂); 31,75 (d, J= 135 Hz, CH₂P); 41,52 (CHS); 42,09 (CHS); 48,37 (q, J= 30 Hz, CF₃CH); 48,68 (q, J= 30 Hz, CF₃CH); 62,21 (m, CH₃CH₂OP); 70,35 (2 x CH₂CH₃); 124,85 (2 x q, J= 281 Hz, CF₃); 170,51 (C=O); 170,67 (C=O); 212,20 (C=S); 212,87 (C=S).

IR (v, cm⁻¹) (CCl₄)

3309 (NH); 2983; 1699 (C=O); 1532; 1442; 1390; 1368; 1291; 1227; 1186; 1135; 1112; 1048; 1028.

Masse (IC, NH₃)

318 (M-HSCSOEt+H⁺); 440 (MH⁺); 457 (MNH₄⁺).

| Microanalyse | Elément: | Carbone | Hydrogène |
|---|---|---|---|
| | Calculé (%) | 38,26 | 5,73 |
| | Trouvé (%) | 38,01 | 5,81 |

### Exemple 11 :

### Ester de l'acide 4-acétylamino-2-éthoxythiocarbonylsulfanyl-5,5,5-trifluoro-pentyle acétique

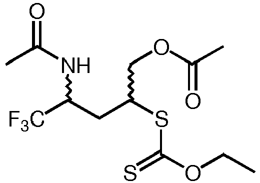

C₁₂H₁₈F₃NO₄S₂ M= 361,40 g.mol⁻¹

### Réaction :

Effectuée selon le mode opératoire général avec 200 mg (0,77 mmol) de xanthate de l'exemple 1 et 247 µL (3 mmol) d'acétate d'allyle dans 1,5 ml de 1,2-dichloroéthane. La réaction est terminée après addition de 5% de DLP et 1 heure 30 minutes de reflux.

### Purification :

Chromatographie sur gel de silice (acétate d'éthyle-éther de pétrole 3/7).

### Produit :

Huile jaune pâle.

### Rendement :

84% (2 diastéréoisomères dans un rapport 1/1).

RMN¹H (δ, ppm) (CDCl₃, 400 MHz)

1,38 (t, J= 7,0 Hz, 3H, CH₂CH₃); 1,40 (t, J= 7,0 Hz, 3H, CH₂CH₃); 1,89-2,15 (m, 3H, CF₃CH(NAc)CH₂); 1,99 (s, 3H, COCH₃); 2,06 (s, 3H, COCH₃); 2,07 (s, 3H, COCH₃); 2,08 (s, 3H, COCH₃); 2,20-2,27 (m, 3H, CF₃CH(NAc)CH₂); 3,92-3,98 (m, 1 H, CHS); 4,09-4,15 (m, 1 H, CHS); 4,24-4,41 (m, 4H, CH₂OCOCH₃); 4,60 (q, J= 7,0 Hz, 2H, CH₃CH₂); 4,62 (q, J= 7,0 Hz, 2H, CH₃CH₂); 4,72-4,91 (m, 2H, CF₃CH); 6,50 (d, J= 9,4 Hz, 1 H, NH); 6,64 (d, J= 9,4 Hz, 1 H, NH).

RMN¹³C (δ, ppm) (CDCl₃, 100 MHz)

13,71 (CH₃CH₂); 13,73 (CH₃CH₂); 20,69 (CH₃CO); 20,78 (CH₃CO); 22,97 (CH₃CO); 22,99 (CH₃CO); 29,04 (CF₃CH(NAc)CH₂); 30,12 (CF₃CH(NAc)CH₂); 45,62 (CHS); 46,00 (CHS); 48,36 (q, J= 30 Hz, CF₃CH); 48,49 (q, J= 30 Hz, CF₃CH); 63,98 (CH₂OCOCH₃); 65,94 (CH₂OCOCH₃); 70,71 (CH₂CH₃); 70,83 (CH₂CH₃); 124,75 (q, J= 281 Hz, CF₃); 124,99 (q, J= 281 Hz, CF₃); 170,49 (C=O); 170,64 (C=O); 170,75 (C=O); 170,86 (C=O); 212,39 (C=S); 212,94 (C=S).

IR (ν, cm⁻¹)(CCl₄)

3442 (NH); 2984; 1752 (C=O); 1703 (C=O); 1506; 1442; 1381; 1367; 1340; 1227; 1186; 1137; 1112; 1052.

Masse (IC, NH₃)

302 (M-HOAc+H⁺); 362 (MH⁺); 379 (MNH₄⁺).

| Microanalyse | Elément: | Carbone | Hydrogène |
|---|---|---|---|
| | Calculé (%) | 39,88 | 5,02 |
| | Trouvé (%) | 40,08 | 5,07 |

### Exemple 12 :

### Ester de l'acide S-[3-acétylamino-4,4,4-trifluoro-1-(2-oxo-pyrrolidin-1yl)-butyl] dithiocarbonique ester O-éthylique

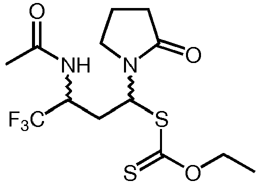

C₁₃H₁₉F₃N₂O₃S₂ M= 372,43 g.mol⁻¹

### Réaction :

Effectuée selon le mode opératoire général avec 200 mg (0,77 mmol) de xanthate de l'exemple 1 et 104 µL (mmol) de vinyl-pyrrolidin-2-one dans 1,5 ml de 1,2-dichloroéthane. La réaction est terminée après addition de 10% de DLP et 3 heures de reflux.

### Purification :

Chromatographie sur gel de silice (dichlorométhane-méthanol 98/2).

### Produit :

Huile jaune pâle instable conduisant à l'énamide de l'exemple 13.

### Rendement :

62% (2 diastéréoisomères dans un rapport 4/6)

21 % de l'énamide de l'exemple 13

RMN¹H (δ, ppm) (CDCl₃, 400 MHz)

1,41 (t, J= 7,0 Hz, 3H, CH₂CH₃); 1,42 (t, J= 7,0 Hz, 3H, CH₂CH₃); 2,02-2,26 (m, 7H, CH₂CO + CF₃CH(NAc)CH₂); 2,07 (s, 3H, COCH₃); 2,09 (s, 3H, COCH₃); 2,35-2,42 (m, 4H, CH₂CH₂CH₂); 2,60-2,67 (m, 1 H, CF₃CH(NAc)CH₂); 3,41-3,57 (m,4H, CH₂N); 4,50-4,62 (m, 1 H, CF₃CH); 4,64 (q, J= 7,0 Hz, 2H, CH₃CH₂); 4,65 (q, J= 7,0 Hz, 2H, CH₃CH₂); 4,74-4,86 (m, 1 H, CF₃CH); 5,90 (dd, J= 12,3 Hz, 4,1 Hz, 1 H, CHS); 6,02 (dd, J= 8,2 Hz, 7,6 Hz, 1 H, CHS); 6,69 (d, J= 10,0 Hz, 1 H, NH); 7,29 (d, J= 10,0 Hz, 1 H, NH).

RMN¹³C (δ, ppm) (CDCl₃, 100 MHz)

13,68 (CH₃CH₂); 13,76 (CH₃CH₂); 18,00 (CH₂CH₂CH₂); 18,15(CH₂CH₂CH₂); 22,94 (CH₃CO); 23,02 (CH₃CO); 30,66 (CH₂CO); 30,84 (CH₂CO); 31,06 (CF₃CH(NAc)CH₂); 31,25 (CF₃CH(NAc)CH₂); 44,95 (CH₂N); 45,24 (CH₂N); 47,49 (q, J= 30 Hz, CF₃CH); 48,08 (q, J= 32 Hz, CF₃CH); 58,33 (CHS); 58,98 (CHS); 70,62 (CH₂CH₃); 70,71 (CH₂CH₃); 124,75 (q, J= 281 Hz, CF₃); 124,82 (q, J= 282 Hz, CF₃); 170,54 (C=O); 170,86 (C=O); 175,52 (C=O); 175,57 (C=O); 210,06 (C=S); 211,76 (C=S).

IR (v, cm⁻¹) (CCl₄)

3439 (NH); 3298 (NH); 2984; 1703 (C=O); 1501; 1416; 1368; 1286; 1265; 1226; 1183; 1132; 1111; 1049.

Masse (IC, NH₃)

251 (M-HSCSOEt+H⁺); 268 (M-HSCSOEt+NH₄⁺); 373 (MH⁺); 390 (MNH₄⁺).

### Exemple 13 :

### Ester de l'acide S-[3-acétylamino-1-{[(4-bromo-phényl)-méthanesulfonyl-amino]-méthyl}-4,4,4-trifluoro-butyl) dithiocarbonique ester O-éthylique

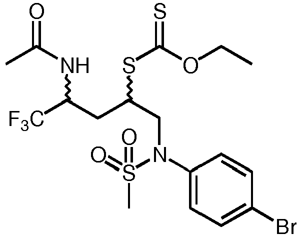

C₁₇H₂₂BrF₃N₂O₄S₃ M=551,463 g.mol⁻¹

### Réaction :

Effectuée selon le mode opératoire général avec 261 mg (1 mmol) de xanthate de l'exemple 1 et 580 mg (2 mmol) de *N*-allyl-*N-*(4-bromo-phenyl)-méthanesulfonamide dans 2 ml de 1,2-dichloroéthane. La réaction est terminée après addition de 20% de DLP et 7 heures de reflux.

### Purification :

Chromatographie sur gel de silice (acétate d'éthyle-éther de pétrole 4/6).

### Produit :

Mousse blanche.

### Rendement :

77% (2 diastéréoisomères dans un rapport 1/1)

RMN¹H (δ, ppm) (CDCl₃, 400 MHz)

1,29 (t, J= 7,0 Hz, 3H, CH₂CH₃) ; 1,33 (t, J= 7,0 Hz, 3H, CH₂CH₃) ; 1,86 (s, 3H, COCH₃) ; 1,90-2,04 (m, 2H, CF₃CH(NAc)CH₂) ; 1,94 (s, 3H, COCH₃) ; 2,19-2,30 (m, 2H, CF₃CH(NAc)CH₂) ; 2,83 (s, 6H, SO₂CH₃) ; 3,56-3,70 (m, 2H, CHS) ; 3,81-3,96 (m, 4H, CH₂N) ; 4,44-4,57 (m, 4H, CH₃CH₂) ; 4,70-4,83 (m, 2H, CF₃CH) ; 6,50 (d, J= 9,4 Hz, 1 H, NH) ; 6,67 (d, J= 9,4 Hz, 1 H, NH) ; 7,20 (d, J= 8,2 Hz, 2H, H_{Ar}(HC=CNSO₂)) ; 7,30 (d, J= 8,2 Hz, 2H, H_{Ar}(HC=CNSO₂)) ; 7,51 (d, J= 8,2 Hz, 2H, H_{Ar}(HC=CBr)) ; 7,54 (d, J= 8,2 Hz, 2H, H_{Ar}(HC=CBr)).

RMN¹³C (δ, ppm) (CDCl₃, 100 MHz)

13,53 (CH₃CH₂) ; 13,61 (CH₃CH₂) ; 22,64 (CH₃CO) ; 22,75 (CH₃CO); 28,18 (CF₃CH(NAc)CH₂) ; 29,52 (CF₃CH(NAc) CH₂) ; 36,70 (CH₃SO₂) ; 36,97 (CH₃SO₂) ; 44,84 (CHS) ; 45,67 (CHS) ; 48,03 (q, J= 32 Hz, 2 x CF₃CH) ; 51,40 (CH₂N) ; 53,37 (CH₂N) ; 70,61 (2 x CH₂CH₃) ; 122,64 (2 x Cq_{Ar}.Br) ; 124,64 (q, J= 281 Hz, CF₃) ; 124,79 (q, J= 281 Hz, CF₃) ; 130,32 (CH_{Ar.}) ; 130,43 (CH_{Ar.}) ; 132,73 (2 x CH_{Ar.}) ; 137,14 (Cq_{Ar.}NSO₂) ; 137,31 (Cq_{Ar.}NSO₂) ; 170,43 (C=O) ; 170,72 (C=O) ; 211,49 (C=S) ; 212,54 (C=S).

IR (v, cm⁻¹) (CCl₄)

3437 (NH) ; 2984 ; 1700 (C=O) ; 1488 ; 1442 ; 1358 ; 1227 ; 1187 ; 1162 ; 1140 ; 1112 ; 1050 ; 1012.

Masse (IC, NH₃)

552 (MH⁺) ; 569 (MNH₄⁺).

| Microanalyse | Elément : | Carbone | Hydrogène |
|---|---|---|---|
| | Calculé (%) | 37,03 | 4,02 |
| | Trouvé (%) | 37,06 | 4,11 |

### Exemple 14 : Ester de l'acide dithiocarbonique S-[1-(2-acétylamino-3,3,3-trifluoropropyl)-2-phényl-cyclopropane] O-éthyle

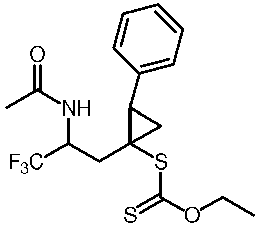

C₁₇H₂₀F₃NO₅S₂ M= 391,48 g.mol⁻¹

### Réaction :

Effectuée selon le mode opératoire général avec 131 mg (0,50 mmol) de xanthate de l'exemple 1 et 130 mg (1,00 mmol) de 1-méthylèn-2-phénylcyclopropane dans le 1,2-dichloroéthane (2 mL). La réaction est terminée après addition de 10% de DLP (20 mg) et 3h de reflux.

### Purification :

Chromatographie sur gel de silice (éther-éther de pétrole 3/7).

### Produit :

### Premier diastéréoisomère :

Rf (éther-éther de pétrole 3/7) = 0,20, huile jaune pâle qui cristallise lentement.

### Second diastéréoisomère :

Rf (éther-éther de pétrole 3/7) = 0,16, huile jaune pâle.

### Rendement :

68% (2 diastéréoisomères dans un rapport 3/1)

### Premier diastéréoisomère (majoritaire) :

RMN¹H (δ, ppm) (CDCl₃, 400 MHz)

1,46 (t, *J =* 5,0 Hz, 3H, CH₂C*H*₃); 1,86 (s, 3H, COC*H*₃); 2,05 (d, *J =* 7,0Hz, 2H, CH₂CHC_{Ar.}) ; 1,86-2,16 (m, 2H, CH₂CHCF₃) ; 2,71 (dd, *J₁* = 8,0Hz, *J₂* = 0,8Hz, 1 H, CHC_{Ar.}) ; 4,59-4,64 (m, 2H, CH₃C*H*₂); 4,64-4,68 (m, 1 H, CHCF₃) ; 5,06 (d, *J* = 9,4 Hz, 1 H, N*H*); 7,20-7,50 (m, 5H, *H*_{Ar.}).

RMN¹³C (δ, ppm) (CDCl₃, 100 MHz)

13,6 (CH₃CH₂); 16,6 (CH₃CO); 21,9 (CH₂CHCF₃); 28,8 (CH₂CHC_{Ar.}); 31,1 (CHC_{Ar.}) ; 32,8 (*C*q_{ciclo.}); 49,9 (q, *J* = 29 Hz, CF₃*C*H); 70,1 (OCH₂CH₃); 124,2 (*C*F₃); 129,6 (2x*CH*_{Ar.}) ; 130,0 (2x*CH*_{Ar}.) ; 130,8 (*C*H_{Ar}.) ; 138,4 (*C*q_{Ar.}); 170,9 (N*C*=O); 212,7 (*C*=S).

IR (v, cm⁻¹)(CCl₄)

3432 (NH); 2927; 1702 (C=O); 1498; 1225; 1224 (C=S); 1221; 1124; 1051.

Masse (IC, NH3)

392 (MH⁺); 409 (MNH₄⁺).

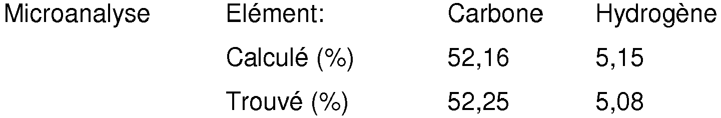

Tf

137 °C (acétate d'éthyle-heptane)

### Second diastéréoisomère (minoritaire) :

RMN¹H (δ, ppm) (CDCl₃, 400 MHz)

1,36 (t, *J =* 5,0 Hz, 3H, CH₂C*H*₃); 1,96 (s, 3H, COC*H*₃); 2,45 (d, *J =* 12,0 Hz, 2H, CH₂CHC_{Ar}.) ; 1,96-2,43 (m, 2H, CH₂CHCF₃) ; 2,83 (dd, *J₁* = 5,7 Hz*, J₂* = 0,8 Hz, 1 H, CHC_{Ar}.) ; 4,64-4,69 (m, 2H, CH₃C*H*₂); 4,89-4,98 (m, 1 H, CHCF₃) ; 6,25 (d, *J* = 11,4 Hz, 1 H, N*H*); 7,10-7,50 (m, 5H, *H*_{Ar}.).

RMN¹³C (δ, ppm) (CD₃OD,100 MHz)

13,9 (CH₃CH₂); 16,7 (CH₃CO); 32,5 (*C*q_{ciclo.}); 31,1 (CHC_{Ar.}) 28,6 (CH₂CHC_{Ar.}); 21,9 (CH₂CHCF₃); 49,6 (q, *J* = 29 Hz, CF₃*C*H); 70,2 (OCH₂CH₃); 124,1 (*C*F₃); 129,5 (2xCHAr.) ; 129,9 (2xCHAr.) ; 130,3 (*C*H_{Ar.}) ; 138,3 (*C*q_{Ar}.); 170,5 (N*C*=O); 212,8 (C=S).

IR (v, cm⁻¹) (CCl₄)

3432 (NH); 2927; 1702 (C=O); 1498; 1225; 1224 (C=S); 1221; 1124; 1051.

Masse (IC, NH3)

392 (MH⁺); 409 (MNH₄⁺).

### Exemple 15 : Ester de l'acide acétique 4-benzoylamino-2-éthoxythio-carbonylsulfanyl-5,5,5-trifluoro-butyle

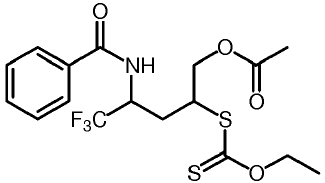

C₁₇H₂₀F₃NO₄S₂ M= 423,08 g.mol⁻¹

### Réaction :

Effectuée selon le mode opératoire général avec 500 mg (1,55 mmol) de xanthate dithiocarbonic acid *S*-(1-benzoylamino-2,2,2-trifluoro-éthyl) ester *O*-éthyl ester [Mode opératoire du xanthate à fournir] et 333 µL (3 mmol) d'acétate d'allyle dans le 1,2-dichloroéthane (3mL). La réaction est terminée après addition de 10 % de DLP (62 mg) et 1 h30 de reflux.

### Purification :

Chromatographie sur gel de silice (éther-éther de pétrole 3/7).

### Produit :

Huile jaune pâle.

### Rendement :

84% (mélange de 2 diastéréoisomères dans un rapport 1/1).

RMN¹H (δ, ppm) (CDCl₃, 400 MHz)

1,32 (t, *J =* 7,0 Hz, 1,5H, CH₂C*H*₃); 1,38 (t, *J =* 7,0 Hz, 1,5H, CH₂C*H*₃); 1,89-2,15 (m, 1 H, CF₃CHNC*H*₂); 1,99 (s, 1,5H, COC*H*₃); 2,06 (s, 1,5H, COC*H*₃); 2,20-2,27 (m, 1 H, CF₃CHNC*H*₂); 3,92-3,98 (m, 0,5H, CHS); 4,09-4,15 (m, 0,5H, CHS); 4,24-4,41 (m, 2H, C*H*₂OCOCH₃); 4,52 (q, *J =* 6,1 Hz, 1 H, CH₃C*H*₂); 4,54 (q, *J =* 5,0 Hz, 1 H, CH₃C*H*₂); 4,98-5,12 (m, 1 H, CF₃C*H*); 6,20 (d, *J =* 8,7 Hz, 0,5H, N*H*); 6,54 (d, J= 8,9 Hz, 0,5H, N*H*) ; 7,31-7,92 (m, 5H, CH_{Ar.}).

RMN¹³C (δ, ppm) (CDCl₃, 100 MHz)

13,7 (*C*H₃CH₂); 22,9 (*C*H₃CO); 29,0 (0,5xCF₃CH*C*H₂); 30,1 (0,5xCF₃CH*C*H₂); 45,6 (0,5xCHS); 46,0 (0,5xCHS); 48,3 (q, J= 30 Hz, 0,5xCF₃*C*H); 48,4 (q, J= 30 Hz, 0,5xCF₃*C*H); 63,9 (0,5x*C*H₂OCOCH₃); 65,9 (0,5x*C*H₂OCOCH₃); 70,7 (0,5x*C*H₂CH₃); 70,8 (0,5x*C*H₂CH₃); 124,5 (q, *J =* 281 Hz, 0,5x*C*F₃); 124,9 (q, *J =* 281 Hz, 0,5xCF3); 132,5 (2,5xCHAr.) ; 133,7 (2,5xCHAr.) ; 145,4 (0,5x*C*q_{Ar.}); 146,3 (0,5xCq_{Ar.}); 170,4 (0,5xO*C*=O); 170,6 (0,5xO*C*=O); 170,7 (0,5x*C*=O); 170,8 (0,5x*C*=O); 212,3 (0,5xC=S); 212,9 (0,5xC=S).

IR (v, cm⁻¹) (CCl₄)

3442 (NH); 2984; 1752 (OC=O); 1703 (NC=O); 1506; 1442; 1381; 1367; 1340; 1224 (C=S); 1186; 1137; 1112; 1052.

Masse (IC, NH3)

424 (MH⁺); 441 (MNH₄⁺).

| Microanalyse | Elément : | Carbone | Hydrogène |
|---|---|---|---|
| | Calculé (%) | 48,22 | 4,76 |
| | Trouvé (%) | 48,08 | 4,83 |

### Exemple 16 : Ester 4-tertbutyloxycarbamate-2-éthoxythiocarbonylsulfanyl-5,5,5-trifluoro-pentylique de l'acide acétique

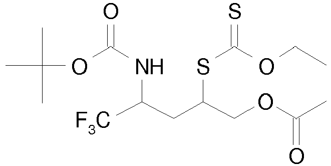

C₁₅H₂₄F₃NO₅S₂ M= 419, 49 g.mol⁻¹

### Réaction :

Effectuée selon le mode opératoire général avec 50 mg (0,2 mmol) de xanthate de l'exemple 2 et 32 mg (0,4 mmol) d'acétate d'allyle dans le 1,2-dichloroéthane (1 mL). La réaction est terminée après addition de 15% de DLP (12 mg) et 4h30 de reflux.

### Purification :

Chromatographie sur gel de silice (acétate d'éthyle-éther de pétrole 5/95).

### Produit :

Huile jaune pâle.

### Rendement :

76% (2 diastéréoisomères dans un rapport 1/1)

### Premier diastéréoisomère :

RMN¹H (δ, ppm) (CDCl₃, 400 MHz)

1,38 (t, *J* = 7,0 Hz, 3H, CH₂C*H*₃); 2,0 (s, 9H, 3xC*H*₃); 2,12 (s, 3H, COC*H*₃); 2,18-2,25 (m, 2H, CF₃CHNC*H*₂); 3,97-4,05 (m, 1 H, C*H*S); 4,30 (dd, *J₁* = 2,9 Hz, *J₂ =* 12,2 Hz, 1 H, 1xC*H*₂OCOCH₃); 4,49 (dd, *J₁* = 2,9 Hz, *J₂* = 12,2 Hz, 1H, 1xC*H*₂OCOCH₃); 4,61 (q, *J* = 5,9 Hz, 1 H, CF₃C*H*); 4,65 (q, *J* = 7,0 Hz, 2H, CH₃C*H*₂); 4,92 (d, *J =* 11,7 Hz, 1 H, N*H*).

RMN¹³C (δ, ppm) (CDCl₃, 100 MHz) 13,7 (*C*H₃CH₂); 18,3 (3x*C*H₃); 28,6 (*C*H₃CO); 30,1 (CF₃CHN*C*H₂); 46,0 (CHS); 48,5 (q, *J =* 30 Hz, CF₃*C*H); 65,9 (*C*H₂OCOCH₃); 70,8 (*C*H₂CH₃); 125,0 (q, *J =* 281 Hz, *C*F₃); 152,2 (*C*(CH₃)₃); 170,6 (*C*=O); 170,9 (*C*=O); 212,7 (C=S).

IR (v, cm⁻¹) (CCl₄)

3442 (NH); 2983; 1753 (C=O); 1422; 1263; 894; 869.

Masse (IC, NH3)

420 (MH⁺); 437 (MNH₄⁺).

| Microanalyse | Elément : | Carbone | Hydrogène |
|---|---|---|---|
| | Calculé (%) | 42,95 | 5,77 |
| | Trouvé (%) | 42,92 | 5,78 |

### Deuxième diastéréoisomère :

RMN¹H (δ, ppm) (CDCl₃, 400 MHz)

1,38 (t, *J* = 7,0 Hz, 3H, CH₂C*H*₃); 2,05 (s, 9H, 3xC*H*₃); 2,14 (s, 3H, COC*H*₃); 2,19-2,22 (m, 2H, CF₃CHNC*H*₂); 4,05-4,12 (m, 1 H, C*H*S); 4,32 (dd, *J₁* = 2,9 Hz, *J₂ =* 12,2 Hz, 1 H, 1xC*H*₂OCOCH₃); 4,42 (dd,*J₁* = 2,9 Hz, *J₂* = 12,2 Hz, 1H, 1xC*H*₂OCOCH₃); 4,56 (q, *J* = 5,8 Hz, 1 H, CF₃C*H*); 4,62 (q, *J* = 7,0 Hz, 2H, CH₃C*H*₂); 4,75 (d, *J* = 12,4 Hz, 1H, N*H*).

RMN¹³C (δ, ppm) (CDCl₃, 100 MHz)

13,7 (*C*H₃CH₂); 18,5 (3x*C*H₃); 28,4 (*C*H₃CO); 29,0 (CF₃CHN*C*H₂); 45,6 (CHS); 48,5 (q, *J =* 30 Hz, CF₃*C*H); 64,0 (*C*H₂OCOCH₃); 70,7 (*C*H₂CH₃); 124,7 (q, *J =* 281 Hz, *C*F₃); 152,4 (*C*(CH₃)₃); 170,5 (*C*=O); 170,7 (*C*=O); 212,4 (C=S).

IR (v, cm⁻¹) (CCl₄)

3442 (NH); 2983; 1732 (C=O); 1422; 1263; 894; 869.

Masse (IC, NH3)

420 (MH⁺); 437 (MNH₄⁺).

### Exemple 17 : Ester O-éthylique S-(3-tertbutyloxycarbamate-1-diéthoxy-méthyl-4,4,4-trifluoro-butyle de l'acide dithiocarbonique

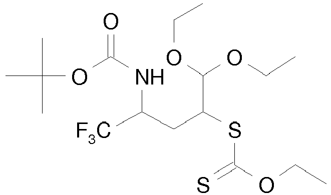

C₁₇H₃₀F₃NO₅S₂ M= 448,57 g.mol⁻¹

### Réaction :

Effectuée selon le mode opératoire général avec 50 mg (0,2 mmol) de xanthate de l'exemple 2 et 48 µL (0,4 mmol) de 3,3-diéthoxy-propène dans le 1,2-dichloroéthane (1 mL). La réaction est terminée après addition de 15% de DLP (12 mg) et 4h30 de reflux.

### Purification :

Chromatographie sur gel de silice (acétate d'éthyle-éther de pétrole 5/95).

### Produit :

Huile épaisse jaune pâle.

### Rendement :

94% (mélange de 2 diastéréoisomères dans un rapport 1/1)

RMN¹H (δ, ppm) (CDCl₃, 400 MHz)

1,23 (t, *J* = 6,1 Hz, 1,5H, CH₂C*H*₃); 1,25 (t, *J* = 6,1 Hz, 1,5H, CH₂C*H*₃); 1,26 (t, *J =* 6,1 Hz, 1,5H, CH₂C*H*₃); 1,28 (t, *J =* 6,1 Hz, 1,5H, CH₂C*H*₃); 1,45 (t, *J =* 6,1 Hz, 3H, CH₂C*H*₃); 1,52 (s, 4,5H, C(C*H*₃)₃); 1,56 (s, 4,5H, C(C*H*₃)₃); 1,75 (dd, *J₁* = 4,0 Hz, *J₂ =* 14,2 Hz, 0,5H, CF₃CHNC*H*₂); 1,85 (dd, *J₁* = 4,0 Hz, *J₂ =* 14,2 Hz, 0,5H, CF₃CHNC*H*₂); 2,15 (dd, *J₁* = 4,0 Hz, *J₂ =* 14,2 Hz, 0,5H, CF₃CHNC*H₂*); 2,55 (dd, *J₁* = 4,0 Hz, *J₂* = 14,2 Hz, 0,5H, CF₃CHNC*H₂*); 3,53 (q, *J* = 6,0 Hz, 1 H, CH₃C*H*₂O); 3,55 (q, *J* = 6,0 Hz, 1 H, CH₃C*H*₂O); 3,63 (q, *J* = 6,0 Hz, 1 H, CH₃C*H*₂O); 3,78 (q, *J =* 6,0 Hz, 1 H, CH₃C*H*₂O); 3,85-4,02 (m, 1 H, CHS); 4,06-4,12 (m, 1 H, C*H*(OEt)₂); 4,48-4,51 (m, 1 H, CF₃C*H*); 4,63 (q, *J =* 6,1 Hz, 1 H, CH₃C*H*₂); 4,73 (q, *J=* 6,1 Hz, 1 H, CH₃C*H*₂); 4,81 (d, *J=* 12,2 Hz, 0,5H, N*H*); 4,83 (d, *J* = 12,2 Hz, 0,5H, N*H*).

RMN¹³C (δ, ppm) (CDCl₃, 100 MHz)

13,8 (CH₃CH₂); 15,2 (*C*H₃CH₂); 15,2 (0,5x*C*H₃CH₂); 15,3 (0,5x*C*H₃CH₂); 19,5 (3x*C*H₃); 25,8 (0,5x*C*H₂CHS); 28,5 (0,5x*C*H₂CHS); 48,4 (q, *J* = 30 Hz, 0,5x*C*F₃*C*H); 49,0 (q, *J =* 30 Hz, 0,5xCF₃*C*H); 49,9 (0,5x*C*HS); 50,4 (0,5x*C*HS); 64,0 (0,5x*C*H₂CH₃); 64,3 (0,5x*C*H₂CH₃); 64,7 (0,5x*C*H₂CH₃); 65,5 (0,5x*C*H₂CH₃); 70,5 (0,5x*C*H₂CH₃); 70,6 (0,5x*C*H₂CH₃);102,7 (0,5x*C*H(OEt)₂); 103,9 (0,5xCH(OEt)₂); 124,9 (q, *J =* 281 Hz, 0,5x*C*F₃); 125,1 (q, *J =* 281 Hz, 0,5x*C*F₃); 150,4 (0,5x*C*(CH₃)₃); 151,2 (0,5xC(CH₃)₃); 170,1 (0,5x*C*=O); 171,3 (0,5x*C*=O); 213,9 (0,5x*C*=S); 214,7 (0,5x*C*=S).

IR (v, cm⁻¹) (CCl₄)

3443 (NH); 2976; 2905; 2805; 1734 (C=O); 1720 (C=O); 1594; 1246; 1213; 1180; 1168; 1070.

Masse (IC, NH3)

402 (MH⁺-EtOH).

| Microanalyse | Elément: | Carbone | Hydrogène |
|---|---|---|---|
| | Calculé (%) | 48,20 | 6,97 |
| | Trouvé (%) | 47,05 | 6,93 |

### Exemple 18 : Diester de O-éthyle et de S-(3-tertbutyl-oxycarbamate-1-diéthoxyméthyl-4,4,4-trifluoro- butyle) de l'acide dithiocarbonique

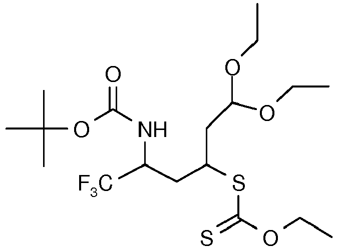

C₁₈H₃₂F₃NO₅S₂ M= 463,58 g.mol⁻¹

### Réaction :

Effectuée selon le mode opératoire général avec 200 mg (0,6 mmol) de xanthate de l'exemple 2 et 181 mg (1,25 mmol) de 4,4-diéthoxy-butène dans le 1,2-dichloroéthane (2 mL). La réaction est terminée après addition de 15% de DLP (36 mg) et 4h30 de reflux.

### Purification :

Chromatographie sur gel de silice (acétate d'éthyle-éther de pétrole 5/95).

### Produit :

Huile épaisse jaune pâle.

### Rendement :

72% (mélange de 2 diastéréoisomères dans un rapport 1/1)

RMN¹H (δ, ppm) (CDCl₃, 400 MHz)

0,92 (dd, *J₁* = 8,8 Hz*, J₂ =* 13,3 Hz, 1 H, SCH-C*H*₂-CH); 0,95 (dd, *J₁* = 8,8 Hz, *J₂ =* 13,3 Hz, 1 H, SCH-C*H*₂-CH); 1,20 (t, *J =* 7,0 Hz, 1,5H, CH₂C*H*₃); 1,25 (t, *J =* 7,0 Hz, 1,5H, CH₂C*H*₃); 1,26 (t, *J =* 7,0 Hz, 1,5H, CH₂C*H*₃); 1,28 (t, *J =* 7,0 Hz, 1,5H, CH₂C*H*₃); 1,45 (t, *J =* 7,0 Hz, 3H, CH₂C*H*₃); 1,52 (s, 4,5H, C(C*H*₃)₃); 1,56 (s, 4,5H, C(C*H*₃)₃); 1,98-2,07 (m, 1 H, CF₃CHNC*H*₂); 2,08-2,15 (m, 1 H, CF₃CHNC*H*₂); 3,68-3,74 (m, 2H, CH₃C*H*₂O); 3,79-3,82 (m, 2H, CH₃C*H*₂O); 3,92-3,99 (m, 1 H, C*H*(OEt)₂); 4,02-4,13 (m, 1 H, CHS); 4,43-4,51 (m, 1 H, CF₃C*H*); 4,59-4,65 (m, 1 H, CH₃C*H*₂O); 3,64-3,67 (m, 1 H, CH₃C*H*₂O); 6,01 (d, *J =* 12,0 Hz, 0,5H, N*H*); 6,13 (d, *J=* 12,0 Hz, 0,5H, N*H*).

RMN¹³C (δ, ppm) (CDCl₃, 100 MHz)

13,7 (*C*H₃CH₂); 13,8 (CH₃CH₂); 15,1 (0,5x*C*H₃CH₂); 15,3 (0,5x*C*H₃CH₂); 19,8 (3x*C*H₃); 21,1 (0,5xCH-CH₂-CH); 21,3 (0,5xCH-CH₂-CH); 28,5 (0,5x*C*H₂CHS); 28,7 (0,5x*C*H₂CHS); 49,2 (q, *J* = 30 Hz, 0,5xCF₃*C*H); 49,5 (q, *J* = 30 Hz, 0,5xCF₃*C*H); 51,8 (0,5x*C*HS); 52,1 (0,5x*C*HS); 64,04 (0,5x*C*H₂CH₃); 64,2 (0,5x*C*H₂CH₃); 65,2 (0,5x*C*H₂CH₃); 65,5 (0,5x*C*H₂CH₃); 70,4 (0,5x*C*H₂CH₃); 70,6 (0,5x*C*H₂CH₃);100,9 (0,5x*C*H(OEt)₂); 101,3 (0,5x*C*H(OEt)₂); 122,8 (q, *J=* 283 Hz, 0,5x*C*F₃); 123,0 (q, *J* = 283 Hz, 0,5x*C*F₃); 158,3 (0,5x*C*(CH₃)₃); 158,4 (0,5x*C*(CH₃)₃); 170,0 (0,5x*C*=O); 171,4 (0,5x*C*=O); 214,0 (0,5x*C*=S); 214,2 (0,5xC=S).

IR (v, cm⁻¹) (CCl₄)

3440 (NH); 2973; 2910; 2810; 1732 (C=O); 1730 (C=O); 1594; 1243; 1210; 1176; 1164; 1064.

Masse (IC, NH3)

420 (MH⁺-EtOH).

### TRANSFORMATIONS DES ADDUITS DE FORMULE (IIA)

### Exemple 19 :

### N-[3-(2-Oxo-pyrrolidin-1-yl)-1-trifluorométhyl-allyl] acétamide

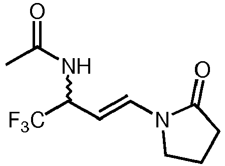

C₁₀H₁₃F₃N₂O₂ M= 250,22 g.mol⁻¹

### Réaction :

Une solution du xanthate de l'exemple 12 (170 mg, 045 mmol) dans 5 ml de chlorobenzène est portée au reflux pendant 2 heures. Le brut réactionnel est ramené à température ambiante puis concentré sous pression réduite avant d'être purifié.

### Purification :

Chromatographie sur gel de silice (dichlorométhane-méthanol 98/2).

### Produit :

Huile jaune pâle.

### Rendement :

Quantitatif.

RMN¹H (δ, ppm) (CDCl₃, 400 MHz)

2,03 (s, 3H, COCH₃); 2,06-2,14 (m, 2H, CH₂CH₂CH₂); 2,46 (dd, J= 7,3 Hz, 7,3 Hz, 2H, CH₂CO); 3,49 (dd, J= 7,3 Hz, 7,3 Hz, 2H, CH₂N); 4,87 (dd, J= 14,7 Hz, 6,4 Hz, 1 H, CH=CHN); 5,22 (qdd, J= 9,4 Hz, 7,6 Hz, 6,4 Hz, 1 H, CF₃CH); 7,20 (d, J= 14,7 Hz, 1 H, CH=CHN); 7,43 (d, J= 9,4 Hz, 1 H, NH).

RMN¹³C (δ, ppm) (CDCl₃, 100 MHz)

17,33 (CH₂CH₂CH₂); 22,82 (CH₃CO); 31,04 (CH₂CO); 45,09 (CH₂N); 50,60 (q, J= 30 Hz, CF₃CH); 101,48 (CH=CHN); 124,53 (q, J= 281 Hz, CF₃); 128,57 (CH=CHN); 170,28 (C=O); 173,84 (C=O).

IR (v, cm⁻¹) (CCl₄)

3444 (NH); 2980; 1708 (C=O); 1667; 1558; 1497; 1400; 1262; 1235; 1186; 1131.

Masse (IC, NH₃)

251 (MH⁺); 268 (MNH₄⁺).

| Microanalyse | Elément: | Carbone | Hydrogène |
|---|---|---|---|
| | Calculé (%) | 48,00 | 5,24 |
| | Trouvé (%) | 47,62 | 5,31 |

### Exemple 20 :

### N-[4-(1,3-dioxo-1,3-dihydro-isoindol-2-yl)-l-trifluorométhyl-butyl] acétamide

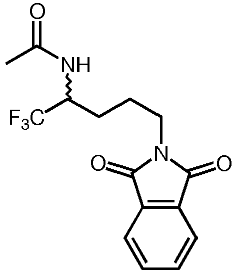

C₁₅H₁₅F₃N₂O₃ M= 328,29 g.mol⁻¹

### Réaction :

A une solution, préalablement dégazée au reflux sous argon, du xanthate adduit de l'exemple 9 (250 mg, 0,56 mmol) dans 4 ml propan-2-ol est additionné du DLP à raison de 10 mol% (22mg, 0,056 mmol) toutes les heures. La réaction est arrêtée après 11 heures de reflux et addition de 110% de DLP (244 mg, 0,61 mmol). Le milieu réactionnel est alors ramené à température ambiante et concentré sous pression réduite avant d'être purifié.

### Purification :

Chromatographie sur gel de silice (acétate d'éthyle-éther de pétrole 4/6).

### Produit :

Cristallin blanc.

### Rendement :

78%

RMN¹H (δ, ppm) (CDCl₃, 400 MHz)

1,48-1,60 (m, 1 H, CF₃CH(NAc)CH₂); 1,74-1,89 (m, 3H, CH₂CH₂N + CF₃CH(NAc)CH₂); 3,69 (t, J= 6,8 Hz, 2H, CH₂CH₂N); 4,59-4,72 (m, 1 H, CF₃CH); 6,40 (d, J= 9,3 Hz, 1 H, NH); 7,69-7,71 (m, 2H, H_{Ar.}); 7,79-7,81 (m, 2H, H_{Ar.}).

RMN¹³C (δ, ppm) (CDCl₃, 100 MHz)

23,02 (CH₃CO); 24,72 (CH₂); 25,14 (CH₂); 37,25 (CH₂N); 50,18 (q, J= 30 Hz, CF₃CH); 123,35 (2 x CH_{Ar}); 125,05 (q, J= 281 Hz, CF₃); 131,93 (Cq_{Ar.}); 134,19 (2 x CH_{Ar.}); 168,48 (2 x C=O_{Ar}.); 170,55 (C=O).

IR (ν, cm⁻¹)(CCl₄)

3445 (NH); 2932; 1774 (C=O); 1717 (C=O); 1505; 1438; 1396; 1369; 1244; 1187; 1136.

PF (°C)

179-180 (acétate d'éthyle - heptane)

Masse (IC, NH₃)

329 (MH⁺); 345 (MNH₄⁺).

| Microanalyse | Elément: | Carbone | Hydrogène |
|---|---|---|---|
| | Calculé (%) | 54,88 | 4,61 |
| | Trouvé (%) | 54,84 | 4,58 |

### Exemple 21 :

Ester de l'acide dithiocarbonique S-{1-[5-(1-acétylamino-2,2,2-trifluoro-éthyl)-2-oxo-[1,3]dioxolan-4-ylméthyl]-2,2-diéthoxy-éthyl} ester *O*-éthylique

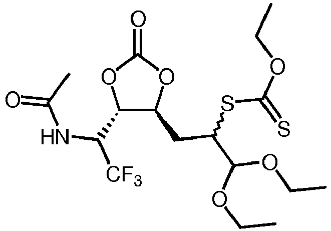

C₁₇H₂₆F₃NO₇S₂ M= 477,52 g.mol⁻¹

### Réaction :

Effectuée selon le mode opératoire général avec 95 mg (0,27 mmol) de xanthate de l'exemple 4 et 125 µl (0,82 mmol) de 3,3-diéthoxy-propène dans 1 ml de 1,2-dichloroéthane. La réaction est terminée après addition de 5% de DLP et 1 heure de reflux.

### Purification :

Chromatographie sur gel de silice (acétate d'éthyle-éther de pétrole 3/7).

### Produit :

Huile jaune pâle.

### Rendement :

40% (2 diastéréoisomères dans un rapport 1/1), isolé avec 17% du produit de double addition :

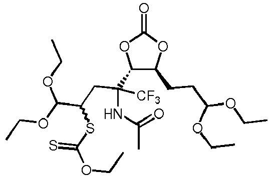

RMN¹H (δ, ppm) b(CDCl₃, 400 MHz)

1,17 (t, J= 7,0 Hz, 3H, CH₂CH₃); 1,19 (t, J= 7,0 Hz, 3H, CH₂CH₃); 1,22 (t, J= 7,0 Hz, 3H, CH₂CH₃); 1,23 (t, J= 7,0 Hz, 3H, CH₂CH₃); 1,42 (t, J= 7,0 Hz, 3H, CH₂CH₃); 1,43 (t, J= 7,0 Hz, 3H, CH₂CH₃); 1,95-2,15 (m, 2H, CH₂CHS); 2,07 (s, 3H, COCH₃); 2,09 (s, 3H, COCH₃); 2,37-2,45 (m, 2H, CH₂CHS); 3,42-3,82 (m, 8H, CH₃CH₂); 4,05-4,15 (m, 2H, CHS); 4,49-4,59 (m, 2H, CHOCO); 4,59 (s, 1 H, CH(OEt)₂); 4,60 (s, 1 H, CH(OEt)₂); 4,63 (q, J= 7,0 Hz, 2H, CH₃CH₂); 4,64 (q, J= 7,0 Hz, 2H, CH₃CH₂); 4,77-4,89 (m, 1 H, CHOCO); 4,97-5,09 (m, 3H, CF₃CH + CHOCO); 6,81 (d, J= 9,4 Hz, 1 H, NH); 6,83 (d, J= 9,4 Hz, 1 H, NH).

RMN¹³C (δ, ppm) (CDCl₃, 100 MHz)

13,78 (2 x CH₃CH₂); 15,15 (CH₃CH₂); 15,23 (CH₃CH₂); 15,29 (2 x CH₃CH₂); 22,70 (CH₃CO); 22,78 (CH₃CO); 33,22 (CH₂CHS); 34,24 (CH₂CHS); 48,94 (CHS); 49,66 (CHS); 51,01 (q, J= 31 Hz, CF₃CH); 51,08 (q, J= 31 Hz, CF₃CH); 64,36 (CH₂CH₃); 64,47 (CH₂CH₃); 65,35 (CH₂CH₃); 65,59 (CH₂CH₃); 70,75 (CH₂CH₃); 70,84 (CH₂CH₃); 76,37 (CHOCO); 77,10 (CHOCO);77,91 (CHOCO); 78,39 (CHOCO); 103,49 (CH(OEt)₂); 103,57 (CH(OEt)₂); 123,58 (q, J= 282 Hz, CF₃); 123,64 (q, J= 283 Hz, CF₃); 153,34 (OC=O); 153,46 (OC=O); 170,58 (NC=O); 170,70 (NC=O); 212,76 (C=S); 213,04 (C=S).

Masse (IC, NH3)

433 (M-EtOH+H⁺); 496 (MNH₄⁺).

### Exemple 22:

### N-(3,3-diméthoxy-1-trifluorométhyl-propyl)-acétamide

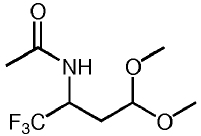

C₈H₁₄F₃NO₃ M= 229,20 g.mol⁻¹

### Réaction :

A une solution du xanthate de l'exemple 5 (200 mg, 0,57 mmol) dans 10 ml de méthanol est ajoutée une quantité catalytique d'acide (±)-10-camphorsulfonique. L'ensemble est portée au reflux pendant 24 heures. Le brut réactionnel est alors ramené à température ambiante puis concentré sous pression réduite avant d'être purifié.

### Purification :

Chromatographie sur gel de silice (acétate d'éthyle-éther de pétrole 7/3).

### Produit :

Cristallin incolore.

### Rendement :

72% (10% de l'aldéhyde correspondant au produit déprotégé sont également isolés).

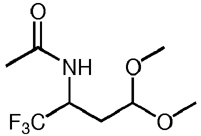

RMN¹H (δ, ppm)(CDCl₃, 400 MHz)

1,90 (ddd, J= 14,4 Hz, 10,0 Hz, 4,0 Hz, 1 H, CH₂); 2,04 (ddd, J= 14,4 Hz, 7,6 Hz, 3,3 Hz, 1 H, CH₂); 2,02 (s, 3H, COCH₃); 3,31 (s, 3H, OCH₃); 3,33 (s, 3H, OCH₃); 4,43 (dd, J= 7,6 Hz, 4,0 Hz, 1 H, CH(OMe)₂); 4,66-4,78 (m, 1 H, CF₃CH); 6,66 (d, J= 9,4 Hz, 1H, NH).

RMN¹³C (δ, ppm) (CDCl₃, 100 MHz)

22,97 (CH₃CO); 31,69 (CH₂); 47,54 (q, J= 31 Hz, CF₃CH); 53,29 (OCH₃); 53,88 (OCH₃); 101,55 (CH(OMe)₂); 125,09 (q, J= 281 Hz, CF₃); 170,46 (NC=O).

IR (ν, cm⁻¹) (CCl₄)

3444 (NH); 2936; 2833; 1704 (C=O); 1505; 1434; 1371; 1285; 1248; 1188; 1138; 1065.

PF (°C)

56-58 (acétate d'éthyle-heptane).

Masse (IC, NH₃)

198 (M-MeOH+H⁺); 230 (MH⁺).

| Microanalyse | Elément: | Carbone | Hydrogène |
|---|---|---|---|
| | Calculé (%) | 41,92 | 6,16 |
| | Trouvé (%) | 41,84 | 6,09 |

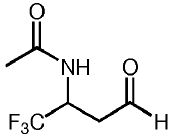

RMN¹H (δ, ppm) (CDCl₃, 400 MHz)

2,01 (s, 3H, COCH₃); 2,79-2,83 (m, 2H, CH₂); 5,08-5,21 (m, 1 H, CF₃CH); 7,15 (d, J= 9,4 Hz, 1 H, NH); 9,68 (s, 1 H, HC=O).

RMN¹³C (δ, ppm) (CDCl₃, 100 MHz)

22,76 (CH₃CO); 41,86 (CH₂); 458554 (q, J= 33 Hz, CF₃CH); 124,76 (q, J= 281 Hz, CF₃); 170,54 (NC=O); 197,18 (HC=O).

### Exemple 23 :

### N-[1-(5-Bromo-1-méthanesulfonyl-2,3-dihydro-1H-indol-3-ylméthyl)-2,2,2-trifluoroéthyl]-acétamide

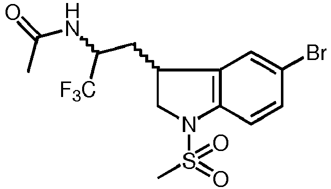

C₁₄H₁₆BrF₃N₂O₃S M= 429,25 g.mol⁻¹

### Réaction :

A une solution, préalablement dégazée au reflux sous argon, du xanthate adduit de l'exemple 13 (396 mg, 0,72 mmol) dans 4 ml de 1,2-dichloroéthane est additionné du DLP à raison de 10 mol% (28mg, 0,07 mmol) toutes les heures. La réaction est arrêtée après 11 h de reflux et addition de 110% de DLP (314 mg, 0,790 mmol). Le milieu réactionnel est alors ramené à température ambiante et concentré sous pression réduite avant d'être purifié.

### Purification :

Chromatographie sur gel de silice (acétate d'éthyle-éther de pétrole 5/5).

### Produit :

Cristallin blanc.

### Rendement :

90% (2 diastéréoisomères dans un rapport 6/4)

### Premier diastéréoisomère (majoritaire)

RMN¹H (δ, ppm) ((CD₃)₂CO, 400 MHz)

1,92-2,10 (m, 1H, CF₃CH(NAc)CH₂); 1,97 (s, 3H, COCH₃); 2,18-2,24 (m, 1H, CF₃CH(NAc)CH₂); 3,01 (s, 3H, SO₂CH₃); 3,52-3,63 (m, 1 H, NCH₂CH); 3,83 (d, J= 10,3 Hz, 5,4 Hz, 1 H, NCH₂); 4,15 (d, J= 10,3 Hz, 10,0 Hz, 1 H, NCH₂); 4,80-4,95 (m, 1 H, CF₃CH); 7,29 (d, J= 8,6 Hz, 1 H, H_{Ar}(HC=CNSO₂)); 7,41 (d, J= 8,6 Hz, 1 H, H_{Ar}(HC=CBr)); 7,66 (s, 1 H, H_{Ar}(HC=CBr)); 7,76 (d, J= 9,4 Hz, 1 H, NH).

RMN¹³C (δ, ppm)(CD₃)₂CO, 400 MHz)

22,76 (CH₃CO); 33,25 (CF₃CH(NAc)CH₂); 34,82 (NCH₂); 37,40 (CH₃SO₂); 49,28 (q, J= 30 Hz, CF₃CH); 57,08 (NCH₂); 115,83 (CH_{Ar.}); 116,26 (Cq_{Ar.}); 126 ,42 (q, J= 281 Hz, CF₃); 129,51 (CH_{Ar.}); 132,12 (CH_{Ar.}); 137,35 (Cq_{Ar.}); 142,52 (Cq_{Ar.}); 170,72 (C=O).

### Second diastéréoisomère (minoritaire)

RMN¹H (δ, ppm) (CD₃)₂CO, 400 MHz)

1,96-2,07 (m, 1 H, CF₃CH(NAc)CH₂); 2,03 (s, 3H, COCH₃); 2,20-2,27 (m, 1 H, CF₃CH(NAc)CH₂); 3,02 (s, 3H, SO₂CH₃); 3,57-3,66 (m, 1 H, NCH₂CH); 3,82 (d, J= 10,0 Hz, 7,5 Hz, 1 H, NCH₂); 4,11 (d, J= 10,0 Hz, 9,5 Hz, 1 H, NCH₂); 4,74-4,87 (m, 1 H, CF₃CH); 7,29 (d, J= 8,5 Hz, 1 H, H_{Ar}(HC=CNSO₂)); 7,40 (d, J= 8,5 Hz, 1 H, H_{Ar}(HC=CBr)); 7,41 (s, 1 H, H_{Ar}(HC=CBr)); 7,67 (d, J= 9,3 Hz, 1 H, NH).

RMN¹³C (δ, ppm) (CD₃)₂CO, 400 MHz)

22,77 (CH₃CO); 32,87 (CF₃CH(NAc)CH₂); 34,70 (NCH₂); 36,71 (CH₃SO₂); 49,04 (q, J= 30 Hz, CF₃CH); 56,42 (NCH₂); 115,86 (CH_{Ar.}); 116,22 (Cq_{Ar.}); 126 ,50 (q, J= 281 Hz, CF₃); 128,38 (CH_{Ar.}); 131,99 (CH_{Ar.}); 137,83 (Cq_{Ar.}); 142,79 (Cq_{Ar.}); 170,87 (C=O).

### Analyses sur le mélange de diastéréoisomères

IR (v, cm⁻¹) (nujol)

3285 (NH); 1759 (C=O); 1549; 1352;1307; 1269; 1214; 1161; 1128; 1112.

Masse (IC, NH₃)

430 (MH⁺); 447 (MNH₄⁺).

| Microanalyse | Elément: | Carbone | Hydrogène |
|---|---|---|---|
| | Calculé (%) | 39,17 | 3,76 |
| | Trouvé (%) | 38,73 | 3,69 |

### Exemple 24 :

### Ester de l'acide S-{2-[5-(1-acétylamino-2,2,2-trifluoro-éthyl)-2-oxo-[1,3]dioxolan-4-yl]-1-triméthylsilanylméthyl-éthyl} dithiocarbonique ester O-éthylique

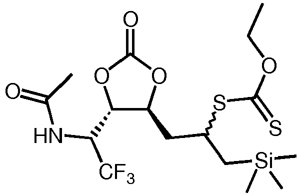

C₁₆H₂₆F₃NO₅S₂Si M= 461,59 g.mol⁻¹

### Réaction :

Effectuée selon le mode opératoire général avec 130 mg (0,37 mmol) de xanthate de l'exemple 4 et 178 µl (1,12 mmol) d'allyl-triméthyl-silane dans 1 ml de 1,2-dichloroéthane. La réaction est terminée après addition de 5% de DLP et 45 minutes de reflux.

### Purification :

Chromatographie sur gel de silice (éther-éther de pétrole 3/7).

### Produit :

Huile jaune pâle.

### Rendement :

72% (2 diastéréoisomères dans un rapport 1/1)

RMN¹H (δ, ppm) (CDCl₃, 400 MHz)

0,07 (s, 9H, (CH₃)₃Si); 0,08 (s, 9H, (CH₃)₃Si); 0,96-1,15 (m, 4H, CH₂Si(CH₃)₃); 1,41 (t, J= 7,0 Hz, 3H, CH₂CH₃); 1,42 (t, J= 7,0 Hz, 3H, CH₂CH₃); 2,12 (s, 6H, 2 x COCH₃); 2,14-2,35 (m, 4H, 2 x OCHCH₂CHS); 3,83-3,90 (m, 1 H, CHS); 3,90-3,97 (m, 1 H, CHS); 4,54-4,62 (m, 2H, 2 x CHSCH₂CHO); 4,63 (q, J= 7,0 Hz, 2H, CH₃CH₂); 4,64 (q, J= 7,0 Hz, 2H, CH₃CH₂); 4,75 (d, 1 H, J= 5,8 Hz, 1 H, CF₃CHCH(NAc)CHO); 4,84 (d, 1 H, J= 6,58 Hz, 1 H, CF₃CHCH(NAc)CHO); 4,93-5,04 (m, 2H, CF₃CH); 7,45 (d, J= 10,5 Hz, 1 H, NH); 7,47 (d, J= 10,5 Hz, 1 H, NH).

RMN¹³C (δ, ppm) (CDCl₃, 100 MHz)

-0,79 à -0,65 (m, (CH₃)₃Si); 13,81 (2 x CH₃CH₂); 20,99 (OCHCH₂CHS); 22,57 (2 x CH₃CO); 22,92 (OCHCH₂CHS); 41,29 (CH₂Si); 42,12 (CH₂Si); 43,72 (CHS); 44,33 (CHS); 50,70 (q, J= 32 Hz, 2 x CF₃CH); 70,25 (CH₂CH₃); 70,29 (CH₂CH₃); 76,75 (CHOC=O); 77,19 (CHOC=O);77,83 (CHOC=O); 77,99 (CHOC=O); 123,45 (q, J= 283 Hz, 2 x CF₃); 154,02 (OC=O); 154,13 (OC=O); 171,66 (NC=O); 171,83 (NC=O); 213,11 (C=S); 213,22 (C=S).

IR (v, cm⁻¹) (CCl₄)

3435 (NH); 3328 (NH); 2954; 1807 (C=O); 1697 (C=O); 1502; 1371; 1301; 1277; 1251; 1221; 1189; 1142; 1111; 1050.

Masse (IC, NH₃)

340 (M-HSCSOEt+H⁺); 463 (MH⁺); 480 (MNH₄⁺).

### Exemple 25 :

### N-[1-(5-éthoxy-2-oxo-[1,3]dithiolan-4-ylméthyl)-2,2,2-trifluoro-éthyl]-acétamide

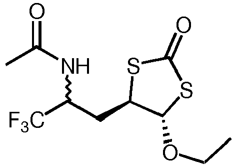

C₁₀H₁₄F₃NO₃S₂ M= 317,35 g.mol⁻¹

### Réaction :

A une solution du xanthate adduit de l'exemple 8 dans 10 ml de méthanol sont additionnées 4 gouttes d'acide sulfurique concentré. Après 48h à température ambiante le méthanol est évaporé sous pression réduite et le résidu repris dans du dichlorométhane. Une solution saturée d'hydrogénocarbonate de sodium est ajoutée et la phase organique est séparée. Après séchage sur sulfate de magnésium, filtration et concentration sous vide, le résidu organique est alors purifié.

### Purification :

Chromatographie sur gel de silice (acétate d'éthyle-éther de pétrole 3/7).

### Produit :

Huile jaune pâle.

### Rendement :

21%

RMN¹H (δ, ppm) (CDCl₃, 400 MHz)

1,24 (t, J= 7,0 Hz, 3H, CH₂CH₃); 1,27 (t, J= 7,0 Hz, 3H, CH₂CH₃); 2,02-2,08 (m, 2H, CF₃CH(NAc)CH₂); 2,07 (s, 3H, COCH₃); 2,08 (s, 3H, COCH₃); 2,45 (ddd, 1 H, J= 14,6 Hz, 8,2 Hz, 4,1 Hz, 1 H, CF₃CH(NAc)CH₂); 2,57 (ddd, 1 H, J= 14,6 Hz, 10,0 Hz, 2,9 Hz, 1 H, CF₃CH(NAc)CH₂); 3,44-3,54 (m, 2H, CH₂CH₃); 3,70-3,84 (m, 2H, CH₂CH₃); 4,03-4,07 (m, 1 H, CH₂CHS); 4,30-4,75 (m, 1 H, CH₂CHS); 4,67-4,88 (m, 2H, CF₃CH); 5,52 (d, J= 1,9 Hz, 1 H, SCH(OEt)); 5,60 (d, J= 3,5 Hz, 1 H, SCH(OEt)); 6,59 (d, J= 10,0 Hz, 1 H, NH); 6,63 (d, J= 9,4 Hz, 1 H, NH).

RMN¹³C (δ, ppm) (CDCl₃, 100 MHz) 14,45 (CH₂CH₃); 14,66 (CH₂CH₃); 22,97 (CH₃CO); 23,07 (CH₃CO); 28,25 (CH₂CHS); 33,31 (CH₂CHS); 47,95 (q, J= 30 Hz, CF₃CH); 48,87 (q, J= 30 Hz, CF₃CH); 53,21 (CH₂CHS); 54,49 (CH₂CHS); 65,68 (CH₂CH₃); 65,92 (CH₂CH₃); 90,58 (SCH(OEt)); 94,07 (SCH(OEt)); 123,51 (q, J= 282 Hz, CF₃); 123,58 (q, J= 282 Hz, CF₃); 170,85 (NC=O); 171,14 (NC=O); 195,48 (SC=O); 195,67 (SC=O).

IR (v, cm⁻¹) (CCl₄)

3439 (NH); 2981; 1701 (C=O); 1666 (C=O); 1549; 1500; 1239; 1192; 1144.

Masse (IC, NH₃)

272 (M-EtOH+H⁺); 318 (MH⁺); 335 (MNH₄⁺).

### Exemple 26 :

### Ester 4-benzoylamino-5,5-5-trifluoro-butylique de l'acide acétique

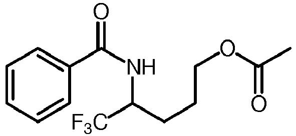

C₁₄H₁₆F₃NO₃ M= 303,11 g.mol⁻¹

### Réaction :

A une solution, préalablement dégazée au reflux sous argon, du xanthate adduit de l'exemple 15 (551 mg, 1,30 mmol) dans le propan-2-ol (8 mL), est additionné du DLP à raison de 10 % (52 mg, 0,13 mmol) toutes les heures. La réaction est arrêtée après 11 h de reflux et addition de 120% de DLP (6,2 g, 15,5 mmol). Le milieu réactionnel est alors ramené à température ambiante et concentré sous pression réduite avant d'être purifié.

### Purification :

Chromatographie sur gel de silice (acétate d'éthyle-éther de pétrole 1/9).

### Produit :

Cristaux translucides.

### Rendement :

62%

RMN¹H (δ, ppm) (CDCl₃, 400 MHz)

1,6 (quin, *J* = 7,2Hz, 2H, CH₂CH₂CH₂) ; 2,01 (s, 3H, COC*H*₃) ; 2,23 (t, *J* = 8,3Hz, 2H, CF₃CHNC*H*₂) ; 4,05 (t, *J* = 8,8Hz, 2H, C*H*₂OCOCH3); 4,85 (m, 1 H, CF₃C*H*); 6,34 (d, *J* = 8,9 Hz, 1 H, N*H*) ; 7,41-7,92 (m, 5H, CH_{Ar}.).

RMN¹³C (δ, ppm) (CDCl₃, 100 MHz)

22,8 (*C*H₃CO); 28,4 (q, *J* = 30 Hz, CF₃*C*H); 33,2 (CF₃CHN*C*H₂); 31,8 (CF₃CHN*C*H₂) ; 63,7 (*C*H₂OCOCH₃); 124,7 (q, *J* = 281 Hz, *C*F₃); 128,5 (2x*C*H_{Ar}.) ; 128,9 (2x*C*HAr.) ; 129,2 (*C*H_{Ar}.) ; 132,4 (*C*q_{Ar}.); 179,6 (*C*=O); 180,0 (*C*=O).

IR (v, cm⁻¹) (CCl₄)

3451 (NH); 2926; 2881; 1710 (C=O); 1688 (C=O); 1510; 1486; 1455; 1367; 1236; 1187; 1147; 1072.

Masse (IC, NH3)

304 (MH⁺); 321 (MNH₄⁺).

| Microanalyse | Elément | Carbone | Hydrogène |
|---|---|---|---|
| | Calculé (%) | 55,44 | 5,32 |
| | Trouvé (% | 55,27 | 5,18 |

Tf

49 °C (éther)

### EXEMPLES DE PROCEDE DE PREPARATION

### DES COMPOSES DE FORMULE (VIIIA)

### Exemple 27 :

### N-(2-acétylamino-3,3,3-trifluoro-1-trifluorométhyl-propyl)-acétamide

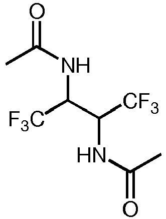

C₈H₁₀F₆N₂O₂ M= 280,17 g.mol⁻¹

### Réaction :

A une solution, préalablement dégazée au reflux sous argon, du xanthate de l'exemple 1 (522 mg, 2 mmol) dans 16 ml de chlorobenzène est additionné du DLP à raison de 10 mol% (80mg, 0,2 mmol) toutes les 10 minutes. La réaction est arrêtée après 2 heures de reflux et addition de 120% de DLP (960 mg, 2,4 mmol). Le milieu réactionnel est alors ramené à température ambiante et concentré sous pression réduite avant d'être purifié

### Purification :

Par précipitation dans un mélange éther-éther de pétrole 1/9.

### Produit :

Solide incolore.

### Rendement :

79% (2 diastéréoisomères dans un rapport 7/5)

### Premier diastéréoisomère (majoritaire)

RMN¹H (δ, ppm) ((CD₃)₂SO, 400 MHz)

1,90 (s, 6H, CH₃CO); 4,90-5,03 (m, 2H, CF₃CH); 8,86 (d, J= 8,7 Hz, 2H, NH).

RMN¹³C (δ, ppm) ((CD₃)₂SO, 400 MHz)

22,21 (2 x CH₃CO); 47,77 (q, J= 29 Hz, 2 x CF₃CH); 124,01 (q, J= 282 Hz, 2 x CF₃); 169,61 (2 x NC=O).

### Second diastéréoisomère (minoritaire)

RMN¹H (δ, ppm) ((CD₃)₂SO, 400 MHz)

1,98 (s, 6H, CH₃CO); 5,17 (qd, J= 8,2 Hz, 9,2 Hz, 2H, CF₃CH); 8,41 (d, J= 9,2 Hz, 2H, NH).

RMN¹³C (δ, ppm) ((CD₃)₂SO, 400 MHz)

22,40 (2 x CH₃CO); 47,19 (q, J= 29 Hz, 2 x CF₃CH); 123,89 (q, J= 282 Hz, 2 x CF₃); 169,54 (2 x NC=O).

Analyses sur le mélange de diastéréoisomères

IR (v, cm⁻¹) (nujol)

3284 (NH); 3072; 1674 (C=O); 1547; 1340; 1301; 1255; 1238; 1181; 1153; 1108.

Masse (IC, NH₃)

281 (MH⁺); 298 (MNH₄⁺).

| Microanalyse | Elément: | Carbone | Hydrogène |
|---|---|---|---|
| | Calculé (%) | 34,30 | 3,60 |
| | Trouvé (%) | 33,67 | 3,63 |

### EXEMPLE DE PROCEDE DE PREPARATION DE COMPOSES DE FORMULE (I_{B}) SYNTHESE DU XANTHATE 2-TRIFLUOROMETHYLETHANOL

### Exemple 28 :

Ester de O-éthyle et de S-(1-hydroxy-2,2,2-trifluoro-éthyl) de l'acide dithiocarbonique

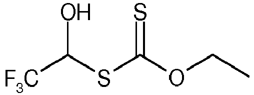

C₅H₇F₃O₂S₂ M= 220,23 g.mol⁻¹

### Réaction :

A une solution de 2,2,2-trifluoro-1-méthoxy-éthanol (300 mg, 2,31 mmol) dans l'acétone (5 mL) est ajouté le sel d'éthylxanthogénate de potassium (737 mg, 4,62 mmol). Le mélange réactionnel est refroidi à 0°C et de l'acide sulfurique (123,6 µL, 2,31 mmol) est ajouté goutte à goutte. Après une heure à 0°C, le mélange réactionnel est concentré sous pression réduite. Le résidu est repris à l'éther, filtré et concentré à nouveau sous pression réduite.

### Produit :

Huile jaune.

### Rendement :

69%

RMN¹H (δ, ppm)(CDCl₃, 400 MHz)

1,54 (t, *J* = 6,3 Hz, 3H, CH₂C*H*₃); 4,71 (q, *J* = 6,3 Hz, 2H, CH₃C*H*₂); 6,02 (q, *J* = 5,7 Hz, 1 H, CF₃C*H*).

RMN¹³C (δ, ppm)(CDCl₃, 100 MHz)

13,7 (*C*H₃CH₂); 57,8 (q, *J* = 30 Hz, CF₃*C*H); 71,4 (*C*H₂CH₃); 124,4 (q, *J* = 280 Hz, *C*F₃); 207,1 (*C*=S).

IR (ν, cm⁻¹)(CCl₄)

2961; 1453; 1314; 1231; 1130; 1052; 1023.

Masse (IC, NH3)

221 (MH⁺), 238 (MNH₄⁺).

### Exemple 29 :

### Ester de O-éthyle et de S-(1-acétoxy-2,2,2-trifluoro-éthyle) de l'acide dithiocarbonique

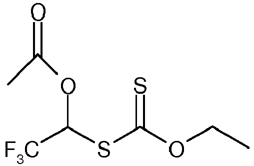

C₇H₉F₃O₃S₂ M= 262,27 g.mol⁻¹

### Réaction :

A une solution de 2,2,2-trifluoro-1-méthoxy-éthanol (3 g, 23,1 mmol) dans l'acétone (15 mL) est ajouté le sel d'éthylxanthogénate de potassium (4,61 g, 46,2 mmol). Le mélange réactionnel est refroidi à 0°C et de l'acide sulfurique (1,24 mL, 23,1 mmol) est ajouté goutte à goutte. Après une heure à 0°C, le mélange réactionnel est concentré sous pression réduite. Le résidu est repris dans l'éther, filtré et concentré à nouveau sous pression réduite.

Le résidu est repris dans l'acétone (5mL), refroidi à 0°C. Sont additionnés goutte à goutte de l'acide sulfurique (1,24 mL, 23,1 mmol), puis de l'anhydride acétique (21,7 mL, 231 mmol).

Après une heure à 0°C, le mélange réactionnel est concentré sous pression réduite, repris dans l'éther, lavé à l'eau puis avec une solution aqueuse saturée en carbonate de potassium, séché et concentré à nouveau sous pression réduite.

### Purification :

Chromatographie sur gel de silice (acétate d'éthyle-éther de pétrole 1/9)

### Produit :

Huile jaune.

### Rendement :

60%

RMN¹H (δ, ppm) (CDCl₃, 400 MHz)

1,48 (t, *J* = 5,8 Hz, 3H, CH₂*C*H₃); 2,21 (s, 3H, COC*H*₃); 4,71 (q, *J* = 5,8 Hz, 2H, CH₃C*H*₂); 7,32 (q, *J* = 6,0 Hz, 1 H, CF₃C*H*).

RMN¹³C (δ, ppm) (CDCl₃, 100 MHz)

12,5 (*C*H₃CH₂); 21,0 (CO*C*H₃); 57,6 (q, *J* = 38 Hz, CF₃*C*H); 70,4 (*C*H₂CH₃); 123,0 (q, *J* = 280 Hz, *C*F₃); 179,5 (*C*=O); 206,9 (C=S).

IR (v, cm⁻¹) (CCl₄)

2986; 2939; 1778 (*C*=O); 1442; 1370; 1344; 1194; 1131; 1032; 868; 823.

Masse (IC, NH3)

262 (MH⁺), 279 (MNH₄⁺).

### Exemple 30 :

### Ester de 1-éthoxythiocarbonylsulfanyl-2,2,2-trifluoro-éthyle de l'acide benzoïque

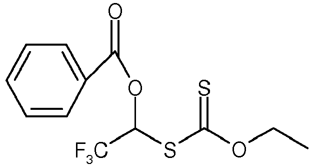

C₁₂H₁₁F₃O₃S₂ M= 324,34 g.mol⁻¹

### Réaction :

A une solution de 2,2,2-trifluoro-1-méthoxy-éthanol (3 g, 23,1 mmol) dans l'acétone (15 mL) est ajouté le sel d'éthylxanthogénate de potassium (4,61 g, 46,2 mmol). Le mélange réactionnel est refroidi à 0°C et l'acide sulfurique (1,24 mL, 23,1 mmol) est ajouté goutte à goutte. Après une heure à 0°C, le mélange réactionnel est concentré sous pression réduite. Le résidu est repris dans l'éther, lavé à l'eau, puis avec une solution aqueuse saturée en carbonate de potassium, séché et concentré à nouveau sous pression réduite.

Ce résidu est alors repris dans le dichlorométhane (5 mL) et refroidi à 0°C. Sont additionnés l'anhydride benzoique (6,27 g, 27,7 mmol), le DMAP (4-diméthylaminopyridine) (845mg, 6,93 mmol), et la triéthylamine (5,51 mL, 39,3 mmol) goutte à goutte.

Après 3 heures à 0°C, le mélange réactionnel est concentré sous pression réduite, repris dans le dichlorométhane, lavé à l'eau puis avec une solution aqueuse saturée en chlorure d'ammonium, séché et concentré à nouveau sous pression réduite.

### Purification :

Chromatographie sur gel de silice (acétate d'éthyle-éther de pétrole 5/95)

### Produit :

Huile jaune foncé.

### Rendement :

58%

RMN¹H (δ, ppm) (CDCl₃, 400 MHz) 1,30 (t, *J =* 6,2 Hz, 3H, CH₂C*H*₃); 3,86-3,91 (m, 1 H, CH₃C*H*₂); 3,96-4,01 (m, 1 H, CH₃C*H*₂); 6,28 (q, *J* = 4,0 Hz, 1 H, CF₃C*H*) 7,50 (t, *J* = 8,0 Hz, 2H_{Ar}, CH_{Ar}=C*H*_{Ar}=CH_{Ar}=C_{qAr},) ; 7,65 (t, *J* = 7,6 Hz, 1HA_{r,} C*H*_{Ar}=CH_{Ar}=CH_{Ar}C_{qAr}) ; 8,13 (t, 2H_{Ar}, *J* = 7,2 Hz, CH_{Ar}=CH_{Ar}=C*H*_{Ar}C_{qAr}).

RMN¹³C (δ, ppm) (CDCl₃, 100 MHz)

14,3 (*C*H₃CH₂); 68,5 (*C*H₂CH₃); 90,0 (q, *J* = 39 Hz, CF₃*C*H); 120,0 (q, *J* = 279 Hz, *C*F₃); 127,3 (2x*C*H_{Ar}); 128,2 (2x*C*H_{Ar}); 129,8 (*C*H_{Ar}) ; 133,5 (*C*_{qAr}); 164,3 (*C*=O); 214,1 (*C*=S).

IR (v, cm⁻¹) (CCl₄)

2984; 2929; 1739 (*C*=O); 1601; 1452; 1290; 1262; 1193; 1169 (C=S); 1113; 1078; 1062; 1025; 988.

Masse (IC, NH3)

325 (MH⁺), 342 (MNH₄⁺).

### ADDITIONS RADICALAIRES

### EXEMPLES DE PROCEDE DE PREPARATION

### DE COMPOSES DE FORMULE (IIB)

### Exemple 31 :

### Diester de O-éthyle et de S-[3-acétyl-1-(1,3-dioxo-1,3-dihydro-isoindol-2-ylméthyl)-4,4,4-trifluoro-butyle] de l'acide dithiocarbonique

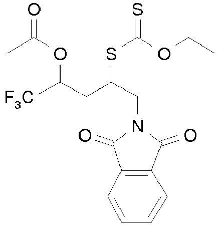

C₁₈H₁₈F₃NO₅S₂ M= 449,47 g.mol⁻¹

### Réaction :

Effectuée selon le mode opératoire général avec 337 mg (1,29 mmol) de xanthate de l'exemple 29 et 481 mg (2,57 mmol) d'allyl phtalimide dans le 1,2-dichloroéthane (3 mL). La réaction est terminée après addition de 15% de DLP (26 mg) et 4h30 de reflux.

### Purification :

Chromatographie sur gel de silice (acétate d'éthyle-éther de pétrole 1/9 puis 2/8).

### Produit :

Huile jaune pâle.

### Rendement :

78% (mélange de 2 diastéréoisomères dans un rapport 1/1)

RMN¹H (δ, ppm) (CDCl₃, 400 MHz)

1,22 (2t, *J* = 7,0 Hz, 3H, CH₂C*H*₃); 1,38-1,42 (m, 2H, CF₃CH(OAc)C*H*₂); 2,18 (s, 1,5H, COC*H*₃); 2,22 (s, 1,5H, COC*H*₃); 3,98-4,02 (m, 2H, C*H*₂N); 4,21-4,24 (m, 0,5H, C*H*S); 4,52 (q, *J* = 7,0 Hz, 1H, CH₃C*H*₂); 4,54 (q, *J* = 7,0 Hz, 1 H, CH₃C*H*₂); 4,54-4,57 (m, 0,5H, C*H*S); 5,63-5,66 (m, 1 H, CF₃C*H*); 7,70-7,73 (m, 2H, *H*_{Ar.}(C_{q}CH=C*H*); 7,79-7,84 (m, 2H, *H*_{Ar.}(C_{q}C*H*=CH)).

RMN¹³C (δ, ppm) (CDCl₃, 100 MHz)

12,2 (0,5x*C*H₃CH₂); 12,3 (0,5x*C*H₃CH₂); 20,0 (0,5x*C*H₃CO); 21, (0,5x*C*H₃CO); 30,2 (0,5xCF₃CH(OAc)*C*H₂); 30,4 (0,5xCF₃CH(OAc)*C*H₂); 39,8 (0,5x*C*H₂N); 40,2 (0,5x*C*H₂N); 43,8 (0,5xCHS); 44,1 (0,5xCHS); 63,5 (q, *J* = 35 Hz, 0,5xCF₃*C*H); 63,7 (q, *J* = 35 Hz, 0,5xCF₃*C*H); 69,9 (0,5x*C*H₂CH₃); 69,9 (0,5x*C*H₂CH₃); 121,6 (*C*H_{Ar.}(C_{q}CH=*C*H)); 121,6 (*C*H_{Ar.}(C_{q}CH=*C*H)); 124,6 (q, *J* = 272 Hz, 0,5x*C*F₃); 124,9 (q, *J* = 280 Hz, 0,5x*C*F₃); 130,8 (*Cq*_{Ar.}); 132,2 (*Cq*_{Ar.}); 165,3 (2x*C*H_{Ar.}(C_{q}*C*H=CH)); 167,0 (*C*=O_{Ar}.); 167,0 (*C*=O_{Ar}.); 168,0 (0,5x*C*=O); 168,0 (0,5x*C*=O); 212,0 (0,5xC=S); 212,5 (0,5xC=S).

IR (v, cm⁻¹) (CCl₄)

2982; 2928; 1766 (C=O); 1723 (C=O); 1615; 1468; 1430; 1392; 1371; 1283; 1213; 1146; 1112; 1049.

Masse (IC, NH3)

450 (MH⁺); 467 (MNH₄⁺).

| Microanalyse | Elément: | Carbone | Hydrogène |
|---|---|---|---|
| | Calculé (%) | 48,10 | 4,04 |
| | Trouvé (%) | 48,06 | 4,09 |

### Exemple 32 :

### Ester de O-éthyle et de S-(3-acétoxy-4,4,4-trifluoro-1-triméthyl-silanylméthyl-butyle)de l'acide dithiocarbonique

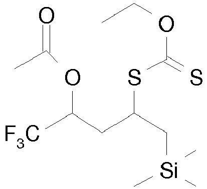

C₁₃H₂₃F₃NO₃S₂Si M= 376,54 g.mol⁻¹

### Réaction :

Effectuée selon le mode opératoire général avec 300 mg (1,14 mmol) de xanthate de l'exemple 29 et 392 mg (3,43 mmol) d'allyltriméthylsilane dans le 1,2-dichloroéthane (4 mL). La réaction est terminée après addition de 5% de DLP (22 mg) et 1 h45 de reflux.

### Purification :

Chromatographie sur gel de silice (acétate d'éthyle-éther de pétrole 1/9 puis 2/8).

### Produit :

Cristaux blancs.

### Rendement :

80% (mélange de 2 diastéréoisomères dans un rapport 2/3)

RMN¹H (δ, ppm) (CDCl₃, 400 MHz)

0,08 (s, 3,6H, Si(C*H*₃)₃); 0,09 (s, 5,4H, Si(C*H*₃)₃); 0,83-1,15 (m, 2H, C*H*₂Si(CH₃)₃); 1,42 (t, *J* = 6,4 Hz, 3H, CH₂C*H*₃); 2,03-2,26 (m, 2H, CF₃CH(OAc)C*H*₂); 2,15 (s, 1,2H, COC*H*₃); 2,17 (s, 1,8H, COC*H*₃); 3,72 (m, 0,4H, C*H*S); 3,95 (m, 0,6H, C*H*S); 4,65 (q, *J* = 6,4 Hz, 2H, CH₃C*H*₂); 5,39-5,45 (m, 0,4H, CF₃C*H*); 5,48-5,52 (m, 0,6H, CF₃C*H*).

RMN¹³C (δ, ppm) (CDCl₃, 100 MHz) -0,7 (1,2xSi(*C*H₃)₃); -0,7 (1,8xSi(*C*H₃)₃); 13,8 (0,4x*C*H₃CH₂); 13,8 (0,6x*C*H₃CH₂); 19,7 (0,4x*C*H₂OAcCHCF₃); 19,8 (0,6x*C*H₂OAcCHCF₃); 22,1 (0,4x*C*H₃CO); 22,5 (0,6x*C*H₃CO); 34,9 (0,4x*C*H₂SiMe₃); 35,2 (0,6x*C*H₂SiMe₃); 43,4 (0,4xCHS); 43,7 (0,6x*C*HS); 68,1 (q, *J* = 32 Hz, 0,4xCF₃*C*H); 68,2 (q, *J* = 32 Hz, 0,6xCF₃*C*H); 70,3 (0,4x*C*H₂CH₃); 70,5 (0,6x*C*H₂CH₃); 122,3 (q, *J* = 281 Hz, 0,4x*C*F₃); 125,4 (q, *J* = 281 Hz, 0,6x*C*F₃); 170,1 (0,4x*C*=O); 170,3 (0,6x*C*=O); 212,1 (0,4xC=S); 213,2 (0,6xC=S).

IR (ν, cm⁻¹)(CCl₄)

2956; 2927; 2855; 2355; 1764 (C=O); 1441; 1371; 1285; 1268; 1214; 1182; 1140; 1112; 979; 938.

Masse (IC, NH3)

377 (MH⁺); 394 (MNH₄⁺).

Tf

120 °C (éther)

### Exemple 33 : Ester de 3-acétoxy-1-éthoxythiocarbonylsulfanyl-4,4,4-trifluoro-butyle de l'acide acétique

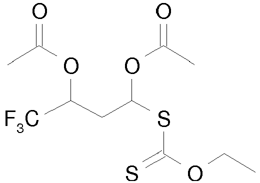

C₁₁H₁₅F₃O₅S₂ M= 348,36 g.mol⁻¹

Réaction : Effectuée selon le mode opératoire général avec 200 mg (0,77 mmol) de xanthate de l'exemple 29 et 77 µL (0,92 mmol) d'acétate de vinyle dans le 1,2-dichloroéthane (1,5 mL). La réaction est terminée après addition de 5% de DLP (15 mg) et 1 h30 de reflux.

### Purification :

Chromatographie sur gel de silice (acétate d'éthyle-éther de pétrole 1/9).

### Produit :

Huile épaisse jaune pâle.

### Rendement :

79% (mélange de 2 diastéréoisomères dans un rapport 1/1)

RMN¹H (δ, ppm) (CDCl₃, 400 MHz)

1,48 (t, *J* = 6,7 Hz, 1,5H, CH₂C*H*₃); 1,49 (t, *J* = 6,7 Hz, 1,5H, CH₂*C*H₃); 2,12 (s, 3H, COC*H*₃); 2,23 (s, 3H, COC*H*₃); 2,39-2,46 (m, 1 H, CF₃CH(OAc)C*H*₂); 2,49-2,52 (m, 1 H, CF₃CH(OAc)C*H*₂); 4,60 (q, *J* = 6,7 Hz, 1 H, CH₃C*H*₂); 4,61 (q, *J* = 6,7 Hz, 1 H, CH₃C*H*₂); 5,43-5,48 (m, 0,5H, CF₃*C*H); 5,49-5,52 (m, 0,5H, CF₃*C*H); 6,68-6,73 (m, 0,5H, CHS); 6,79-6,84 (m, 0,5H, CHS).

RMN¹³C (δ, ppm) (CDCl₃, 100 MHz)

13,4 (0,5x*C*H₃CH₂); 13,6 (0,5x*C*H₃CH₂); 20,8 (0,5x*C*H₃CO); 20,9 (0,5x*C*H₃CO); 22,9 (0,5x*C*H₃CO); 23,1 (0,5x*C*H₃CO); 33,1 (0,5x*C*H₂CHS); 33,5 (0,5x*C*H₂CHS); 47,6 (q, *J* = 32 Hz, 0,5xCF₃*C*H); 47,8 (q, *J* = 32 Hz, 0,5xCF₃*C*H); 70,5 +(0,5x*C*H₂CH₃); 70,7 (0,5x*C*H₂CH₃); 77,2 (0,5xCHS); 77,9 (0,5xCHS); 125,2 (q, *J* = 281 Hz, 0,5x*C*F₃); 125,7 (q, *J* = 281 Hz, 0,5x*C*F₃); 168,9 (0,5xO*C*=O); 169,8 (0,5xO*C*=O); 170,6 (0,5x*C*=O); 170,8 (0,5x*C*=O); 209,2 (0,5xC=S); 209,7 (0,5xC=S).

IR (v, cm⁻¹) (CCl₄)

3511; 2983; 2938; 2869; 2412; 1764 (C=O); 1720 (C=O); 1641; 1430; 1398; 1371; 1284; 1228; 1146; 1111; 1086; 1048; 1016.

Masse (IC, NH3)

289 (M H⁺-AcOH); 349 (MH⁺); 366 (MNH₄⁺).

### Exemple 34 : Diester de O-éthyle et de S-(acétoxy-1-diéthoxyméthyl-4,4,4-trifluoro-pentyle) de l'acide dithiocarbonique

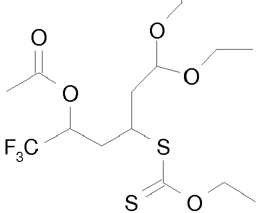

C₁₅H₂₅F₃O₅S₂ M= 406,49 g.mol⁻¹

### Réaction :

Effectuée selon le mode opératoire général avec 200 mg (0,76 mmol) de xanthate de l'exemple 29 et 220 mg (1,5 mmol) de 4,4-diéthoxy-butène dans le 1,2-dichloroéthane (2 mL). La réaction est terminée après addition de 5% de DLP (15 mg) et 1h30 de reflux.

### Purification :

Chromatographie sur gel de silice (acétate d'éthyle-éther de pétrole 1/9).

### Produit :

Huile épaisse jaune pâle.

### Rendement :

82% (mélange de 2 diastéréoisomères dans un rapport 1/1)

RMN¹H (δ, ppm) (CDCl₃, 400 MHz)

1,20 (t, *J =* 7,0 Hz, 6H, CH(OCH₂C*H*₃)₂); 1,25 (t, *J =* 7,0 Hz, 3H, CH₂C*H*₃); 1,42 (dd, *J₁* = 7,5 Hz*, J₂* = 9,1 Hz, 2H, CH-C*H₂*-CH(OCH₂CH₃)₂); 2,25-2,28 (m, 2H, CF₃CH(OAc)C*H₂*); 3,51-3,55 (m, 2H, CH₃C*H*₂O); 3,59-3,65 (m, 2H, CH₃C*H*₂O); 4,62-4,67 (m, 2H, CH₃C*H*₂O); 5,05 (dd, *J*₁ = 9,2 Hz*, J₂* = 12,0 Hz, 0,5H, C*H*(OEt)₂); 5,12 (dd, *J*₁ = 9,2 Hz*, J₂* = 12,0 Hz, 0,5H, C*H*(OEt)₂); 5,47-5,52 (m, 0,5H, C*H*S); 5,61-5,64 (m, 0,5H, CHS); 5,89-5,93 (m, 1 H, CF₃C*H*).

RMN¹³C (δ, ppm) (CDCl₃, 100 MHz)

13,9 (*C*H₃CH₂); 14,5 (*C*H₃CH₂); 16,2 (0,5x*C*H₃CH₂); 17,2 (0,5x*C*H₃CH₂); 21,0 (0,5xCH-*C*H₂-CH(OCH₂CH₃)₂)); 21,2 (0,5xCH-*C*H₂-CH(OCH₂CH₃)₂)); 24,3 (0,5x*C*H₃CO); 24,5 (0,5x*C*H₃CO); 30,2 (0,5xCF₃CH(OAc)*C*H₂); 30,6 (0,5xCF₃CH(OAc)*C*H₂); 43,1 (q, *J* = 30 Hz, 0,5xCF₃*C*H); 43,5 (q, *J* = 30 Hz, 0,5xCF₃*C*H); 51,2 (0,5x*C*HS); 51,5 (0,5xCHS); 61,8 (0,5x*C*H₂CH₃); 62,1 (0,5x*C*H₂CH₃); 64,0 (0,5x*C*H₂CH₃); 64,2 (0,5x*C*H₂CH₃); 69,7 (0,5x*C*H₂CH₃); 69,8 (0,5x*C*H₂CH₃); 100,9 (0,5x*C*H(OEt)₂); 101,3 (0,5x*C*H(OEt)₂); 122,9 (q, *J* = 283 Hz, 0,5x*C*F₃); 123,1 (q, *J* = 283 Hz, 0,5x*C*F₃); 158,3 (0,5x*C*=O); 158,6 (0,5x*C*=O); 214,1 (0,5xC=S); 214,4 (0,5xC=S).

IR (ν, cm⁻¹)(CCl₄)

2978; 2928; 2336; 1764 (C=O); 1442; 1372; 1283; 1215; 1182; 1142; 1112; 1052. Masse (IC, NH3)

363 (MH+-EtOH).

### Exemple 35 : Ester de O-éthyle et de S-(3-acétoxy-1-cyanométhyl-4,4,4-trifluoro)butyle de l'acide dithiocarbonique

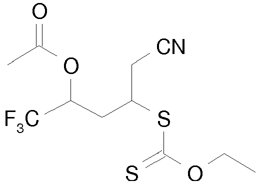

C₁₁H₁₄F₃NO₃S₂ M= 329,36 g.mol⁻¹

### Réaction :

Effectuée selon le mode opératoire général avec 200 mg (0,77 mmol) de xanthate de l'exemple 29 et 109 µL (2,31 mmol) de but-3-ènenitrile dans le 1,2-dichloroéthane (2 mL). La réaction est terminée après addition de 10% de DLP (31 mg) et 3h de reflux.

### Purification :

Chromatographie sur gel de silice (acétate d'éthyle-éther de pétrole 5/95).

### Produit :

Huile épaisse jaune pâle.

### Rendement :

92% (mélange de 2 diastéréoisomères dans un rapport 3/2)

RMN¹H (δ, ppm) (CDCl₃, 400 MHz)

1,41 (t, *J* = 7,0 Hz, 1,8H, CH₂C*H*₃); 1,46 (t, *J* = 7,0 Hz, 1,2H, CH₂C*H*₃); 2,15 (s, 1,8H, COC*H*₃); 2,21 (s, 1,2H, COC*H*₃); 2,08-2,31 (m, 2H, CF₃CH(OAc)C*H*₂); 2,82-3,07 (m, 2H, C*H*₂CN); 3,90-3,95 (m, 0,6H, C*H*S); 4,03-4,07 (m, 0,4H, C*H*S); 4,61 (q, *J* = 7,0 Hz, 1,2H, CH₃C*H*₂); 4,63 (q, *J* = 7,0 Hz, 0,8H, CH₃C*H*₂); 5,41-5,45 (m, 0,6H, CF₃C*H*); 5,57-5,60 (m, 0,4H, CF₃C*H*).

RMN¹³C (δ, ppm) (CDCl₃, 100 MHz)

13,7 (*C*H₃CH₂); 20,1 (0,6xCF₃CH(OAc)*C*H₂); 22,3 (*C*H₃CO); 24,2 (0,4xCF₃CH(OAc)*C*H₂); 30,8 (0,6x*C*H₂CN); 31,5 (0,4x*C*H₂CN); 42,0 (0,6x*C*HS); 42,4 (0,4x*C*HS); 66,4 (q, *J* = 30 Hz, 0,6xCF₃*C*H); 66,7 (q, *J* = 30 Hz, 0,4xCF₃*C*H); 70,6 (0,6x*C*H₂CH₃); 70,9 (0,4x*C*H₂CH₃); 115,9 (0,6xCN); 116,1 (0,4x*C*N); 121,6 (q, *J* = 281 Hz, 0,6x*C*F₃); 124,9 (q, *J* = 281 Hz, 0,4x*C*F₃); 168,5 (0,6x*C*=O); 169,1 (0,4x*C*=O); 210,5 (0,6x*C*=S); 210,9 (0,4x*C*=S).

IR (ν, cm⁻¹)(CCl₄)

2928; 1764 (C=O); 1372; 1279; 1210; 1186; 1111; 1083; 1049; 1006; 969. Masse (IC, NH3)

330 (MH⁺); 347 (MNH₄⁺).

### Exemple 36 :

### Diester de O-éthyle et de S-1-(2-acétoxy-3,3,3-trifluoro-propyl)-4-oxo-pentyle de l'acide dithiocarbonique

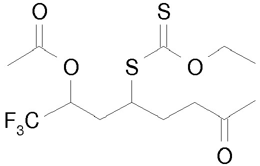

C₁₃H₁₉F₃O₄S₂ M= 360,42 g.mol⁻¹

### Réaction :

Effectuée selon le mode opératoire général avec 200 mg (0,76 mmol) de xanthate selon l'exemple 29 et 225 mg (2,28 mmol) de hex-5-èn-2-one dans le 1,2-dichloroéthane (2 mL). La réaction est terminée après addition de 10% de DLP (30 mg) et 3h de reflux.

### Purification :

Chromatographie sur gel de silice (acétate d'éthyle-éther de pétrole 5/95).

### Produit :

Huile jaune pâle.

### Rendement

88% (mélange de 2 diastéréoisomères dans un rapport 3/2)

RMN¹H (δ, ppm) (CDCl₃, 400 MHz)

1,42 (t, *J* = 7,0 Hz, 1,8H, CH₂*C*H₃); 1,47 (t, *J* = 7,0 Hz, 1,2H, CH₂*C*H₃); 1,69-1,74 (m, 1,2H, C*H*₂COCH₃); 1,78-1,82 (m, 0,8H, C*H*₂COCH₃); 1,98 (s, 1,8H, COC*H*₃); 2,10 (s, 1,8H, COC*H*₃); 2,11 (s, 1,2H, COC*H*₃); 2,12 (s, 1,2H, COC*H*₃); 2,02-2,31 (m, 2H, C*H*₂CH₂COCH₃); 2,51-2,70 (m, 2H, C*H*₂CHS); 3,68-3,74 (m, 0,6H, C*H*S); 3,91-3,95 (m, 0,4H, C*H*S); 4,58 (q, *J* = 7,0 Hz, 1,2H, CH₃C*H*₂); 4,64 (q, *J* = 7,0 Hz, 0,8H, CH₃C*H*₂); 5,39-5,51 (m, 0,6H, CF₃C*H*); 5,49-5,54 (m, 0,4H, CF₃C*H*).

RMN¹³C (δ, ppm) (CDCl₃, 100 MHz)

13,3 (*C*H₃CH₂); 20,3 (*C*H₃CO); 24,4 (0,6x*C*H₂COCH₃); 25,7 (0,4x*C*H₂COCH₃); 29,6 (0,6x*C*H₃CO); 31,5 (0,4x*C*H₃CO); 32,5 (0,6x*C*H₂CH₂COCH₃); 33,5 (0,4x*C*H₂CH₂COCH₃); 39,8 (0,6x*C*H₂CHS); 40,0 (0,4x*C*H₂CHS); 45,9 (0,6x*C*HS); 46,4 (0,4x*C*HS); 66,7 (q, *J* = 32 Hz, 0,6xCF₃*C*H); 67,0 (q, *J* = 30 Hz, 0,4xCF₃*C*H); 69,9 (0,6x*C*H₂CH₃); 70,3 (0,4x*C*H₂CH₃); 124,5 (q, *J* = 281 Hz, 0,6x*C*F₃); 124,6 (q, *J =* 281 Hz, 0,4x*C*F₃); 168,6 (0,6xO*C*=O); 169,2 (0,4xO*C*=O); 206,7 (0,6x*C*=O); 206,9 (0,4x*C*=O); 212,4 (0,6x*C*=S); 213,2 (0,4x*C*=S).

IR (v, cm⁻¹)(CCl₄)

2983; 2926; 2335; 1764 (OC=O); 1721 (NC=O); 1441; 1371; 1283; 1214; 1182; 1141; 1053; 909.

Masse (IC, NH3)

361 (MH⁺), 378 (MNH₄⁺).

| Microanalyse | Elément: | Carbone | Hydrogène |
|---|---|---|---|
| | Calculé (%) | 44,91 | 5,65 |
| | Trouvé (%) | 44,91 | 5,66 |

### Exemple 37 :

### Ester de 4-[4-bromo-phényl)-méthanesulfonyl-amino]-3-éthoxycarbonyl-sulfanyl-1-trifluorométhyl-butyle] de l'acide acétique

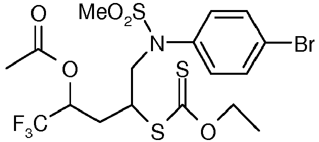

C₁₇H₂₁BrF₃NO₃S₃ M= 552,42 g.mol⁻¹

### Réaction :

Effectuée selon le mode opératoire général avec 1,54 g (5,88 mmol) de xanthate de l'exemple 29 et 2,55 mg (8,88 mmol) de N-allyl-N-(4-bromophényl)-méthanesulfonamide dans le 1,2-dichloroéthane (8 mL). La réaction est terminée après addition de 20% de DLP (468 mg) et 8h00 de reflux.

### Purification :

Chromatographie sur gel de silice (acétate d'éthyle-éther de pétrole 1/9).

### Produit :

Huile jaune pâle.

### Rendement :

71 % (2 diastéréoisomères dans un rapport 1/1)

### Premier diastéréoisomère

### RMN¹H (δ, ppm) (CDCl₃, 400 MHz)

1,28 (t, *J* = 7,0 Hz, 3H, CH₂C*H*₃); 1,96 (s, 3H, COC*H*₃); 2,29-2,35 (m, 2H, CF₃CH(OAc)C*H*₂); 2,93 (s, 3H, SO₂C*H*₃); 3,67-3,73 (m, 1 H, C*H*S); 3,94-3,99 (m, 2H, C*H*₂N); 4,31 (q, *J* = 7,0 Hz 2H, CH₃C*H*₂); 4,59-4,64 (m, 1 H, CF₃C*H*); 7,23 (d, *J* = 8,0 Hz, 2H, *H*_{Ar}(*H*C=CNSO₂)); 7,53 (d, *J* = 8,0 Hz, 2H, *H*_{Ar}(*H*C=CBr)).

RMN¹³C (δ, ppm) (CDCl₃, 100 MHz)

13,6 (*C*H₃CH₂); 24,8 (*C*H₃CO); 31,3 (CF₃CH(OAc)*C*H₂); 31,6 (*C*H₃SO₂); 34,0 (CHS); 48,6 (q, *J* = 30 Hz, CF₃*C*H); 52,3 (*C*H₂N); 70,0 (*C*H₂CH₃); 122,0 (*C*q_{Ar}.Br); 129,9 (q, *J* = 281 Hz, *C*F₃); 131,4 (*C*H_{Ar}. (H*C*=CNSO₂)); 133,53 (*C*H_{Ar}.(H*C*=CNSO₂)); 133,5 (2x*C*H_{Ar}. (*H*C=CBr)); 146,5 (*C*q_{Ar}.NSO₂); 180,0 (*C*=O); 213,1 (*C*=S).

IR (v, cm⁻¹)(CCl₄)

2983; 1705 (C=O); 1439; 1358; 1226; 1184; 1162; 1135; 1110; 1039; 1008.

Masse (IC, NH3)

553 (MH⁺); 570 (MNH₄⁺).

| Microanalyse | Elément: | Carbone | Hydrogène |
|---|---|---|---|
| | Calculé (%) | 36,96 | 3,83 |
| | Trouvé (%) | 36,75 | 3,89 |

### Deuxième diastéréoisomère

RMN¹H (δ, ppm) (CDCl₃, 400 MHz)

1,25 (t, *J* = 7,0 Hz, 3H, CH₂C*H*₃); 1,96 (s, 3H, COC*H*₃); 2,24-2,31 (m, 2H, CF3CH(OAc)C*H₂*); 2,87 (s, 3H, SO₂C*H*₃); 3,51-3,59 (m, 1 H, CHS); 3,78-3,83 (m, 2H, C*H*₂N); 4,27 (q, *J* = 7,0 Hz 2H, CH₃C*H*₂); 4,61-4,65 (m, 1 H, CF₃C*H*; 7,18 (d, *J* = 8,0 Hz, 2H, *H*_{Ar}(*H*C=CNSO₂)); 7,51 (d, *J* = 8,0 Hz, 2H, *H*_{Ar}(*H*C=CBr)).

RMN¹³C (δ, ppm) (CDCl₃, 100 MHz)

13,5 (*C*H₃CH₂); 22,5 (*C*H₃CO); 30,0 (CF₃CH(OAc)*C*H₂); 31,5 (*C*H₃SO₂); 33,9 (CHS); 48,6 (q, *J* = 30 Hz, CF₃*C*H); 51,6 (*C*H₂N); 76,7 (*C*H₂CH₃); 122,0 (*C*q_{Ar}.Br); 129,5 (q, *J* = 281 Hz, *C*F₃); 131,2 (*C*H_{Ar}. (H*C*=CNSO₂)); 133,5 (*C*H_{A}r. (H*C*=CNSO₂)); 133,5 (2x*C*HAr. (H*C*=CBr)); 146,1 (*C*q_{Ar}.NSO2); 179,9 (*C*=O); 213,0 (*C*=S).

IR (ν, cm⁻¹)(CCl₄)

2987; 1709 (C=O); 1442; 1354; 1221; 1178; 1157; 1117; 1035; 1001. Masse (IC, NH3)

553 (MH⁺); 570 (MNH₄⁺).

| Microanalyse | Elément: | Carbone | Hydrogène |
|---|---|---|---|
| | Calculé (%) | 36,96 | 3,83 |
| | Trouvé (%) | 36,87 | 3,74 |

### Transformations des adduits

### Exemple 38 :

### 4-Acétoxy-5,5,5-trilfuoro-pent-1-ène

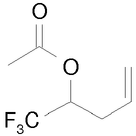

C₇H₉F₃O₂ M= 182,14 g.mol⁻¹

Réaction : A une solution du xanthate de l'exemple 32 (200 mg, 0,53 mmol) dans le tétrahydrofurane (3 mL) est ajoutée une solution normale de fluorure de tétrabutylammonium dans le THF (265 mL, 1,06 mmol). Après 2h à température ambiante, le mélange est concentré sous pression réduite avant d'être purifié.

### Purification :

Chromatographie sur gel de silice (acétate d'éthyle-éther de pétrole 1/9).

### Produit :

Huile jaune pâle.

### Rendement :

74%.

RMN¹H (δ, ppm) (CDCl₃, 400 MHz)

2,14 (s, 3H, OCOC*H*₃); 3,08-3,14 (m, 2H, C*H*₂); 3,65-3,72 (m, 1 H, C*H*=CH₂); 4,43-4,47 (m, 2H, CH=C*H*₂); 4,58-4,63 (m, 1 H, CF₃C*H*).

RMN¹³C (δ, ppm) (CDCl₃, 100 MHz)

19,7 (*C*H₂); 22,3 (*C*H₃CO); 67,5 (q, *J =* 30 Hz, CF₃*C*H); 69,8 (CH=*C*H₂); 102,5 (*C*H=CH₂); 123,8 (q, *J=* 281 Hz, *C*F₃); 170,2 (*C*=O).

IR (v, cm⁻¹)(CCl₄)

2830; 2340; 1765 (C=O); 1431; 1385; 1272; 1195; 1128; 985; 927.

Masse (IC, NH3)

183 (MH⁺); 200 (MNH₄⁺).

| Microanalyse | Elément: | Carbone | Hydrogène |
|---|---|---|---|
| | Calculé (%) | 46,06 | 4,77 |
| | Trouvé (%) | 47,16 | 4,98 |

### Exemple 39 :

### Ester de 1-[5-bromo-1-méthanesulfonyl-2,3-dihydro-1H-indol-3-ylméthyl)-2,2,2-trifluoro-éthyle] de l'acide acétique

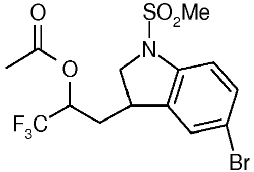

C₁₄H₁₅BrF₃NO₄S M= 430,24 g.mol⁻¹

### Réaction :

A une solution, préalablement dégazée au reflux sous argon, du xanthate adduit de l'exemple 37 (300 mg, 0,54 mmol) dans le 1,2-dichloroéthane (4 mL) est additionné du DLP à raison de 10 mol% (21 mg, 0,054 mmol) toutes les heures. La réaction est arrêtée après 13h30 de reflux et addition de 120% de DLP (258 mg, 0,65 mmol). Le milieu réactionnel est alors ramené à température ambiante et concentré sous pression réduite avant d'être purifié.

### Purification :

Chromatographie sur gel de silice (acétate d'éthyle-éther de pétrole 1/9).

### Produit :

Huile jaune pâle.

### Rendement :

88% (mélange de 2 diastéréoisomères dans un rapport 1/1)

RMN¹H (δ, ppm) (CDCl₃, 400 MHz)

1,78-1,87 (m, 0,5H, CF₃CH(OAc)C*H*₂); 1,86-1,93 (m, 0,5H, CF₃CH(OAc)C*H*₂); 1,98 (s, 1,5H, COC*H*₃); 2,01 (s, 1,5H, COC*H*₃); 2,19-2,23 (m, 0,5H, CF₃CH(OAc)C*H₂*); 2,22-2,26 (m, 0,5H, CF₃CH(OAc)C*H₂*); 3,04 (s, 1,5H, SO₂C*H*₃); 3,06 (s, 1,5H, SO₂C*H*₃); 3,65-3,70 (m, 0,5H, C*H*CH₂N) ; 3,71-3,74 (m, 0,5H, C*H*CH₂N); 3,87 (dd, *J₁* = 10,1 Hz, *J₂* = 9,9 Hz, 0,5H, C*H*₂N); 3,90 (dd, *J₁* = 10,1 Hz, *J2 =* 10,0 Hz, 0,5H, C*H*₂N); 4,08 (dd, *J₁* = 10,0 Hz, *J₂* = 9,9 Hz, 0,5H, C*H*₂N); 4,10 (dd, *J₁* = 10,0 Hz, *J₂* = 10,0 Hz, 0,5H, C*H*₂N); 4,79-4,85 (m, 0,5H, CF₃C*H*); 4,87-4,90 (m, 0,5H, CF₃C*H*); 7,25 (d, *J* = 8,5 Hz, 0,5H, H_{Ar}(*H*C=CNSO₂)); 7,27 (d, *J =* 8,6 Hz, 0,5H, H_{Ar}(*H*C=CNSO₂)); 7,40 (d, *J =* 8,5 Hz, 0,5H, H_{Ar}(*H*C=CBr)); 7,42 (d, *J =* 8,6 Hz, 0,5H, H_{Ar}(*H*C=CBr)); 7,70 (s, 0,5H, H_{Ar}(*H*C=CBr)); 7,72 (s, 0,5H, *H*_{Ar}(*H*C=CBr)).

RMN¹³C (δ, ppm) (CDCl₃, 100 MHz)

22,8 (*C*H₃CO); 32,8 (0,5xCF₃CH(OAc)*C*H₂); 33,1 (0,5x CF₃CH(OAc)*C*H₂); 34,4 (0,5x*C*H₂N); 34,6 (0,5x*C*H₂N); 36,8 (0,5x *C*H₃SO₂); 36,9 (0,5x*C*H₃SO₂); 48,5 (q, *J* = 30 Hz, 0,5xCF₃*C*H); 48,7 (q, *J* = 30 Hz, 0,5xCF₃*C*H); 55,8 (0,5x*C*HCH₂N); 56,3 (0,5x*C*HCH₂N); 115,5 (0,5x*C*H_{Ar}=CNSO₂); 115,7 (0,5x*C*H_{Ar}=CNSO₂); 116,3 (0,5x*Cq*_{Ar.}Br); 116,6 (0,5x*Cq*_{Ar.}Br); 126,8 (q, *J =* 281 Hz, 0,5x*C*F₃); 127,0 (q, *J =* 281 Hz, 0,5x*C*F₃); 128,3 (0,5x*C*H_{Ar.}=CBr); 128,5 (0,5x*C*H_{Ar.}=CBr); 132,0 (0,5x*C*H_{Ar.}=CBr); 132,1 (0,5x*C*H_{Ar.}=CBr); 137,5 (0,5x*Cq*_{Ar.}NSO₂); 137,8 (0,5x*Cq*_{Ar}.NSO₂); 142,8 (0,5x*Cq*_{Ar.}); 143,0 (0,5x*Cq*_{Ar.}); 170,9 (0,5x*C*=O); 171,3 (0,5x*C*=O).

IR (v, cm⁻¹) (CCl₄)

1759 (C=O); 1549; 1352; 1307; 1269; 1214; 1161; 1128; 1112.

Masse (IC, NH3)

431 (MH⁺); 448 (MNH₄⁺).

| Microanalyse | Elément: | Carbone | Hydrogène |
|---|---|---|---|
| | Calculé (%) | 39,08 | 3,51 |
| | Trouvé (%) | 39,17 | 3,63 |

### EXEMPLES DE PROCEDES DE PREPARATION DES COMPOSES DE FORMULE (VIIIB)

### Exemple 40: Ester de 2-benzoyloxy-3,3,3-trifluoro-1-trifluorométhyl-propyle de l'acide benzoïque

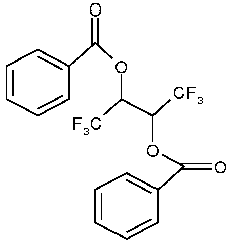

C₁₈H₁₂F₆O₄ M= 406,28 g.mol⁻¹

### Réaction :

A une solution, préalablement dégazée au reflux sous argon, du xanthate de l'exemple 30 (430 mg, 1,3 mmol) dans le chlorobenzène (14 mL) est additionné du DLP à raison de 10 mol% (52 mg, 0,13 mmol) toutes les 10 min. La réaction est arrêtée après 4h de reflux et addition de 140% de DLP (724 mg, 1,8 mmol). Le milieu réactionnel est alors ramené à température ambiante et concentré sous pression réduite avant d'être purifié.

### Purification :

Par précipitation dans un mélange éther-éther de pétrole 5/95.

### Produit :

Solide incolore.

### Rendement :

65% (mélange de 2 diastéréoisomères dans un rapport 1/1)

RMN¹H (δ, ppm) (CD₃)₂SO, 400 MHz)

4,57-4,64 (m, 1 H, CF₃C*H*); 4,66-4,69 (m, 1 H, CF₃C*H*); 7,31-7,39 (m, 4H_{Ar}, CH_{Ar}=C*H*_{Ar}=CH_{Ar}Cq_{Ar}) ; 7,48-7,53 (m, 2H_{Ar}, C*H*_{Ar}=CH_{Ar}=CH_{Ar}Cq_{Ar}) ; 7,77-7,89 (m, 4H_{Ar}, CH_{Ar}=CH_{Ar}=C*H*_{Ar}Cq_{Ar}).

RMN¹³C (δ, ppm) ((CD₃)₂SO,400 MHz)

78,4 (q, *J* = 31 Hz, CF₃*C*H); 79,8 (q, *J* = 31 Hz, CF₃*C*H); 117,03 (q, *J* = 283 Hz, *C*F₃); 118,09 (q, *J* = 283 Hz, *C*F₃); 126,4 (2xCHAr); 126,8 (2xCHAr); 129,1 (2x*C*H_{Ar}); 129,3 (2x*C*H_{Ar}); 129,7 (2x*C*H_{Ar}); 131,5 (*C*q_{Ar}); 131,8 (*C*q_{Ar}); 159,5 (*C*=O); 160,0 (*C*=O).

IR (v, cm⁻¹) (nujol)

2976; 1702 (C=O); 1547; 1340; 1301; 1255; 1238; 1181; 1153; 1108.

Masse (IC, NH3)

407 (MH⁺); 424 (MNH₄⁺).

### EXEMPLES DE PROCEDE DE PREPARATION DE COMPOSES DE FORMULE (IC)

### Synthèse du xanthate 2-trifluorométhyléthylchloro

### Exemple 41 : Diester de O-éthyle et de S-1-chloro-2,2,2-trifluoro-éthyle de l'acide dithiocarbonique

### a) Diester de O-éthyle et de S-1-hydroxy-2,2,2-trifluoro-éthyle de l'acide dithiocarbonique

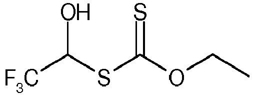

C₅H₇F₃O₂S₂ M= 220,23 g.mol⁻¹

### Réaction :

A une solution de 2,2,2-trifluoro-1-méthoxy-éthanol (300 mg, 2,31 mmol) dans l'acétone (5 mL) est ajouté le sel d'éthylxanthogénate de potassium (737 mg, 4,62 mmol). Le mélange réactionnel est refroidi à 0°C et de l'acide sulfurique (123,6 µL, 2,31 mmol) est ajouté goutte à goutte. Après une heure à 0°C, le mélange réactionnel est concentré sous pression réduite. Le résidu est repris dans l'éther, filtré et concentré à nouveau sous pression réduite.

### Produit :

Huile jaune.

### Rendement :

69%

RMN¹H (δ, ppm) (CDCl₃, 400 MHz)

1,54 (t, *J =* 6,3 Hz, 3H, CH₂C*H*₃); 4,71 (q, *J =* 6,3 Hz, 2H, CH₃C*H*₂); 6,02 (q, *J* = 5,71 Hz, 1 H, CF₃C*H*).

RMN¹³C (δ, ppm) (CDCl₃, 100 MHz)

13,7 (*C*H₃CH₂); 57,8 (q, *J=* 30 Hz, CF₃*C*H); 71,4 (*C*H₂CH₃); 124,4 (q, *J=* 280 Hz, *C*F₃); 207,1 (C=S).

IR (v, cm⁻¹)(CCl₄)

2961; 1453; 1314; 1231; 1130; 1052; 1023.

Masse (IC, NH3)

221 (MH⁺), 238 (MNH₄⁺).

### b) Diester de O-éthyle et de S-1-chloro-2,2,2-trifluoro-éthyle de l'acide dithiocarbonique

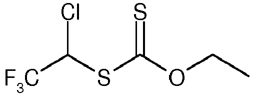

C₅H₆CIF₃OS₂ M= 238,68 g.mol⁻¹

### Réaction :

Une solution de l'alcool 41a) (1,38 g, 6,3 mmol) et de pentachlorure de phosphore (1,30 g, 6,3 mmol) est agitée à température ambiante pendant 1 h. Après évaporation sous pression réduite, le résidu est purifié.

### Purification :

Chromatographie sur gel de silice (éther de pétrole puis acétate d'éthyle 1/9 puis 2/8).

### Produit :

Huile jaune.

### Rendement :

18%

RMN¹H (δ, ppm) (CDCl₃, 400 MHz)

1,49 (t, *J =* 6,8 Hz, 3H, CH₂C*H*₃); 4,75 (q, *J =* 6,8 Hz, 2H, CH₃C*H*₂); 6,27 (q, *J* = 8,4 Hz, 1 H, CF₃C*H*).

RMN¹³C (δ, ppm)(CDCl₃, 100 MHz)

13,9 (*C*H₃CH₂); 22,6 (q, *J*= 35 Hz, CF₃*C*H); 71,5 (*C*H₂CH₃); 121,1 (q, J= 282 Hz, CF₃); 211,1 (C=S).

IR (v, cm⁻¹)(CCl₄)

2986; 2939; 1442; 1370; 1344; 1194; 1131; 1032; 868; 823.

Masse (IC, NH3)

238 (M), 240 (M+2); 193 (MH+-EtOH).

| Microanalyse | Elément: | Carbone | Hydrogène |
|---|---|---|---|
| | Calculé (%) | 25,16 | 2,53 |
| | Trouvé (%) | 25,19 | 2,51 |

### ADDITIONS RADICALAIRES

### EXEMPLES DE PROCEDES DE PREPARATION

### DE COMPOSES DE FORMULE (IIC)

Le mode opératoire général est tel qu'énoncé précédemment pour la préparation des composés (IIa).

### Exemple 42 : Diester de O-éthyle et de S-3-chloro-4,4,4-trifluoro-1-triméthylsilanylméthylbutyle de l'acide dithiocarbonique

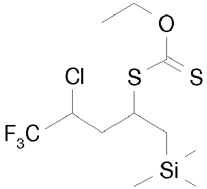

C₁₁H₂₀CIF₃OS₂Si M= 352,94 g.mol⁻¹

### Réaction :

Effectuée selon le mode opératoire général avec 133 mg (0,64 mmol) de xanthate de l'exemple 41 et 220 mg (2,29 mmol) d'allyltriméthylsilane dans le 1,2-dichloroéthane (2 mL). La réaction est terminée après addition de 5% de DLP (13 mg) et 1h30 de reflux.

### Purification :

Chromatographie sur gel de silice (éther de pétrole à acétate d'éthyle-éther de pétrole 1/9).

### Produit :

Huile jaune.

### Rendement :

88% (mélange de 2 diastéréoisomères dans un rapport 2/3)

RMN¹H (δ, ppm) (CDCl₃, 400 MHz)

0,12 (s, 3,6H, Si(C*H*₃)₃); 0,31 (s, 5,4H, Si(C*H*₃)₃); 1,13-1,18 (m, 0,8H, C*H*₂Si(CH₃)₃); 1,30-1,35 (m, 1,2H, C*H*₂Si(CH₃)₃); 1,48 (t, *J* = 7,0 Hz, 3H, CH₂C*H*₃); 2,08-2,14 (m, 0,8H, CF₃CHClC*H*₂); 2,24-2,29 (m, 1,2H, CF₃CHClC*H*₂); 4,01-4,08 (m,0,4H, CHS); 4,10-4,15 (m, 0,6H, CHS); 4,18-4,23 (m, 0,4H, CF₃C*H*); 4,27-4,35 (m, 0,6H, CF₃C*H*); 4,69-4,76 (m, 2H, CH₃C*H*₂);

RMN¹³C (δ, ppm) (CDCl₃, 100 MHz)

-0,7 (1,2xSi(*C*H₃)₃); -0,8 (1,8xSi(*C*H₃)₃); 13,9 (*C*H₃CH₂); 27,3 (0,4x*C*H₂Si(CH₃)₃); 28,2 (0,6x*C*H₂Si(CH₃)₃); 33,3 (0,4x*C*H₂CF₃CHCl); 35,5 (0,6x*C*H₂CF₃CHCl); 43,3 (0,4xCHS); 47,4 (0,6xCHS); 51,3 (q, *J* = 28 Hz, 0,4xCF₃*C*H); 52,4 (q, *J =* 28 Hz, 0,6xCF₃*C*H); 70,5 (*C*H₂CH₃); 120,3 (q, *J =* 281 Hz, 0,4x*C*F₃); 122,4 (q, *J =* 281 Hz, 0,6x*C*F₃); 213,1 (0,4xC=S); 215,2 (0,6xC=S).

IR (v, cm⁻¹) (CCl₄)

2956; 1471; 1441; 1415; 1373; 1328; 1313; 1220; 1178; 1123; 1051; 982; 937.

Masse (IC, NH3)

354 (MH⁺); 371 (MNH₄⁺).

| Microanalyse | Elément: | Carbone | Hydrogène |
|---|---|---|---|
| | Calculé (%) | 37,43 | 5,71 |
| | Trouvé (%) | 37,66 | 5,94 |

### Exemple 43 :

### Ester de 4-chloro-2-éthoxythiocarbonylsulfanyl-5,5,5-trifluoro-pentyle de l'acide acétique

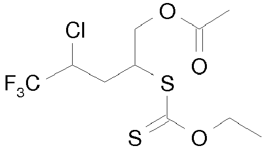

C₁₀H₁₄ClF₃O₃S₂ M= 338,80 g.mol⁻¹

### Réaction :

Effectuée selon le mode opératoire général avec 200 mg (0,84 mmol) de xanthate de l'exemple 41 et 232 µL (2,5 mmol) d'acétate d'allyle dans le 1,2-dichloroéthane (2 mL). La réaction est terminée après addition de 5% de DLP (17 mg) et 1h30 de reflux.

### Purification :

Chromatographie sur gel de silice (acétate d'éthyle-éther de pétrole 1/9).

### Produit :

Huile jaune pâle.

### Rendement :

74% (2 diastéréoisomères dans un rapport 6/1).

### Premier diastéréoisomère (majoritaire)

RMN¹H (δ, ppm) (CDCl₃, 400 MHz)

1,44 (t, *J* = 7,0 Hz, 3H, CH₂C*H*₃); 2,06 (s, 3H, COC*H*₃); 2,35-2,55 (m, 2H, CF₃CHClC*H*₂); 4,18-4,22 (m, 1 H, CHS); 4,30-4,35 (m, 2H, C*H*₂OCOCH₃); 4,43 (q, *J* = 7,0 Hz, 2H, CH₃C*H*₂); 4,49-4,55 (m, 1 H, CF₃C*H*).

RMN¹³C (δ, ppm) (CDCl₃, 100 MHz)

13,1 (*C*H₃CH₂); 20,7 (*C*H₃CO); 32,0 (CF₃CHCl*C*H₂); 42,3 (CHS); 56,3 (q, *J=* 28 Hz, CF₃*C*H); 64,0 (*C*H₂OCOCH₃); 70,5 (*C*H₂CH₃); 118,7 (q, *J =* 281 Hz, *C*F₃); 169,1 (*C*=O); 210,3 (C=S).

IR (v, cm⁻¹) (CCl₄)

2984; 1751 (C=O); 1461; 1438; 1379; 1363; 1310; 1266; 1228; 1185; 1130; 1050.

Masse (IC, NH3)

339 (MH⁺); 356 (MNH₄⁺).

| Microanalyse | Elément: | Carbone | Hydrogène |
|---|---|---|---|
| | Calculé (%) | 35,44 | 4,17 |
| | Trouvé (%) | 35,17 | 4,08 |

### Deuxième diastéréoisomère (minoritaire)

RMN¹H (δ, ppm) (CDCl₃, 400 MHz) 1,46 (t, *J* = 7,0 Hz, 3H, CH₂C*H*₃); 2,11 (s, 3H, COC*H*₃); 2,21-2,31 (m, 2H, CF₃CHClC*H*₂); 4,17-4,22 (m, 1 H, CHS); 4,29-4,37 (m, 2H, C*H*₂OCOCH₃); 4,45 (q, *J =* 7,0 Hz, 2H, CH₃C*H*₂); 4,51-4,58 (m, 1 H, CF₃C*H*).

### RMN¹³C (δ, ppm) (CDCl₃, 100 MHz)

13,2 (*C*H₃CH₂); 20,9 (*C*H₃CO); 31,1 (CF₃CHCl*C*H₂); 43,5 (CHS); 58,4 (q, *J=* 28 Hz, CF₃*C*H); 66,1 (*C*H₂OCOCH₃); 68,1 (*C*H₂CH₃); 120,4 (q, *J =* 281 Hz, *C*F₃); 170,2 (*C*=O); 212,4 (C=S).

### IR (v, cm⁻¹)(CCl₄)

2980; 1749 (C=O); 1450; 1437; 1357; 1308; 1264; 1225; 1183; 1135; 1047.

Masse (IC, NH3)

339 (MH⁺); 356 (MNH₄⁺).

| Microanalyse | Elément: | Carbone | Hydrogène |
|---|---|---|---|
| | Calculé (%) | 35,44 | 4,17 |
| | Trouvé (%) | 35,46 | 4,12 |

### Exemple 44 :

### Ester de O-éthyle et de S-3-chloro-1-(1,3-dioxo-1,3-dihydro-isoindol-2-ylméthyl)-4,4,4-trifluoro-butyle de l'acide dithiocarbonique

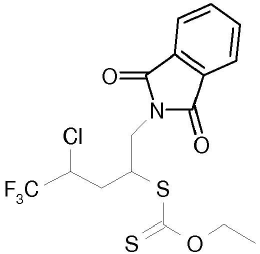

C₁₆H₁₅CIF₃NO₃S₂ M= 425,88 g.mol⁻¹

### Réaction :

Effectuée selon le mode opératoire général avec 200 mg (0,84 mmol) de xanthate de l'exemple 41 et 427 mg (2,5 mmol) de N-allylphtalimide dans le 1,2-dichloroéthane (2 mL). La réaction est terminée après addition de 10% de DLP et 3h de reflux.

### Purification :

Chromatographie sur gel de silice (acétate d'éthyle-éther de pétrole 5/95).

### Produit :

Cristaux translucides.

### Rendement :

65% (mélange de 2 diastéréoisomères dans un rapport 1/1).

RMN¹H (δ, ppm) (CDCl₃, 400 MHz)

1,37-1,43 (m, 3H, CH₂C*H*₃); 2,15-2,24 (m, 0,5H, CF₃CHClC*H*₂) ; 2,29-2,33 (m, 1 H, CF₃CHClC*H*₂) ; 2,39-2,43 (m, 0,5H, CF3CHClC*H*₂); 3,93-4,02 (m, 2H, C*H*₂N); 4,01-4,06 (m, 1 H, CH₃C*H*₂); 4,04-4,14 (m, 1 H, CH₃C*H*₂); 4,31-4,38 (m, 0,5H, CHS); 4,38-4,47 (m, 0,5H, CHS); 5,49-5,56 (m, 0,5H, CF₃C*H*) ; 5,59-5,66 (m, 0,5H, CF₃C*H*); 7,72-7,74 (m, 2H, *H*_{Ar.}(CqCH=C*H*)); 7,76-7,84 (m, 2H, H_{Ar.}(CqC*H*=CH)_{.}).

RMN¹³C (δ, ppm) (CDCl₃, 100 MHz)

12,2 (0,5x*C*H₃CH₂); 12,3 (0,5x*C*H₃CH₂); 30,8 (0,5xCF₃CHCl*C*H₂); 31,5 (0,5xCF₃CHCl*C*H₂); 38,7 (0,5x*C*H₂N); 39,5 (0,5x*C*H₂N); 43,5 (0,5xCHS); 44,0 (0,5xCHS); 49,5 (q, *J* = 31 Hz, 0,5xCF₃*C*H); 50,0 (q, *J* = 35 Hz, 0,5xCF₃*C*H); 115,9 (0,5x*C*H₂CH₃); 116,0 (0,5x*C*H₂CH₃); 118,4 (q, *J =* 278 Hz, 0,5x*C*F₃); 120,3 (q, *J* = 278 Hz, 0,5x*C*F₃); 129,8 (2x*Cq*_{Ar.}); 132,8 (2x*C*H_{Ar.}(CqCH=*C*H)); 133,2 (2x*C*H_{Ar.}(Cq*C*H=CH)); 159,3 (*C*=O_{Ar}.); 162,4 (*C*=O_{Ar}.); 211,5 (0,5xC=S); 212,7 (0,5xC=S).

IR (v, cm⁻¹)(CCl₄)

2926; 1776; 1722 (C=O); 1430; 1391; 1328; 1265; 1223; 1189; 1131; 1112; 1048; 887.

Masse (IC, NH3)

427 (MH⁺); 444 (MNH₄⁺).

| Microanalyse | Elément: | Carbone | Hydrogène |
|---|---|---|---|
| | Calculé (%) | 49,64 | 3,82 |
| | Trouvé (%) | 49,51 | 3,93 |

### Exemple 45 :

### Diester de O-éthyle et de S-1-(2-chloro-3,3,3-trifluoro-propyl)-4-oxo-pentyle de l'acide dithiocarbonique

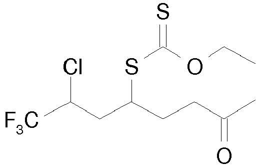

C₁₁H-₁₆Cl₃O₂S₂ M= 336,83 g.mol⁻¹

### Réaction :

Effectuée selon le mode opératoire général avec 450 mg (1,89 mmol) de xanthate de l'exemple 41 et 657 µL (5,67 mmol) de hex-5-èn-2-one dans le 1,2-dichloroéthane (4 mL). La réaction est terminée après addition de 10% de DLP (75 mg) et 3h15 de reflux.

### Purification :

Chromatographie sur gel de silice (acétate d'éthyle-éther de pétrole 5/95).

### Produit :

Huile jaune foncé.

### Rendement :

55% sur les 4 étapes, par rapport à l'hémiacétal trifluoroacétaldehyde (mélange de 2 diastéréoisomères dans un rapport 1/1)

RMN¹H (δ, ppm) (CDCl₃, 400 MHz)

0,95 (t, *J =* 7,0 Hz, 1,5H, CH₂C*H*₃); 0,98 (t, *J =* 7,0 Hz, 1,5H, CH₂C*H*₃); 1,19-1,25 (m, 1 H, C*H*₂COCH₃); 1,35 (s, 1,5H, COC*H*₃); 1,36 (s, 1,5H, COC*H*₃); 1,35-1,39 (m, 1 H, C*H*₂COCH₃); 1,41-1,47 (m, 2H, C*H*₂CH₂COCH₃); 1,69-1,74 (m, 2H, C*H*₂CHCF₃); 3,10 (q, *J* = 7,0 Hz, 1 H, CH₃C*H*₂); 3,12 (q, *J =* 7,0 Hz, 1 H, CH₃C*H*₂); 3,39-3,46 (m, 0,5H, C*H*S); 3,51-3,53 (m, 0,5H, C*H*S); 4,54-4,59 (m, 0,5H, CF₃C*H*); 4,59-4,61 (m, 0,5H, CF₃C*H*).

RMN¹³C (δ, ppm)(CDCl₃, 100 MHz)

13,8 (*C*H₃CH₂); 22,5 (0,5x*C*H₂COCH₃); 22,7 (0,5x*C*H₂COCH₃); 28,2 (0,5x*C*H₃CO); 28,3 (0,5x*C*H₃CO); 29,0 (0,5x*C*HCH₂COCH₃); 29,2 (0,5x*C*H₂CH₂COCH₃); 29,3 (0,5x*C*H₂CHCF₃); 29,4 (0,5x*C*H₂CHCF₃); 31,8 (0,5xCHS); 32,1 (0,5xCHS); 35,7 (q, *J =* 31 Hz, 0,5xCF₃*C*H); 35,8 (q, *J =* 31 Hz, 0,5xCF₃*C*H); 69,6 (0,5x*C*H₂CH₃); 70,2 (0,5x*C*H₂CH₃); 126,8 (q, *J* = 281 Hz, 0,5x*C*F₃); 127,3 (q, *J =* 281 Hz, 0,5x*C*F₃); 208,2 (0,5x*C*=O); 209,3 (0,5x*C*=O); 215,1 (0,5xC=S); 216,2 (0,5xC=S).

IR (v, cm⁻¹)(CCl₄)

2926; 2854; 1691 (C=O); 1465; 1216; 1112; 1052.

Masse (IC, NH3)

337 (MH⁺), 354 (MNH₄⁺).

| Microanalyse | Elément: | Carbone | Hydrogène |
|---|---|---|---|
| | Calculé (%) | 39,23 | 4,79 |
| | Trouvé (%) | 39,03 | 4,73 |

### Exemple 46 :

### Ester de diméthyle 4-Chloro-2-éthoxythiocarbonylsulfanyl-5,5,5-trifluoro-pentyle de l'acide phosphonique

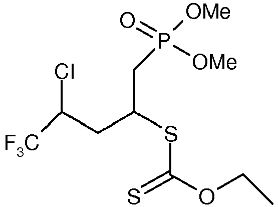

C₁₀H₁₇CIF₃O₄PS₂ M= 388,79 g.mol⁻¹

### Réaction :

Effectuée selon le mode opératoire général avec 450 mg (1,89 mmol) de xanthate de l'exemple 41 et 529 µL (5,67 mmol) de diméthylallyl phosphate dans le 1,2-dichloroéthane (2 mL). La réaction est terminée après addition de 10% de DLP (75 mg) et 3h15 de reflux.

### Purification :

Chromatographie sur gel de silice (acétate d'éthyle-éther de pétrole 1/9) puis augmentation de polarité jusqu'à acétate d'éthyle pur.

### Produit :

Huile jaune foncé.

### Rendement :

27% sur les 4 étapes (2 diastéréoisomères dans un rapport 3/2).

RMN¹H (δ, ppm) (CDCl₃, 400 MHz)

1,21-1,27 (m, 1,8H, CH₂C*H*₃); 1,25-1,30 (m, 1,2H, CH₂C*H*₃); 2,20-2,27 (m, 1 H, CHS); 3,31-3,39 (m, 1,2H, CF₃CHClC*H*₂); 3,49-3,54 (m, 0,8H, CF₃CHClC*H*₂); 3,54-3,59 (m, 2H, C*H*₂P) ; 3,65-3,69 (m, 3H, OC*H*₃); 3,69-3,74 (m, 3H, OC*H*₃) ; 4,51-4,57 (m, 1,2H, CH₃C*H*₂); 4,59-4,65 (m, 0,8H, CH₃C*H*₂); 5,81-5,88 (m, 1 H, CF₃C*H*).

RMN¹³C (δ, ppm) (CDCl₃, 100 MHz)

12,8 (0,4x*C*H₃CH₂); 13,0 (0,6x*C*H₃CH₂); 21,3 (0,4xO*C*H₃); 22,7 (0,6xO*C*H₃); 23,5 (0,4xO*C*H₃); 24,3 (0,6xO*C*H₃); 27,4 (d, *J*= 134Hz, 0,4x*C*H₂P); 28,1 (d, *J*= 138Hz, 0,6x*C*H₂P); 29,3 (q, *J*= 30 Hz, 0,4xCF₃*C*H); 30,1 (q, *J*= 30 Hz, 0,6xCF₃*C*H); 31,3 (0,4xCF₃CHCl*C*H₂); 31,4 (0,6xCF₃CHCl*C*H₂); 32,0 (0,4xCHS); 32,3 (0,6xCHS); 67,0 (0,4x*C*H₂CH3); 70,3 (0,6x*C*H₂CH₃); 123,4 (q, *J*= 278 Hz, 0,4x*C*F₃); 124,5 (q, *J*= 278 Hz, 0,6x*C*F₃); 212,0 (0,4xC=S); 212,1 (0,6xC=S).

IR (v, cm⁻¹) (CCl₄)

2957; 2928; 2855; 1732; 1710; 1263; 1235; 1135; 1043.

Masse (IC, NH3)

390 (MH⁺); 268 (MH⁺-HSCSOEt).

### Exemple 47 :

### Diester de O-éthyle et de S-3-chloro-1-cyanométhyl-4,4,4-trifluoro-butyle de l'acide dithiocarbonique

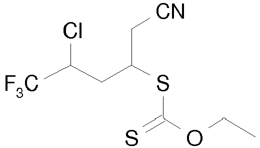

C₉H₁₁ClF₃NOS₂ M= 305,77 g.mol⁻¹

### Réaction :

Effectuée selon le mode opératoire général avec 450 mg (1,89 mmol) de xanthate de l'exemple 41 et 109 µL (5,67 mmol) de but-3-ènenitrile dans le 1,2-dichloroéthane (4 mL). La réaction est terminée après addition de 10% de DLP (75 mg) et 3h15 de reflux.

### Purification :

Chromatographie sur gel de silice (acétate d'éthyle-éther de pétrole 2/8).

### Produit :

Huile jaune foncé.

### Rendement :

52% sur les 4 étapes (mélange de 2 diastéréoisomères dans un rapport 2/1)

RMN¹H (δ, ppm) (CDCl₃, 400 MHz)

1,41 (t, *J =* 5,7 Hz, 2H, CH₂C*H*₃); 1,45 (t, *J =* 5,7 Hz, 1 H, CH₂C*H*₃); 2,12-2,63 (m, 2H, CF₃CHClC*H*₂); 3,00-3,06 (m, 2H, C*H*₂CN); 3,99-4,04 (m, 0,3H, C*H*S); 4,07-4,12 (m, 0,7H, CHS); 4,38-4,42 (m, 0,3H, CF₃C*H*); 4,46-4,53 (m, 0,7H, CF₃C*H*) ; 4,71 (q, *J*= 5,7 Hz, 0,7H, CH₃C*H*₂); 4,74 (q, *J*= 5,7 Hz, 1,3H, CH₃C*H*₂).

RMN¹³C (δ, ppm) (CDCl₃, 100 MHz)

13,7 (*C*H₃CH₂); 23,1 (0,3x*C*H₂CF₃CHCl); 25,2 (0,7x*C*H₂CF₃CHCl); 25,4 (0,3x*C*H₂CN); 30,5 (0,7x*C*H₂CN); 41,0 (0,3xCHS); 42,0 (0,7xCHS); 52,4 (q, *J =* 43 Hz, 0,3xCF₃*C*H); 53,7 (q, *J =* 43 Hz, 0,7xCF₃*C*H); 70,9 (0,3x*C*H₂CH₃); 71,0 (0,7x*C*H₂CH₃); 115,5 (0,3x*C*N); 116,1 (0,7xCN); 122,3 (q, *J*= 286 Hz, 0,3x*C*F₃); 124,4 (q, *J*= 286 Hz, 0,7x*C*F₃); 209,4 (0,3xC=S); 210,3 (0,7xC=S).

IR (v, cm⁻¹) (CCl₄)

2926; 1745; 1266; 1237; 1180; 1130; 1050.

Masse (IC, NH3)

306 (MH⁺); 323 (MNH₄⁺).

### Exemple 48 :

### Diester de O-éthyle et de S-3-chloro-1-diéthoxyméthyl-4,4,4-trifluoro-pentyle de l'acide thiocarbonique

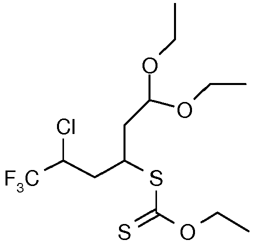

C₁₃H₂₂CIF₃O₃S₂ M= 382,89 g.mol⁻¹

### Réaction :

Effectuée selon le mode opératoire général avec 400 mg (1,69 mmol) de xanthate de l'exemple 41 et 730 mg (5,67 mmol) de 4,4-diéthoxy-butène dans le 1,2-dichloroéthane (4 mL). La réaction est terminée après addition de 15% de DLP (101 mg) et 4h45 de reflux.

### Purification :

Chromatographie sur gel de silice (acétate d'éthyle-éther de pétrole 5/95).

### Produit :

Huile épaisse jaune pâle.

### Rendement :

49% sur les 4 étapes (2 diastéréoisomères dans un rapport 1/1)

### Premier diastéréoisomère

RMN¹H (δ, ppm) (CDCl₃, 400 MHz)

1,26 (t, *J*= 8,2 Hz, 3H, CH₂C*H*₃); 1,37-1,42 (m, 2H, CH-C*H*₂-CH(OEt)₂); 1,53 (t, *J* = 8,1 Hz, 6H, 2xCH₂*C*H₃); 1,66-1,71 (m, 2H, CF₃CHClC*H*₂) ; 3,17 (t, 1 H, *J* = 8,2 Hz, C*H*(OEt)₂); 3,47-3,53 (m, 1 H, CHS); 4,62 (q, *J=* 8,2 Hz, 2H, CH₃C*H*₂O) ; 4,74 (q, J= 8,1 Hz, 4H, 2xCH₃C*H*₂O) ; 6,31 (q, *J*= 7,8 Hz, 1 H, CF₃C*H*).

RMN¹³C (δ, ppm)(CDCl₃, 100 MHz)

13,8 (2x*C*H₃CH₂); 14,2 (*C*H₃CH₂); 22,6 (CH-CH₂-CH-(OEt)₂); 28,3 (*C*H₂CHS); 29,6 (q, *J =* 30 Hz, CF₃*C*H); 31,8 (C*H*S); 35,8 (*C*H₂CH₃); 35,9 (*C*H₂CH₃); 69,7 (*C*H₂CH₃); 100,8 (*C*H(OEt)₂); 124,5 (q, *J*= 281 Hz, *C*F₃); 215,2 (C=S).

IR (v, cm⁻¹) (CCl₄)

2957; 2927; 2855; 1705; 1465; 1442; 1367; 1293; 1243; 1217; 1189; 1113; 1050.

Masse (IC, NH3)

384 (M-H⁺); 338 (MH+-EtOH).

| Microanalyse | Elément: | Carbone | Hydrogène |
|---|---|---|---|
| | Calculé (%) | 40,78 | 5,79 |
| | Trouvé (%) | 40,63 | 5,42 |

### Deuxième diastéréoisomère

RMN¹H (δ, ppm)(CDCl₃, 400 MHz)

1,26 (t, *J*= 8,2 Hz, 3H, CH₂C*H*₃); 1,41-1,47 (m, 2H, CH-C*H₂*-CH(OEt)₂); 1,56 (t, *J* = 8,0 Hz, 6H, 2xCH₂C*H*₃); 1,61-1,68 (m, 2H, CF₃CHClC*H*₂) ; 3,67-3,72 (m, 1 H, C*H*(OEt)₂); 3,85-3,92 (m, 1 H, CHS); 4,71 (q, *J =* 8,2 Hz, 2H, CH₃C*H*₂O) ; 4,78 (q, *J =* 8,0 Hz, 4H, 2xCH₃C*H*₂O) ; 6,12 (q, *J =* 7,8 Hz, 1 H, CF₃C*H*).

RMN¹³C (δ, ppm) (CDCl₃, 100 MHz)

13,7 (2x*C*H₃CH₂); 14,0 (*C*H₃CH₂); 22,5 (CH-*C*H₂-CH-(OEt)₂); 28,4 (*C*H₂CHS); 29,3 (q, *J =* 30 Hz, CF₃*C*H); 31,6 (CHS); 34,7 (*C*H₂CH₃); 34,8 (*C*H₂CH₃); 69,5 (*C*H₂CH₃); 100,3 (*C*H(OEt)₂); 123,9 (q, J= 281 Hz, *C*F₃); 216,0 (C=S).

IR (v, cm⁻¹)(CCl₄)

2963; 2931; 2851; 1701; 1467; 1448; 1295; 1257; 1219; 1195; 1115; 1057.

Masse (IC, NH3)

384 (M-H⁺); 338 (MH+-EtOH).

| Microanalyse | Elément: | Carbone | Hydrogène |
|---|---|---|---|
| | Calculé (%) | 40,78 | 5,79 |
| | Trouvé (%) | 40,67 | 5,34 |

### Exemple 49 :

### Diester de O-éthyle et de S-3-chloro-1-(4-chlorophénoxyméthyl)-4,4,4-trifluorobutyle de l'acide dithiocarbonique

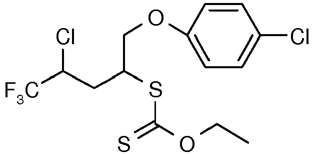

C₁₄H₁₅Cl₂F₃O₂S₂ M= 407,35 g.mol⁻¹

### Réaction :

Effectuée selon le mode opératoire général avec 400 mg (1,69 mmol) de xanthate de l'exemple 41 et 852 mg (5,67 mmol) de 1-allyloxy-4-chloro-benzène dans le 1,2-dichloroéthane (4 mL). La réaction est terminée après addition de 15% de DLP (338 mg) et 4h45 de reflux.

### Purification :

Chromatographie sur gel de silice (acétate d'éthyle-éther de pétrole 2/98).

### Produit :

Huile jaune pâle.

### Rendement :

28% sur les 4 étapes (mélange de 2 diastéréoisomères dans un rapport 2/1).

RMN¹H (δ, ppm) (CDCl₃, 400 MHz)

1,37 (t, *J =* 8,3 Hz, 3H, CH₂C*H*₃); 1,47-1,51 (m, 0,7H, CF₃CHClC*H*₂); 1,51-1,58 (m, 1,3H, CF₃CHClC*H*₂); 3,01-3,09 (m, 2H, OC*H*₂CHS); 3,57 (q, *J =* 8,3 Hz, 2H, CH₃C*H*₂) ; 4,01-4,13 (m, 0,3H, CHS); 4,13-4,21 (m, 0,7H, CHS); 4,73 (q, *J=* 5,5 Hz, 0,3H, CF₃C*H*); 4,94 (q,*J* = 5,5 Hz, 0,7H, CF₃C*H*); 6,75-6,85 (m, 2H, H_{Ar.}(C*H*=CCl)) ; 7,13-7,27 (m, 2H, H_{Ar.}(C*H*=CO)).

RMN¹³C (δ, ppm) (CDCl₃, 100 MHz)

13,5 (0,3x*C*H₃CH₂); 13,6 (0,7x*C*H₃CH₂); 23,0 (0,3x*C*H₂CHCF₃); 23,0 (0,7x*C*H₂CHCF₃); 28,7 (0,3xCHS); 28,9 (0,7xCHS); 29,2 (q, *J* = 32 Hz, 0,3xCF₃*C*H); 29,2 (q, *J* = 32 Hz, 0,7xCF₃*C*H); 32,1 (0,3xCH₂O) ; 32,2 (0,7xCH₂O) ; 63,4 (0,3x*C*H₂CH₃); 63,5 (0,7x*C*H₂CH₃); 116,2 (Cq_{Ar}Cl) ; 128,2 (q, *J* = 284 Hz, 0,3x*C*F₃); 128,4 (q, *J =* 284 Hz, 0,7x*C*F₃); 130,3 (C_{Ar}H, CH=CCI) ; 130,4 (C_{Ar.}(*C*H=CCl)); 132,7 (2xC_{Ar.} (*C*H=CO)) ; 137,3 (0,3xCq_{Ar}O) ; 137,4 (0,7xCq_{Ar}O) ; 210,7 (0,3xC=S); 211,3 (0,7x*C*=S).

IR (v, cm⁻¹)(CCl₄)

2926; 2358; 2350; 1552; 1492; 988; 962.

Masse (IC, NH3)

408 (MH⁺); 425 (MNH₄⁺).

| Microanalyse | Elément: | Carbone | Hydrogène |
|---|---|---|---|
| | Calculé (%) | 41,29 | 3,71 |
| | Trouvé (%) 4 | 1,01 | 3,57 |

### Exemple 50 :

### Diester de O-éthyle et de S-3-chloro-4,4,4-trifluoro-1-(2-oxo-pyrrolidin-1-yl)-butyle de l'acide dithiocarbonique

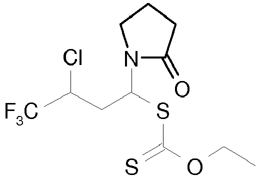

C₁₁H₁₅CIF₃NO₂S₂ M= 350,83 g.mol⁻¹

### Réaction :

Effectuée selon le mode opératoire général avec 400 mg (1,69 mmol) de xanthate de l'exemple 41 et 563 mg (5,07 mmol) de vinyl-pyrrolidin-2-one dans le 1,2-dichloroéthane (4 mL). La réaction est terminée après addition de 15% de DLP (100 mg) et 4h45 de reflux.

### Purification :

Chromatographie sur gel de silice (acétate d'éthyle-éther de pétrole 1/9).

### Produit :

Huile jaune pâle.

### Rendement :

22% sur les 4 étapes (mélange de 2 diastéréoisomères dans un rapport 1/1).

RMN¹H (δ, ppm) CDCl₃, 400 MHz)

1,94 (t, *J =* 7,0 Hz, 1,5H, CH₂C*H*₃); 2,01 (t, *J =* 7,0 Hz, 1,5H, CH₂C*H*₃); 2,43 (m, 2H, CH₂C*H*₂CH₂); 2,51 (m, 2H, C*H*₂C=O); 3,24-3,37 (m, 2H, NC*H*₂); 3,41-3,55 (m, 2H, CF₃CHClC*H*₂); 3,77 (q, *J* = 7,0 Hz, 1 H, CH₃C*H*₂); 3,83 (q, *J* = 7,0 Hz, 1 H, CH₃C*H*₂); 6,12-6,16 (m, 0,5H, CHS); 6,17-6,21 (m, 0,5H, CHS); 7,08-7,15 (m, 0,5H, CF₃C*H*) ; 7,19-7,24 (m, 0,5H, CF₃C*H*).

RMN¹³C (δ, ppm) CDCl₃, 100 MHz)

17,3 (0,5x*C*H₃CH₂); 18,4 (0,5x*C*H₃CH₂); 28,0 (0,5xCH₂*C*H₂CH₂); 28,4 (0,5xCH₂*C*H₂CH₂); 29,1 (0,5x*C*H₂C=O); 29,2 (0,5x*C*H₂C=O); 30,4 (0,5xCF₃CHCl*C*H₂); 30,6 (0,5xCF₃CHCl*C*H₂); 43,1 (0,5x*C*H₂N); 43,5 (0,5x*C*H₂N); 44,3 (q, *J* = 28 Hz, 0,5xCF₃*C*H); 46,3 (q, *J* = 28 Hz, 0,5xCF₃*C*H); 57,5 (0,5xCHS); 58,4 (0,5xCHS); 70,5 (0,5x*C*H₂CH₃); 70,6 (0,5x*C*H₂CH₃); 127,4 (q, *J =* 275 Hz, 0,5x*C*F₃); 128,4 (q, *J =* 275 Hz, 0,5x*C*F₃); 177,8 (0,5x*C*=O); 178,3 (0,5x*C*=O); 218,1 (0,5xC=S) ; 220,1 (0,5xC=S).

IR (v, cm⁻¹)(CCl₄)

2926; 2335 ; 1702 (C=O); 1260; 1114; 1049.

Masse (IC, NH3)

352 (MH⁺); 369 (MNH₄⁺).

| Microanalyse | Elément: | Carbone | Hydrogène |
|---|---|---|---|
| | Calculé (%) | 37,66 | 4,60 |
| | Trouvé (%) | 37,63 | 4,49 |

### TRANSFORMATION DES ADDUITS

### Exemple 51 :

### 1-(3-Chloro-4,4,4-trifluoro-but-1-ènyl)-pyrrolidin-2-one

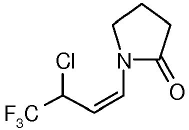

C₈H₉CIF₃NO M= 227,62 g.mol⁻¹

### Réaction :

Une solution de xanthate de l'exemple 50 (200 mg, 0,57 mmol) dans le chlorobenzène (5 mL) est portée au reflux pendant 2h. Le brut réactionnel est ramené à température ambiante puis concentré sous pression réduite avant d'être purifié.

### Purification :

Chromatographie sur gel de silice (dichlorométhane-méthanol 98/2).

### Produit :

Huile jaune pâle.

### Rendement :

Quantitatif

RMN¹H (δ, ppm) (CDCl₃, 400 MHz)

2,07-2,12 (m, 2H, CH₂C*H*₂CH₂); 2,45-2,53 (m, 2H, C*H*₂C=O); 2,87-2,93 (m, 2H, NC*H*₂); 4,69-4,77 (m, 1 H, CF₃C*H*) ; 6,75 (dd, *J₁* = 13,3 Hz, *J₂* = 6,1 Hz, 1 H, C*H*=CHN) ; 7,41 (d, *J=* 13,3 Hz, 1 H, NC*H*=CH).

RMN¹³C (δ, ppm) (CDCl₃, 100 MHz) 22,3 (CH₂*C*H₂CH₂); 28,8 (*C*H₂CO); 33,4 (*C*H₂N); 45,1 (q, *J* = 28 Hz, CF₃*C*H); 115,1 (*C*H=CHN) ; 119,1 (N*C*H=CH) ; 141,5 (q, *J* = 275 Hz, *C*F₃); 174,3 (*C*=O).

IR (v, cm⁻¹)(CCl₄)

2926; 2854 ; 2359 ; 1741 (C=O); 1699 ; 1594 ; 1460 ; 1407; 1362; 1193.

Masse (IC, NH3)

228 (MH⁺); 244 (MNH₄⁺).

| Microanalyse | Elément: | Carbone | Hydrogène |
|---|---|---|---|
| | Calculé (%) | 42,22 | 3,99 |
| | Trouvé (%) | 42,09 | 3,87 |

### Exemple 52 :

### 2-(4-Chloro-5,5,5-trifluoro-pentyl)-isoindole-1,3-dione

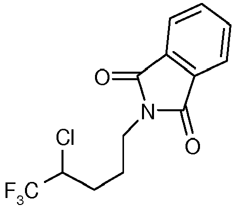

C₁₃H₁₁CIF₃NO₂ M= 305,69 g.mol⁻¹

### Réaction :

A une solution, préalablement dégazée au reflux sous argon, du xanthate adduit de l'exemple 44 (134 mg, 0,32 mmol) dans le propan-2-ol (2 mL) est additionné du DLP à raison de 10 mol% (26 mg, 0,032 mmol) toutes les heures. La réaction est arrêtée après 13h de reflux et addition de 110% de DLP (140 mg, 0,35 mmol). Le milieu réactionnel est alors ramené à température ambiante et concentré sous pression réduite avant d'être purifié.

### Purification :

Chromatographie sur gel de silice (acétate d'éthyle-éther de pétrole 2/98).

### Produit :

Cristaux translucides.

### Rendement :

78%

RMN¹H (δ, ppm) (CDCl₃, 400 MHz)

1,57-1,63 (m, 2H, CF₃CHClC*H*₂) ; 2,26-2,32 (m, 2H, C*H*₂CH₂N); 3,93-4,02 (t, *J =* 6,5 Hz, 2H, C*H*₂N); 7,17-7,23 (m, 1 H, CF3C*H*); 7,62-7,71 (m, 2H, H_{Ar.}(CqCH=C*H)*); 7,73-7,84 (m, 2H, *H*_{Ar.}(CqC*H*=CH)).

RMN¹³C (δ, ppm) (CDCl₃, 100 MHz)

25,1 (*C*H₂CH₂N); 28,5 (CF₃CHCl*C*H₂); 39,8 (*C*H₂N); 49,5 (q, *J* = 31 Hz, CF₃*C*H); 123,5 (2x*C*H_{Ar.}(CqCH=*C*H)); 125,0 (q, *J =* 281 Hz, *C*F₃); 133,5 (2x*Cq*_{Ar}); 148,5 (2x*C*H_{Ar.}(Cq*C*H=CH)); 168,8 (2x*C*=O_{Ar.}).

IR (v, cm⁻¹)(CCl₄)

2940; 1774; 1502; 1408; 1373; 1346; 1245; 1167; 1127.

Masse (IC, NH3)

307 (MH⁺); 323 (MNH₄⁺).

| Microanalyse | Elément: | Carbone | Hydrogène |
|---|---|---|---|
| | Calculé (%) | 51,08 | 3,63 |
| | Trouvé (%) | 51,01 | 3,58 |

## Revendications

1. Composé de formule générale (I) : 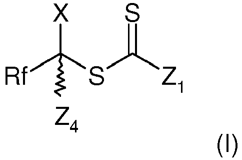 dans laquelle
- X représente un groupe -NZ₂Z₃, -OZ₅ ou un atome d'halogène (Hal) choisi parmi CI, Br et I, où
- Z₂ et Z₃ représentent, indépendamment l'un de l'autre, un atome d'hydrogène, un groupe choisi parmi les alkyles, cycloalkyles, aryles, et les groupements électroattracteurs, étant entendu que l'un au moins des radicaux Z₂ et Z₃ présente avantageusement un effet électroattracteur vis-à-vis de la densité électronique de l'atome d'azote auquel ils sont liés,
- Z₂ et Z₃ peuvent être liés pour former un hétérocycle avec l'atome d'azote,
- Z₅ représente un atome d'hydrogène, un groupe choisi parmi les alkyles, cycloalkyles, aryles ou les groupements électroattracteurs vis-à-vis de la densité électronique de l'atome d'oxygène auquel il est lié,
- Z₁ représente un groupe choisi parmi :
(i) les groupes alkyle, acyle, aryle, aralkyle, alcène ou alcyne, les cycles hydrocarbonés ou les hétérocycles,
(ii) un groupe -OR^{a} ou -SR^{a} dans lequel R^{a} est un groupement choisi parmi:
- un groupement alkyle, halogénoalkyle, alcényle, alcynyle, acyle, aryle, arylalkyle, arylalcényle, arylalcynyle, ou bien un cycle hydrocarboné ou un hétérocycle, ou bien une chaîne polymère ;
- un groupement -CR^{b}R^{c}PO(OR^{d})(OR^{e}) dans lequel :
• R^{b} et R^{c} représentent chacun, indépendamment l'un de l'autre, un atome d'hydrogène, un atome d'halogène, un groupe alkyle, perfluoroalkyle, un cycle hydrocarboné ou un hétérocycle, ou bien encore un groupement -NO₂, -NCO, CN, ou un groupement choisi parmi les groupements de type -R^{f}, -SO₃R^{f}, -OR^{f}, -SR^{f}, -NR^{f}R^{g}, -COOR^{f}, -O₂CR^{f}, -CONR^{f}R^{g}, -NCOR^{f}R^{g}, dans lesquels R^{f} et R^{g} désignent chacuns, de façon indépendante, un groupement alkyle, alcényle, alcynyle, cycloalcényle, cycloalcynyle, aryle, éventuellement condensé à un hétérocycle, alkaryle, arylalkyle, hétéroaryle,
• ou bien R^{b} et R^{c} forment ensemble avec l'atome de carbone auxquels ils sont rattachés un groupement C=O ou C=S ou bien un cycle hydrocarboné ou un hétérocycle ; et
• R^{d} et R^{e} représentent chacun, indépendamment l'un de l'autre, un radical répondant à une des définitions données ci-dessus pour le groupement R^{f} ;
• ou bien R^{d} et R^{e} forment ensemble une chaîne hydrocarbonée comportant de 2 à 4 atomes de carbone, éventuellement interrompue par un groupement choisi parmi -O-, -S- et -NR^{h}-; où R^{h} répond à l'une des définitions données ci-dessus pour le groupement R^{f} ;
(iii) un groupement -NRⁱR^{j}, dans lequel :
- Rⁱ et R^{j}, représentent indépendamment l'un de l'autre un radical choisi parmi un groupement alkyle, halogénoalkyle, alcényle, alcynyle, acyle, ester, aryle, arylalkyle, arylalcényle, arylalcynyle, ou bien encore un cycle hydrocarboné ou un hétérocycle ; ou
- Rⁱ et R^{j} forment ensemble une chaîne hydrocarbonée, comportant de 2 à 4 atomes de carbone, éventuellement interrompue par un groupement -O-, -S-, ou -NR^{H}- où R^{H} répond à l'une des définitions données ci-dessus pour le groupement R^{f},
- Z₄ représente un atome d'hydrogène, un groupement alkyle ou cycloalkyle, et
- Rf représente
(i) un atome d'halogène, de préférence le fluor ;
(ii) fluoroalkyle ;
(iii) un radical aryle poly- ou per-halogéné, ou
(iv) un radical choisi parmi R_{A}-CF₂, R_{A}-CF₂-CF₂-, R_{A}-CF₂-CF(CF₃)-, CF₃-C(R_{A})F- et (CF₃)R_{A}- avec R_{A} choisi parmi un groupe alkyle, acyle, aryle, aralkyle, alcène ou alcyne, les cycles hydrocarbonés ou les hétérocycles,
et les sels de tels composés.

2. Composé selon la revendication 1, **caractérisé en ce qu'**il répond à la formule générale (Ia) : 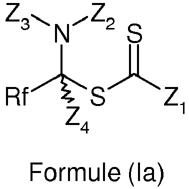 dans laquelle Z₁, Z₂, Z₃, Z₄ et Rf sont tels que définis dans la revendication 1.

3. Composé selon la revendication 2, dans laquelle Z₂ et Z₃ représentent, indépendamment l'un de l'autre, un atome d'hydrogène, un groupe choisi parmi les alkyles, cycloalkyles, aryles, et les groupements électroattracteurs, étant entendu que l'un au moins des radicaux Z₂ et Z₃ présente avantageusement un effet électroattracteur vis-à-vis de la densité électronique de l'atome d'azote auquel ils sont liés.

4. Composé selon la revendication 1, **caractérisé en ce qu'**il répond à la formule générale (Ib) : 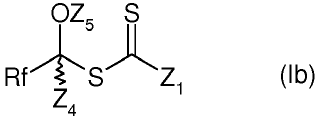 dans laquelle Z₁, Z₄, Z₅ et Rf sont tels que définis dans la revendication 1.

5. Composé selon la revendication 1, **caractérisé en ce qu'**il répond à la formule générale (Ic) : 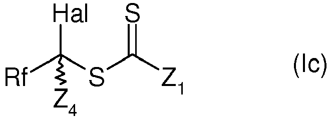 dans laquelle Rf, Z₁, Z₄ et Hal sont tels que définis dans la revendication 1.

6. Composé selon l'une des revendications précédentes, **caractérisé en ce que** Z₄ est un atome d'hydrogène.

7. Composé selon l'une quelconque des revendications précédentes, **caractérisé en ce que** Rf est un groupement perfluoroalkyle ou un radical aryle poly- ou perhalogéné comportant au moins un atome de fluor.

8. Composé selon la revendication 7, **caractérisé en ce que** le groupement perfluoroalkyle est le radical trifluorométhyle.

9. Composé selon l'une quelconque des revendications 1 à 4 et 6 à 8, **caractérisé en ce que** Z₅ ou l'un au moins des groupements Z₂ et Z₃ représente un groupement électroattracteur, comme les groupes acyle, aroyle, carboxyle, alkyloxycarbonyle, aryloxycarbonyle, aralkyloxycarbonyle, carbamoyle, alkylcarbamoyle, arylcarbamoyle, cyano-, sulfonyle, alkylsulfonyle, arylsulfonyle.

10. Composé selon la revendication 9, **caractérisé en ce que** Z₅ ou l'un au moins des groupes Z₂ et Z₃ représente un groupement électroattracteur acyle, alkoxycarbonyle ou aralkyloxycarbonyle.

11. Composé selon la revendication 10, **caractérisé en ce que** le groupement électroattracteur est choisi parmi les groupes acétyle, t-butoxycarbonyle et benzyloxycarbonyle.

12. Composé selon l'une quelconque des revendications 9 à 11, **caractérisé en ce que** l'autre groupe Z₂ ou Z₃ représente un atome d'hydrogène.

13. Composé selon l'une quelconque des revendications précédentes, **caractérisé en ce que** Z₁ représente un groupement -OR^{a}, R^{a} étant tel que défini dans la revendication 1.

14. Composé selon l'une quelconque des revendications précédentes **caractérisé en ce que** R^{a} représente un groupement alkyle.

15. Composé selon l'une quelconque des revendications 1, 5 à 8, 13 et 14, **caractérisé en ce que** le groupement Hal est un atome de chlore.

16. Composé selon l'une quelconque des revendications 1 ou 4, **caractérisé en ce que** Z₅ est un atome d'hydrogène.

17. Composé selon l'une des revendications précédentes, **caractérisé en ce qu'**il s'agit :
- du dithiocarbonate *S*-[1-(N-acétylamino)-2,2,2-trifluoroéthyl]-O-éthyle ;
- du diester de O-éthyle et de S-1-benzoylamino-2,2,2-trifluoro-éthyle de l'acide dithiocarbonique ;
- de l'ester d'O-éthyle et de S-(1-hydroxy-2,2,2 -trifluoro-éthyle) de l'acide dithiocarbonique ;
- de l'ester d'O-éthyle et de S-(1-acétyl-2,2,2-trifluoro-éthyle) de l'acide dithiocarbonique ;
- de l'ester de 1-éthoxythiocarbonylsulfanyl-2,2,2-trifluoro-éthyle de l'acide benzoïque ;
- de l'ester de O-éthyle et de S-1-chloro-2,2,2-trifluoro-éthyle de l'acide dithiocarbonique.

18. Procédé de préparation d'un composé de formule (Ib) dans laquelle Z₅ est différent de H comprenant :
a. la mise en oeuvre d'un composé tel que défini à la revendication 16 et d'un composé Z₅-Y, où Z₅ est tel que défini dans les revendications 1, 4, 9 à 11 et Y désigne un groupe partant ; et éventuellement
b. la récupération du produit obtenu.

19. Procédé de préparation d'un composé de formule (Ic) comprenant :
a. la mise en oeuvre d'un composé tel que défini à la revendication 16 en présence d'un agent d'halogénation ; et éventuellement
b. la récupération du produit obtenu.

20. Procédé de préparation d'un composé selon la revendication 16 comprenant :
a) la mise en oeuvre d'un composé de formule (A) : 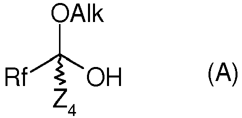 avec un acide minéral et un composé MS-(C=S)-Z₁ où Z₁ est tel que défini dans les revendications 1 à 17 et M désigne un métal alcalin et Alk désigne un groupe alkyle ; et le cas échéant
b) la récupération du produit obtenu.

21. Procédé de préparation d'un composé de formule (Ia), ledit procédé comprenant les étapes successives suivantes :
a) une substitution nucléophile de la fonction alkoxyle de l'hémiacétal Rf-C(OAlk)(OH)Z₄ (A) par addition d'un dérivé Z₂Z₃NH de façon à obtenir un composé de formule Rf-C(NZ₂Z₃)(OH)Z₄, où Alk désigne un groupe alkyle et où Rf, Z₂, Z₃ sont tels que définis dans les revendications 1 à 17,
b) une halogénation de la fonction hydroxyle du composé obtenu à l'issu de l'étape (a),
c) une substitution du groupement halogène introduit dans l'étape (b) par un dérivé thiocarbonylsulfanyle sous forme de sel de métal alcalin, MS-(CS)-Z₁, où Z₁ est tel que défini dans les revendications 1 à 17 et où M désigne un métal alcalin.

22. Utilisation en synthèse organique radicalaire d'un composé de formule (I), 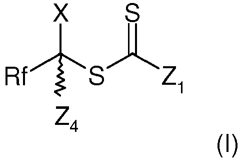 dans laquelle
- X représente un groupe -NZ₂Z₃, -OZ₅ ou un atome d'halogène (Hal) choisi parmi CI, Br et I, où
- Z₂ et Z₃ représentent, indépendamment l'un de l'autre, un atome d'hydrogène, un groupe choisi parmi les alkyles, cycloalkyles, aryles, et les groupements électroattracteurs, étant entendu que l'un au moins des radicaux Z₂ et Z₃ présente avantageusement un effet électroattracteur vis-à-vis de la densité électronique de l'atome d'azote auquel ils sont liés,
- Z₂ et Z₃ peuvent être liés pour former un hétérocycle avec l'atome d'azote,
- Z₅ représente un atome d'hydrogène, un groupe choisi parmi les alkyles, cycloalkyles, aryles ou les groupements électroattracteurs vis-à-vis de la densité électronique de l'atome d'oxygène auquel il est lié,
- Z₁ représente un groupe choisi parmi :
(i) les groupes alkyle, acyle, aryle, aralkyle, alcène ou alcyne, les cycles hydrocarbonés ou les hétérocycles,
(ii) un groupe -OR^{a} ou -SR^{a} dans lequel R^{a} est un groupement choisi parmi:
- un groupement alkyle, halogénoalkyle, alcényle, alcynyle, acyle, aryle, arylalkyle, arylalcényle, arylalcynyle, ou bien un cycle hydrocarboné ou un hétérocycle, ou bien une chaîne polymère ;
- un groupement -CR^{b}R^{c}PO(OR^{d})(OR^{e}) dans lequel :
• R^{b} et R^{c} représentent chacun, indépendamment l'un de l'autre, un atome d'hydrogène, un atome d'halogène, un groupe alkyle, perfluoroalkyle, un cycle hydrocarboné ou un hétérocycle, ou bien encore un groupement -NO₂, -NCO, CN, ou un groupement choisi parmi les groupements de type -R^{f}, -SO₃R^{f}, -OR^{f}, -SR^{f}, -NR^{f}R^{g}, -COOR^{f}, -O₂CR^{f}, -CONR^{f}R^{g}, -NCOR^{f}R^{g}, dans lesquels R^{f} et R^{g} désignent chacuns, de façon indépendante, un groupement alkyle, alcényle, alcynyle, cycloalcényle, cycloalcynyle, aryle, éventuellement condensé à un hétérocycle, alkaryle, arylalkyle, hétéroaryle,
• ou bien R^{b} et R^{c} forment ensemble avec l'atome de carbone auxquels ils sont rattachés un groupement C=O ou C=S ou bien un cycle hydrocarboné ou un hétérocycle ; et
• R^{d} et R^{e} représentent chacun, indépendamment l'un de l'autre, un radical répondant à une des définitions données ci-dessus pour le groupement R^{f} ;
• ou bien R^{d} et R^{e} forment ensemble une chaîne hydrocarbonée comportant de 2 à 4 atomes de carbone, éventuellement interrompue par un groupement choisi parmi -O-, -S- et -NR^{h}-; où R^{h} répond à l'une des définitions données ci-dessus pour le groupement R^{f} ;
(iii) un groupement -NRⁱR^{j}, dans lequel :
- Rⁱ et R^{j}, représentent indépendamment l'un de l'autre un radical choisi parmi un groupement alkyle, halogénoalkyle, alcényle, alcynyle, acyle, ester, aryle, arylalkyle, arylalcényle, arylalcynyle, ou bien encore un cycle hydrocarboné ou un hétérocycle ; ou
- Rⁱ et R^{j} forment ensemble une chaîne hydrocarbonée, comportant de 2 à 4 atomes de carbone, éventuellement interrompue par un groupement -O-, -S-, ou -NR^{H}- où R^{H} répond à l'une des définitions données ci-dessus pour le groupement R^{f},
- Z₄ représente un atome d'hydrogène, un groupement alkyle ou cycloalkyle, et
- Rf représente
(i) un atome d'halogène, de préférence le fluor ;
(ii) fluoroalkyle ;
(iii) un radical aryle poly- ou per-halogéné, ou
(iv) un radical choisi parmi R_{A}-CF₂, R_{A}-CF₂-CF₂-, R_{A}-CF₂-CF(CF₃)-, CF₃-C(R_{A})F- et (CF₃)R_{A}- avec R_{A} choisi parmi un groupe alkyle, acyle, aryle, aralkyle, alcène ou alcyne, les cycles hydrocarbonés ou les hétérocycles,
et les sels de tels composés,
à titre de source de radicaux :

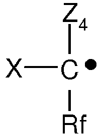

23. Utilisation selon la revendication 22, **caractérisée en ce qu'**il s'agit de l'utilisation d'un composé de formule (Ia), à titre de source de radicaux (Z₂Z₃N)(Rf)(Z₄)C^{•}.

24. Utilisation selon la revendication 22 pour introduire sur une oléfine un groupement :

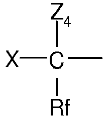

25. Utilisation selon la revendication 24, pour introduire un groupement (Z₂Z₃N)(Rf)(Z₄)C- sur une oléfine.

26. Utilisation selon la revendication 24 pour introduire sur une oléfine un des groupements suivants : 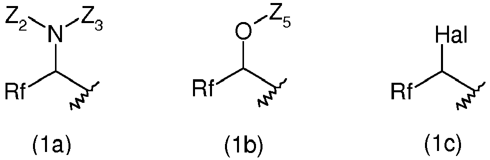 dans lesquels R_{f}, Z₂, Z₃, Z₅ et Hal sont tels que définis à la revendication 22.

27. Composé de formule (II) : 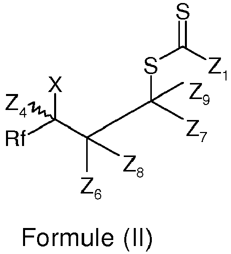 dans laquelle :
- X représente un groupe -NZ₂Z₃, -OZ₅ ou un atome d'halogène (Hal) choisi parmi CI, Br et I, où
- Z₂ et Z₃ représentent, indépendamment l'un de l'autre, un atome d'hydrogène, un groupe choisi parmi les alkyles, cycloalkyles, aryles, et les groupements électroattracteurs, étant entendu que l'un au moins des radicaux Z₂ et Z₃ présente avantageusement un effet électroattracteur vis-à-vis de la densité électronique de l'atome d'azote auquel ils sont liés,
- Z₂ et Z₃ peuvent être liés pour former un hétérocycle avec l'atome d'azote,
- Z₅ représente un atome d'hydrogène, un groupe choisi parmi les alkyles, cycloalkyles, aryles ou les groupements électroattracteurs vis-à-vis de la densité électronique de l'atome d'oxygène auquel il est lié,
- Rf représente
(i) un atome d'halogène, de préférence le fluor ;
(ii) fluoroalkyle ;
(iii) un radical aryle poly- ou per-halogéné, ou
(iv) un radical choisi parmi R_{A}-CF₂, R_{A}-CF₂-CF₂-, R_{A}-CF₂-CF(CF₃)-, CF₃-C(R_{A})F- et (CF₃)R_{A}- avec R_{A} choisi parmi un groupe alkyle, acyle, aryle, aralkyle, alcène ou alcyne, les cycles hydrocarbonés ou les hétérocycles,
- Z₁ et Z₄ sont tels que définis dans les revendications 1 à 17,
- Z₆, Z₇, Z₈ et Z₉ représentent indépendamment un atome d'hydrogène, un atome d'halogène, un groupe alkyle, halogénoalkyle, alcényle, alcynyle, acyle, aryle, arylalkyle, arylalcényle, arylalcynyle, ou bien un cycle hydrocarboné ou un hétérocycle, une chaîne polymère, un groupe -(CH₂)ₘ-OR^{k}, -(CH₂)ₘ-CH(OR^{k})(OR^{l}), CH(OR^{k})(OR^{l})-, -(CH₂)ₘ-SR^{k}, -(CH₂)ₘ-SO₃R^{k}, -(CH₂)ₘ-NO₂, -(CH₂)ₘ-CN, -(CH₂)ₘ-R^{k}, -[(CH₂)ₘ-P(O)(OR^{k})(OR^{l})], (CH₂)ₘ-SiR^{k}R^{l}R^{m}, -(CH₂)ₘ-COOR^{k}, -(CH₂)ₘ-NCOR^{k}, -(CH₂)ₘ-NR^{k}R^{l}, dans lesquels :
• R^{k}, R^{l} et R^{m} désignent chacun de façon indépendante un groupe alkyle, acyle, aryle, alcényle, alcynyle, aralkyle, alkaryle, alkylsulfonyle, arylsulfonyle, un cycle hydrocarboné ou un hétérocycle,
• ou bien R^{k} et R^{l} forment ensemble avec l'atome auquel ils sont rattachés, un cycle hydrocarboné ou un hétérocycle;
• m désignant un nombre entier supérieur ou égal à 1,
ou bien Z₆, Z₇, Z₈ et Z₉ forment deux à deux un ou plusieurs cycle(s) hydrocarboné(s) ou hétérocycle(s), les groupes Z₆, Z₇, Z₈ et Z₉ ne formant pas de cycle étant choisis parmi les radicaux précités.

28. Composé selon la revendication 27, dans laquelle X représente un groupe -NZ₂Z₃, -OZ₅ ou Hal choisi parmi CI, Br et I, où Z₂, Z₃, Z₅ et Hal sont tels que définis dans les revendications 1 à 17.

29. Composé selon l'une quelconque des revendications 27 ou 28, **caractérisé en ce qu'**il répond à la formule (IIa) : 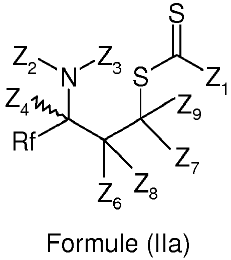 dans laquelle Z₁, Z₂, Z₃, Z₄, Z₆, Z₈, Z₉, Z₇ et Rf sont tels que définis dans l'une quelconque des revendications 27 ou 28.

30. Composé selon l'une quelconque des revendications 27 à 29 choisi parmi les composés suivants:
- l'ester de l'acide *S*-[1-(2-acétylamino-3,3,3-trifluoro-propyl)-4-oxo-pentyl] dithiocarbonique ester *O*-éthylique,
- l'ester de l'acide *S*-[5-(1-acétylamino-2,2,2-trifluoro-éthyl)-2-oxo-[1,3]dioxolan-4-yl] dithiocarbonique ester *O*-éthylique,
- l'ester de l'acide 3-acétylamino-1-éthoxythiocarbonylsulfanyl-4,4,4-trifluoro-butyle acétique,
- l'ester de l'acide *S*-(3-acétylamino-4,4,4-trifluoro-1-triméthyl-silanylméthyl-butyl) dithiocarbonique ester *O*-éthylique,
- l'ester de l'acide *S*-(3-acétylamino-1-cyanométhyl-4,4,4-trifluorobutyl) dithiocarbonique ester *O*-éthylique,
- l'ester de l'acide *S*-(3-acétylamino-1-diéthoxyméthyl-4,4,4-trifluorobutyl) dithiocarbonique ester *O*-éthylique,
- l'ester de l'acide *S*-[3-acétylamino-1-(1,3-dioxo-1,3-dihydro-isoindol-2-ylméthyl)-4,4,4-trifluoro-butyl] dithiocarbonique ester *O*-éthylique,
- l'ester de l'acide (4-acétylamino-2-éthoxythiocarbonylsulfanyl-5,5,5-trifluoro-pentyl)-phosphonique diéthylique,
- l'ester de l'acide 4-acétylamino-2-éthoxythiocarbonylsulfanyl-5,5,5-trifluoro-pentyl acétique,
- l'ester de l'acide *S*-[3-acétylamino-4,4,4-trifluoro-1-(2-oxo-pyrrolidin-1yl)-butyl] dithiocarbonique ester *O*-éthylique,
- l'ester de l'acide *S*-[3-acétylamino-1-{[(4-bromo-phényl) méthanesulfonyl-amino]-méthyl}-4,4,4-trifluoro-butyl) dithiocarbonique ester *O*-éthylique,
- l'ester de l'acide dithiocarbonique S-[1-(2-acétylamino-3,3,3-trifluoropropyl)-2-phényl-cyclopropane]O-éthyle,
- l'ester de l'acide acétique 4-benzoylamino-2-éthoxythio-carbonyl-sulfanyl-5,5,5-trifluoro-butyle,
- l'ester 4-tertbutyloxycarbamate-2-éthoxythiocarbonyl-sulfanyl-5,5,5-trifluoro-pentylique de l'acide acétique,
- l'ester O-éthylique *S*-(3-tertbutyloxycarbamate-1-diéthoxy-méthyl-4,4,4-trifluoro-butyle de l'acide dithiocarbonique,
- le diester de O-éthyle et de *S*-(3-tertbutyl-oxycarbamate-1-diéthoxy-méthyl-4,4,4-trifluoro-pentyle) de l'acide dithiocarbonique,
- le diester de O-éthyle et de S-[3-acétoxy-1-(1,3-dioxo-1,3-dihydro-isoindol-2-ylméthyl)-4,4,4-trifluoro-butyle] de l'acide dithiocarbonique,
- l'ester de O-éthyle et de S-(3-acétoxy-4,4,4-trifluoro-1-triméthyl-silanylméthyl-butyle) de l'acide dithiocarbonique,
- l'ester de 3-acétoxy-1-éthoxythiocarbonylsulfanyl-4,4,4-trifluoro-butyle de l'acide acétique,
- le diester de O-éthyle et de *S*-(3-acétoxy-1-diéthoxyméthyl-4,4,4-trifluoro-pentyle) de l'acide dithiocarbonique,
- l'ester de O-éthyle et de *S*-(3-acétoxy-1-cyanométhyl-4,4,4-trifluoro)butyle de l'acide dithiocarbonique,
- le diester de O-éthyle et de *S*-1-(2-acétoxy-3,3,3-trifluoro-propyl)-4-oxo-pentyle de l'acide dithiocarbonique,
- l'ester de 4-[4-bromo-phényl)-méthanesulfonyl-amino]-3-éthoxy-carbonylsulfanyl-1-trifluorométhyl-butyle de l'acide acétique,
- le diester de O-éthyle et de *S*-3-chloro-4,4,4-trifluoro-1-triméthylsilanylméthylbutyle de l'acide dithiocarbonique,
- l'ester de 4-chloro-2-éthoxythiocarbonylsulfanyl-5,5,5-trifluoro-pentyle de l'acide acétique,
- l'ester de O-éthyle et de *S*-3-chloro-1-(1,3-dioxo-1,3-dihydro-isoindol-2-ylméthyl)-4,4,4-trifluoro-butyle de l'acide dithio-carbonique,
- le diester de O-éthyle et de *S*-1-(2-chloro-3,3,3-trifluoro-propyl)-4-oxo-pentyle de l'acide dithiocarbonique,
- l'ester de diméthyle et de 4-Chloro-2-éthoxythiocarbonyl-sulfanyl-5,5,5-trifluoro-pentyle de l'acide phosphonique,
- le diester de O-éthyle et de *S*-3-chloro-1-cyanométhyl-4,4,4-trifluoro-butyle de l'acide dithiocarbonique,
- le diester de O-éthyle et de *S*-3-chloro-1-diéthoxyméthyl-4,4,4-trifluoro-pentyle de l'acide dithiocarbonique,
- le diester de O-éthyle et de S-3-chloro-1-(4-chloro-phénoxyméthyl)-4,4,4-trifluoro-butyle de l'acide dithio-carbonique,
- le diester de O-éthyle et de *S*-3-chloro-4,4,4-trifluoro-1-(2-oxo-pyrrolidin-1-yl)-butyle de l'acide dithiocarbonique.

31. Procédé de préparation d'un composé de formule (II) tel que défini à la revendication 27, ledit procédé comprenant la mise en réaction d'un composé de formule (I) tel que défini à la revendication 1, avec au moins une oléfine de formule (III) : 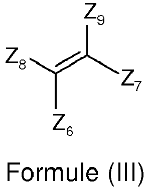 dans laquelle Z₆, Z₇, Z₈ et Z₉ sont tels que définis dans l'une quelconque des revendications 27 à 30, en présence d'une source de radicaux libres, dans un solvant organique inerte vis-à-vis des radicaux, et la récupération dudit composé de formule générale (II).

32. Procédé selon la revendication 31, **caractérisé en ce que** l'oléfine de formule (III) mise en oeuvre est choisie parmi : acétate de vinyle, hex-5-èn-2-one, acétate d'allyle, vinyltriméthylsilane, but-3-ènenitrile, 3,3-diéthoxypropène, diéthyle allylphosphonate.

33. Composé de formule générale (II) susceptible d'être obtenu selon le procédé tel que défini dans les revendications 31 à 32.

34. Procédé de préparation d'un composé de formule générale (IV) : 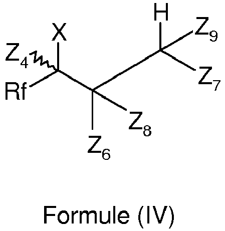 dans laquelle X, Rf, Z₄, Z₆, Z₇, Z₈ et Z₉ sont tels que définis dans l'une quelconque des revendications 27 à 30,
ledit procédé comprenant la mise en oeuvre d'un composé de formule (II) selon l'une quelconque des revendications 27 à 30 dans une réaction de réduction.

35. Procédé de préparation d'un composé de formule générale (V) : 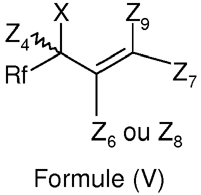 dans laquelle Rf, X, Z₄, Z₆, Z₇, Z₈ et Z₉ sont tels que définis dans les revendications 27 à 30,
ledit procédé comprenant la mise en oeuvre d'un composé de formule (II) dans laquelle l'un au moins des groupes Z₆ et Z₈ représente un atome d'hydrogène selon l'une quelconque des revendications 27 à 30 dans une réaction d'élimination.

36. Procédé de préparation d'un composé de formule générale (VI) : 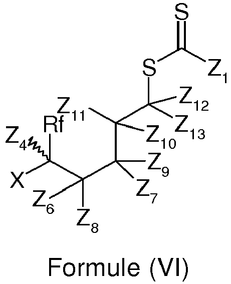 dans laquelle Rf, X, Z₄, Z₆, Z₇, Z₈ et Z₉ sont tels que définis dans les revendications 27 à 30, Z₁₀, Z₁₁, Z₁₂ et Z₁₃ répondant aux définitions précitées pour Z₆, Z₇, Z₈ et Z₉.
ledit procédé comprenant la mise en oeuvre d'un composé de formule (II) selon l'une quelconque des revendications 27 à 30 dans une réaction d'addition radicalaire sur une oléfine de formule:

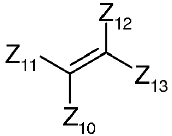

37. Procédé de préparation d'un composé de formule générale (VII) : 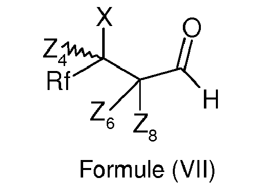 dans laquelle Rf, X, Z₄, Z₆, Z₈ sont tels que définis dans les revendications 27 à 30,
ledit procédé comprenant la mise en réaction d'un composé de formule (II) dans laquelle Z₇ et Z₉ représentent chacun un atome d'hydrogène et un groupement acyloxyle, en présence d'un acide organique ou minéral.

38. Composé choisi parmi:
- le *N*-[3-(2-oxo-pyrrolidin-1-yl)-1-trifluorométhyl-allyl] acétamide,
- le *N*-[4-(1,3-dioxo-1,3-dihydro-isoindol-2-yl)-1-trifluorométhyl-butyl] acétamide,
- l'ester de l'acide *S*-{1-[5-(1-acétylamino-2,2,2-trifluoro-éthyl)-2-oxo-[1,3]dioxolan-4-ylméthyl]-2,2-diéthoxy-éthyl} dithiocarbonique ester *O-*éthylique,
- le *N*-[1-(5-bromo-1-méthanesulfonyl-2,3-dihydro-1H-indol-3-ylméthyl)-2,2,2-trifluoro-éthyl]-acétamide,
- le *N*-(3,3-diméthoxy-1-trifluorométhyl-propyl)-acétamide,
- l'ester de l'acide *S*-{2-[5-(1-acétylamino-2,2,2-trifluoro-éthyl)-2-oxo-[1,3]dioxolan-4-yl]-1-triméthylsilanylméthyl-éthyl} dithiocarbonique ester *O-*éthylique,
- *N*-[1-(5-éthoxy-2-oxo-[1,3]dithiolan-4-ylméthyl)-2,2,2-trifluoro-éthyl]-acétamide,
- l'ester 4-benzoylamino-5,5-5-trifluoro-butylique de l'acide acétique,
- 4-acétoxy-5,5,5-trifluoro-pent-1-ène,
- l'ester de 1-[5-bromo-1-méthanesulfonyl-2,3-dihydro-1H-indol-3-ylméthyl)-2,2,2-trifluoro-éthyle] de l'acide acétique,
- l'ester de 2-benzoyloxy-3,3,3-trifluoro-1-trifluorométhyl-propyle de l'acide benzoïque,
- 1-(3-chloro-4,4,4-trifluoro-but-1-ènyl)-pyrrolidin-2-one,
- 2-(4-chloro-5,5,5-trifluoro-pentyl)-isoindole-1,3-dione.

39. Composé de formule générale (VIII) : 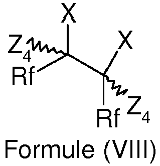 dans laquelle Z₄ est tel que défini dans les revendications 1 à 17,
- X représente un groupe -NZ₂Z₃, où
- Z₂ et Z₃ représentent, indépendamment l'un de l'autre, un atome d'hydrogène, un groupe choisi parmi les alkyles, cycloalkyles, aryles, et les groupements électroattracteurs, étant entendu que l'un au moins des radicaux Z₂ et Z₃ présente avantageusement un effet électroattracteur vis-à-vis de la densité électronique de l'atome d'azote auquel ils sont liés,
- et Rf représente
(i) un atome de fluor ;
(ii) un fluoroalkyle ;
(iii) un radical aryle per-halogéné, ou
(iv) un radical choisi parmi R_{A}-CF₂, R_{A}-CF₂-CF₂-, R_{A}-CF₂-CF(CF₃)-, CF₃-C(R_{A})F- avec R_{A} choisi parmi un groupe alkyle, acyle, aryle, aralkyle, alcène ou alcyne, les cycles hydrocarbonés ou les hétérocycles,
ou (CF₃)R_{A}- avec R_{A} choisi parmi un groupe alkyle, acyle, aralkyle, alcène ou alcyne, les cycles hydrocarbonés ou les hétérocycles.

40. Procédé de préparation d'au moins un composé de formule générale (VIII) tel que défini dans la revendication 39, ledit procédé comprenant :
- une étape de dimérisation radicalaire d'un composé de formule générale (I) 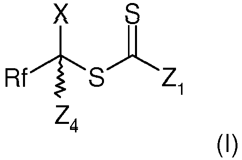 dans laquelle
- X représente un groupe -NZ₂Z₃, -OZ₅ ou un atome d'halogène (Hal) choisi parmi CI, Br et I, où
- Z₂ et Z₃ représentent, indépendamment l'un de l'autre, un atome d'hydrogène, un groupe choisi parmi les alkyles, cycloalkyles, aryles, et les groupements électroattracteurs, étant entendu que l'un au moins des radicaux Z₂ et Z₃ présente avantageusement un effet électroattracteur vis-à-vis de la densité électronique de l'atome d'azote auquel ils sont liés,
- Z₅ représente un atome d'hydrogène, un groupe choisi parmi les alkyles, cycloalkyles, aryles ou les groupements électroattracteurs vis-à-vis de la densité électronique de l'atome d'oxygène auquel il est lié,
- Z₁ représente un groupe choisi parmi :
(i) les groupes alkyle, acyle, aryle, aralkyle, alcène ou alcyne, les cycles hydrocarbonés ou les hétérocycles,
(ii) un groupe -OR^{a} ou -SR^{a} dans lequel R^{a} est un groupement choisi parmi:
- un groupement alkyle, halogénoalkyle, alcényle, alcynyle, acyle, aryle, arylalkyle, arylalcényle, arylalcynyle, ou bien un cycle hydrocarboné ou un hétérocycle, ou bien une chaîne polymère ;
- un groupement -CR^{b}R^{c}PO(OR^{d})(OR^{e}) dans lequel :
• R^{b} et R^{c} représentent chacun, indépendamment l'un de l'autre, un atome d'hydrogène, un atome d'halogène, un groupe alkyle, perfluoroalkyle, un cycle hydrocarboné ou un hétérocycle, ou bien encore un groupement -NO₂, -NCO, CN, ou un groupement choisi parmi les groupements de type -R^{f}, -SO₃R^{f}, -OR^{f}, -SR^{f}, -NR^{f}R^{g}, -COOR^{f}, -O₂CR^{f}, -CONR^{f}R^{g}, -NCOR^{f}R^{g}, dans lesquels R^{f} et R⁹ désignent chacuns, de façon indépendante, un groupement alkyle, alcényle, alcynyle, cycloalcényle, cycloalcynyle, aryle, éventuellement condensé à un hétérocycle, alkaryle, arylalkyle, hétéroaryle,
• ou bien R^{b} et R^{c} forment ensemble avec l'atome de carbone auxquels ils sont rattachés un groupement C=O ou C=S ou bien un cycle hydrocarboné ou un hétérocycle ; et
• R^{d} et R^{e} représentent chacun, indépendamment l'un de l'autre, un radical répondant à une des définitions données ci-dessus pour le groupement R^{f} ;
• ou bien R^{d} et R^{e} forment ensemble une chaîne hydrocarbonée comportant de 2 à 4 atomes de carbone, éventuellement interrompue par un groupement choisi parmi -O-, -S- et -NR^{h}-; où R^{h} répond à l'une des définitions données ci-dessus pour le groupement R^{f} ;
(iii) un groupement -NRⁱR^{j}, dans lequel :
- Rⁱ et R^{j}, représentent indépendamment l'un de l'autre un radical choisi parmi un groupement alkyle, halogénoalkyle, alcényle, alcynyle, acyle, ester, aryle, arylalkyle, arylalcényle, arylalcynyle, ou bien encore un cycle hydrocarboné ou un hétérocycle ; ou
- Rⁱ et R^{j} forment ensemble une chaîne hydrocarbonée, comportant de 2 à 4 atomes de carbone, éventuellement interrompue par un groupement -O-, -S-, ou -NR^{H}-, où R^{H} répond à l'une des définitions données ci-dessus pour le groupement R^{f},
- Z₄ représente un atome d'hydrogène, un groupement alkyle ou cycloalkyle, et
- Rf représente
(i) un atome d'halogène, de préférence le fluor ;
(ii) fluoroalkyle ;
(iii) un radical aryle poly- ou per-halogéné, ou
(iv) un radical choisi parmi R_{A}-CF₂, R_{A}-CF₂-CF₂-, R_{A}-CF₂-CF(CF₃)-, CF₃-C(R_{A})F- et (CF₃)R_{A}- avec R_{A} choisi parmi un groupe alkyle, acyle, aryle, aralkyle, alcène ou alcyne, les cycles hydrocarbonés ou les hétérocycles,
- et la récupération dudit composé de formule (VIII).

## Patentansprüche

1. Verbindung der allgemeinen Formel (I): 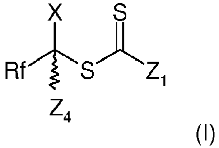 wobei
- X eine -NZ₂Z₃-, -OZ₅-Gruppe oder ein Halogenatom (Hal) ausgewählt aus Cl, Br und I darstellt, wobei
- Z₂ und Z₃, unabhängig voneinander, ein Wasserstoffatom, eine Gruppe ausgewählt aus Alkylen, Cycloalkylen, Arylen und Elektronen-ziehenden Gruppen darstellen, wobei mindestens eines der Radikale Z₂ und Z₃ vorzugsweise einen Elektronen-ziehenden Effekt gegenüber der Elektronendichte des Stickstoffatoms, an welches sie gebunden sind, aufweist,
- Z₂ und Z₃ verbunden sein können, um einen Heterozyklus mit dem Stickstoffatom zu bilden,
- Z₅ ein Wasserstoffatom, eine Gruppe ausgewählt aus Alkylen, Cycloalkylen, Arylen oder gegenüber der Elektronendichte des Sauerstoffatoms, an welches es gebunden ist, Elektronen-ziehenden Gruppen darstellt,
- Z₁ eine Gruppe darstellt ausgewählt aus:
(i) Alkyl-, Acyl-, Aryl-, Aralkyl, Alken- oder Alkin-Gruppen, Kohlenwasserstoffzyklen oder Heterozyklen,
(ii) einer -OR^{a} oder -SR^{a}-Gruppe, wobei R^{a} eine Gruppe ist ausgewählt aus:
- einer Alkyl-, Halogenalkyl-, Alkenyl-, Alkinyl-, Acyl-, Aryl-, Arylalkyl-, Arylalkenyl-, Arylalkinyl-Gruppe, oder einem Kohlenwasserstoffzyklus oder Heterozyklus, oder einer Polymerkette;
- einer -CR^{b}R^{c}PO(OR^{d})(OR^{e})-Gruppe, wobei:
• R^{b} und R^{c} jeweils unabhängig voneinander ein Wasserstoffatom, ein Halogenatom, eine Alkyl-, Perfluoroalkyl-Gruppe, einen Kohlenwasserstoffzyklus oder einen Heterozyklus, oder aber eine -non-, -NCO-, CN-Gruppe oder eine Gruppe ausgewählt aus den Gruppen der Sorte -R^{f}, -SO₃R^{f}, -OR^{f}, -SR^{f}-, -NR^{f}R^{g}, -COOR^{f}, -O₂CR^{f}, -CONR^{f}R^{g},-NCOR^{f}R^{g} darstellen, wobei R^{f} und R^{g} jeweils unabhängig eine Alkyl-, Alkenyl-, Alkinyl-, Cycloalkenyl-, Cycloalkinyl-, Aryl-, optional kondensiert mit einem Heterozyklus, Alkaryl, Arylalkyl, Heteroaryl bezeichnen,
• oder aber R^{b} und R^{c} bilden zusammen mit dem Kohlenstoffatom, an welches sie gebunden sind, eine C=O oder C=S-Gruppe oder einen Kohlenwasserstoffzyklus oder einen Heterozyklus; und
• R^{d} und R^{e} jeweils unabhängig voneinander ein Radikal entsprechend einer der vorangehend für die R^{f}-Gruppe gegebenen Definitionen darstellen;
• oder aber R^{d} und R^{e} bilden zusammen eine Kohlenwasserstoffkette mit 2 bis 4 Kohlenstoffatomen, optional unterbrochen durch eine Gruppe ausgewählt aus -O-, -S- und -NR^{h}-; wobei R^{h} einer der vorangehend für die R^{f}-Gruppe gegebenen Definitionen entspricht;
(iii) einer -NRⁱR^{j}-Gruppe, wobei:
- Rⁱ und Rⁱ unabhängig voneinander ein Radikal ausgewählt aus einer Alkyl-, Halogenalkyl-, Alkenyl-, Alkinyl-, Acyl-, Ester-, Aryl-, Arylalkyl-, Arylalkenyl-, Arylalkinyl-Gruppe oder einem Kohlenwasserstoffzyklus oder einem Heterozyklus darstellen; oder
- Rⁱ und R^{j} zusammen eine Kohlenwasserstoffkette mit 2 bis 4 Kohlenstoffatomen bilden, optional unterbrochen durch eine -O-, -S- oder -NR^{H}-Gruppe, wobei R^{H} einer der vorangehend für die Gruppe R^{f} gegebenen Definitionen entspricht,
- Z₄ ein Wasserstoffatom, eine Alkyl- oder Cycloalkyl-Gruppe darstellt und
- R^{f}
(i) ein Halogenatom, vorzugsweise Fluor;
(ii) Fluoroalkyl;
(iii) ein poly- oder perhalogeniertes Aryl-Radikal, oder
(iv) ein Radikal ausgewählt aus R_{A}-CF₂, R_{A}-CF₂-CF₂-, R_{A}-CF₂-CF(CF₃)-, CF₃-C(R_{A})F- und (CF₃)R_{A}- mit R_{A} ausgewählt aus einer Alkyl-, Acyl-, Aryl-, Aralkyl-, Alken- oder Alkin-Gruppe, Kohlenwasserstoffzyklen oder Heterozyklen
darstellt,
und die Salze dieser Verbindungen.

2. Verbindung gemäß Anspruch 1, **gekennzeichnet dadurch, dass** sie der allgemeinen Formel (Ia) entspricht: 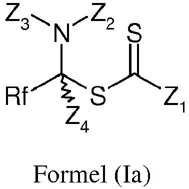 wobei Z₁, Z₂, Z₃, Z₄ und Rf wie in Anspruch 1 definiert sind.

3. Verbindung gemäß Anspruch 2, wobei Z₂ und Z₃ unabhängig voneinander ein Wasserstoffatom, eine Gruppe ausgewählt aus Alkylen, Cycloalkylen, Arylen und Elektronen-ziehenden Gruppen darstellen, wobei mindestens eines der Radikale Z₂ und Z₃ vorzugsweise einen Elektronen-ziehenden Effekt gegenüber der Elektronendichte des Stickstoffatoms, an welches sie gebunden sind, aufweist.

4. Verbindung gemäß Anspruch 1, **gekennzeichnet dadurch, dass** sie der allgemeinen Formel (Ib) entspricht,: 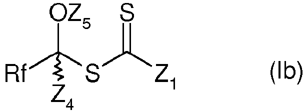 wobei Z₁, Z₄, Z₅ und Rf wie in Anspruch 1 definiert sind.

5. Verbindung gemäß Anspruch 1, **gekennzeichnet dadurch, dass** sie der allgemeinen Formel (Ic) entspricht: 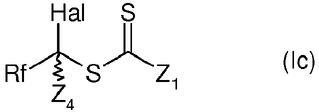 wobei Rf, Z₁, Z₄ und Hal wie in Anspruch 1 definiert sind.

6. Verbindung gemäß einem der voranstehenden Ansprüche, **gekennzeichnet dadurch, dass** Z₄ ein Wasserstoffatom ist.

7. Verbindung gemäß einem der voranstehenden Ansprüche, **gekennzeichnet dadurch, dass** Rf eine Perfluoroalkyl-Gruppe oder eine poly- oder perhalogeniertes Aryl-Radikal mit mindestens einem Fluoratom ist.

8. Verbindung gemäß Anspruch 7, **gekennzeichnet dadurch, dass** die Perfluoroalkyl-Gruppe das Radikal Trifluoromethyl ist.

9. Verbindung gemäß einem der Ansprüche 1 bis 4 und 6 bis 8, **gekennzeichnet dadurch, dass** Z₅ oder mindestens eine der Gruppen Z₂ und Z₃ eine Elektronen-ziehende Gruppe darstellt, wie Acyl-, Aroyl-, Carboxyl-, Alkyloxycarbonyl-, Aryloxycarbonyl-, Aralkyloxycarbonyl-, Carbamoyl-, Alkylcarbamoyl-, Arylcarbamoyl-, Cyano-, Sulfonyl-, Alkylsulfonyl-, Arylsulfonyl-Gruppen.

10. Verbindung gemäß Anspruch 9, **gekennzeichnet dadurch, dass** Z₅ oder mindestens eine der Gruppen Z₂ und Z₃ eine Elektronen-ziehende Acyl-, Alkoxycarbonyl oder Aralkyloxycarbonyl-Gruppe darstellt.

11. Verbindung gemäß Anspruch 10, **gekennzeichnet dadurch, dass** die Elektronen-ziehende Gruppe ausgewählt ist aus Acetyl-, t-Butoxycarbonyl- und Benzoyloxycarbonyl-Gruppen.

12. Verbindung gemäß einem der Ansprüche 9 bis 11, **gekennzeichnet dadurch, dass** die andere Z₂- oder Z₃-Gruppe ein Wasserstoffatom darstellt.

13. Verbindung gemäß einem der voranstehenden Ansprüche, **gekennzeichnet dadurch, dass** Z₁ eine -OR^{a}-Gruppe darstellt, wobei R^{a} wie in Anspruch 1 definiert ist.

14. Verbindung gemäß einem der voranstehenden Ansprüche, **gekennzeichnet dadurch, dass** R^{a} eine Alkyl-Gruppe darstellt.

15. Verbindung gemäß einem der Ansprüche 1, 5 bis 8, 13 und 14, **gekennzeichnet dadurch, dass** die Hal-Grappe ein Chloratom ist.

16. Verbindung gemäß einem der Ansprüche 1 bis 4, **gekennzeichnet dadurch, dass** Z₅ ein Wasserstoffatom ist.

17. Verbindung gemäß einem der voranstehenden Ansprüche, **gekennzeichnet dadurch, dass** sie ist:
- *S*-[1-(N-acetylamino)-2,2,2-trifluoroethyl]-O-ethyl-Dithiocarbonat;
- O-Ethyl- und S-1-Benzoylamino-2,2,2-trifluoroethyl-Diester der Dithiocarbonsäure;
- O-Ethyl- und S-(1-Hydroxy-2.2.2-trifluoroethyl)-Ester der Dithiocarbonsäure;
- O-Ethyl- und S-(1-Acetyl-2,2,2-trifluoroethyl)-Ester der Dithiocarbonsäure;
- 1-Ethoxythiocarbonylsulfanyl-2,2,2-trifluoroethyl)-Ester der Benzoesäure;
- O-Ethyle- und S-1-Chloro-2,2,2-tufluoroethyl-Ester der Dithiocarbonsäure.

18. Verfahren zur Herstellung einer Verbindung der Formel (Ib), wobei Z₅ verschieden von H ist, umfassend:
a. das Umsetzen einer wie in Anspruch 16 definierten Verbindung und einer Verbindung Z₅-Y, wobei Z₅ wie in den Ansprüchen 1, 4, 9 bis 11 definiert ist und Y eine Abgangsgruppe bezeichnet; und optional
b. das Gewinnen des erhaltenen Produktes.

19. Verfahren zur Herstellung einer Verbindung der Formel (Ic), umfassend:
a. das Umsetzen einer wie in Anspruch 16 definierten Verbindung in Anwesenheit eines Halogenierungsmittels; und optional
b. das Gewinnen des erhaltenen Produktes.

20. Verfahren zur Herstellung einer Verbindung gemäß Anspruch 16, umfassend:
a) das Umsetzen einer Verbindung der Formel (A): 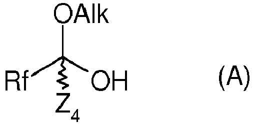 mit einer Mineralsäure und einer Verbindung MS-(C=S)-Z₁, wobei Z₁ wie in den Ansprüchen 1 bis 17 definiert ist und M ein Alkalimetall bezeichnet und Alk eine Alkyl-Gruppe bezeichnet; und gegebenenfalls
b) das Gewinnen des erhaltenen Produktes.

21. Verfahren zur Herstellung einer Verbindung der Formel (Ia), wobei das Verfahren die folgenden Schritte umfasst:
a) eine nukleophile Substitution der Alkoxyl-Funktion des Halbacetals Rf-C(OAlk)(OH)Z₄ (A) durch Addition eines Derivats Z₂Z₃NH, um eine Verbindung der Formel Rf-C(NZ₂Z₃)(OH)Z₄ zu erhalten, wobei Alk eine Alkyl-Gruppe bezeichnet und wobei Rf, Z₂, Z₃ wie in den Ansprüchen 1 bis 17 definiert sind,
b) eine Halogenierung der Hydroxyl-Funktion der aus Schritt (a) erhaltenen Verbindung,
c) eine Substitution der in Schritt (b) eingeführten Halogen-Gruppe durch ein Thiocarbonylsulfanyl-Derivat in Form des Alkalimetallsalzes, MS-(CS)-Z₁, wobei Z₁ wie in den Ansprüche 1 bis 17 definiert ist und M ein Alkalimetall bezeichnet.

22. Verwendung einer Verbindung der Formel (I) in einer organischen, radikalischen Synthese, 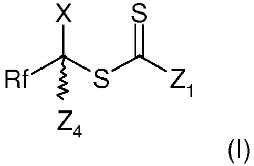 wobei
- X eine -NZ₂Z₃-, -OZ₅-Gruppe oder ein Halogenatom (Hal) ausgewählt aus Cl, Br und I darstellt, wobei
- Z₂ und Z₃, unabhängig voneinander, ein Wasserstoffatom, eine Gruppe ausgewählt aus Alkylen, Cycloalkylen, Arylen und Elektronen-ziehenden Gruppen darstellen, wobei mindestens eines der Radikale Z₂ und Z₃ vorzugsweise einen Elektronen-ziehenden Effekt gegenüber der Elektronendichte des Stickstoffatoms, an welches sie gebunden sind, aufweist,
- Z₂ und Z₃ verbunden sein können, um einen Heterozyklus mit dem Stickstoffatom zu bilden,
- Z₅ ein Wasserstoffatom, eine Gruppe ausgewählt aus Alkylen, Cycloalkylen, Arylen oder gegenüber der Elektronendichte des Sauerstoffatoms, an welches es gebunden ist, Elektronen-ziehenden Gruppen darstellt,
- Z₁ eine Gruppe darstellt ausgewählt aus:
(i) Alkyl-, Acyl-, Aryl-, Aralkyl, Alken- oder Alkin-Gruppen, Kohlenwasserstoffzyklen oder Heterozyklen,
(ii) einer -OR^{a} oder -SR^{a}-Gruppe, wobei R^{a} eine Gruppe ist ausgewählt aus:
- einer Alkyl-, Halogenalkyl-, Alkenyl-, Alkinyl-, Acyl-, Aryl-, Arylalkyl-, Arylalkenyl-, Arylalkinyl-Gruppe, oder einem Kohlenwasserstoffzyklus oder einem Heterozyklus, oder einer Polymerkette;
- einer -CR^{b}R^{c}PO(OR^{d})(OR^{e})-Gruppe, wobei:
• R^{b} und R^{c} jeweils unabhängig voneinander ein Wasserstoffatom, ein Halogenatom, eine Alkyl-, Perfluoroalkyl-Gruppe, einen Kohlenwasserstoffzyklus oder einen Heterozyklus, oder aber eine -NO₂-, -NCO-, CN-Gruppe oder eine Gruppe ausgewählt aus den Gruppen der Sorte R^{f}, -SO;R^{f}, -OR^{f}, -SR^{f}-, -NR^{f}R^{g}, -COOR^{f}, -O₂R^{f}, -CONR^{f}R^{g},-NCOR^{f}R^{g} darstellen, wobei R^{f} und R^{g} jeweils unabhängig eine Alkyl-, Alkenyl-, Alkinyl-, Cycloalkenyl-, Cycloalkinyl-, Aryl-, optional kondensiert an einen Heterozyklus, Alkaryl, Arylalkyl, Heteroaryl bezeichnen,
• oder aber R^{b} und R^{c} bilden zusammen mit dem Kohlenstoffatom, an welches sie gebunden sind, eine C=O oder C=S-Gruppe oder einen Kohlenwasserstoffzyklus oder einen Heterozyklus; und
• R^{d} und R^{e} jeweils unabhängig voneinander ein Radikal entsprechend einer der vorangehend für die R^{f}-Gruppe gegebenen Definitionen darstellen;
• oder aber R^{d} und R^{e} bilden zusammen eine Kohlenwasserstoffkette mit 2 bis 4 Kohlenstoffatomen, optional unterbrochen durch eine Gruppe ausgewählt aus -O-, -S- und -NR^{h}-; wobei R^{h} einer der vorangehend für die R^{f}-Gruppe gegebenen Definitionen entspricht;
(iii) einer -NRⁱR^{j}-Gruppe, wobei:
- Rⁱ und R^{j} unabhängig voneinander ein Radikal ausgewählt aus einer Alkyl-, Halogenalkyl-, Alkenyl-, Alkinyl-, Acyl-, Ester-, Aryl-, Arylalkyl-, Arylalkenyl-, Arylalkinyl-Gruppe oder einem Kohlenwasserstoffzyklus oder einem Heterozyklus darstellen; oder
- Rⁱ und R^{j} zusammen eine Kohlenwasserstoffkette mit 2 bis 4 Kohlenstoffatomen bilden, optional unterbrochen durch eine -O-, -S- oder -NR^{H}-Gruppe, wobei R^{H} einer der vorangehend für die Gruppe R^{f} gegebenen Definitionen entspricht,
- Z₄ ein Wasserstoffatom, eine Alkyl- oder Cycloalkyl-Gruppe darstellt und
- R^{f}
(i) ein Halogenatom, vorzugsweise Fluor;
(ii) Fluoroalkyl;
(iii) ein poly- oder perhalogeniertes Aryl-Radikal, oder
(iv) ein Radikal ausgewählt aus R_{A}-CF₂, R_{A}-CF₂-CF₂-, R_{A}-CF₂-CF(CF₃)-, CF₃-C(R_{A})F- und (CF₃)R_{A}- mit R_{A} ausgewählt aus einer Alkyl-, Acyl-, Aryl-, Aralkyl-, Alken- oder Alkin-Gruppe, Kohlenwasserstoffzyklen oder Heterozyklen
darstellt,
und die Salze dieser Verbindungen,
als Radikalquelle:

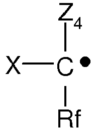

23. Verwendung gemäß Anspruch 22, **gekennzeichnet dadurch, dass** sie die Verwendung einer Verbindung der Formel (la) als Radikalquelle (Z₂Z₃N)(Rf)(Z₄)C• ist.

24. Verwendung gemäß Anspruch 22 zum Einführen einer Gruppe: 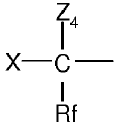 in ein Olefin.

25. Verwendung gemäß Anspruch 24, zum Einführen einer (Z₂Z₃N)(Rf)(Z₄)C-Gruppe in ein Olefin.

26. Verwendung gemäß Anspruch 24 zum Einführen einer der folgenden Gruppen: 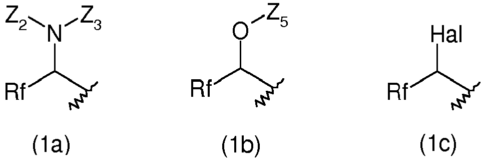 in ein Olefin, wobei R_{f}, Z₂, Z₃, Z₅ und Hal wie in Anspruch 22 definiert sind.

27. Verbindung der Formel (II): 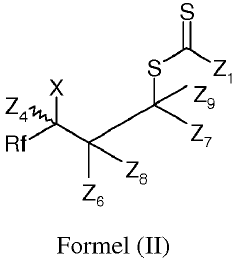 wobei
- X eine -NZ₂Z₃-, -OZ₅-Gruppe oder ein Halogenatom (Hal) ausgewählt aus Cl, Br und I darstellt, wobei
- Z₂ und Z₃, unabhängig voneinander, ein Wasserstoffatom, eine Gruppe ausgewählt aus Alkylen, Cycloalkylen, Arylen und Elektronen-ziehenden Gruppen darstellen, wobei mindestens eines der Radikale Z₂ und Z₃ vorzugsweise einen Elektronen-ziehenden Effekt gegenüber der Elektronendichte des Stickstoffatoms, an welches sie gebunden sind, aufweist,
- Z₂ und Z₃ verbunden sein können, um einen Heterozyklus mit dem Stickstoffatom zu bilden,
- Z₅ ein Wasserstoffatom, eine Gruppe ausgewählt aus Alkylen, Cycloalkylen, Arylen oder gegenüber der Elektronendichte des Sauerstoffatoms, an welches es gebunden ist, Elektronen-ziehenden Gruppen darstellt,
- R^{f}
(i) ein Halogenatom, vorzugsweise Fluor;
(ii) Fluoroalkyl;
(iii) ein poly- oder perhalogeniertes Aryl-Radikal, oder
(iv) ein Radikal ausgewählt aus R_{A}-CF₂, R_{A}-CF₂-CF₂-, R_{A}-CF₂-CF(CF₃)-, CF₃-C(R_{A})F- und (CF₃)R_{A}- mit R_{A} ausgewählt aus einer Alkyl-, Acyl-, Aryl-, Aralkyl-, Alken- oder Alkin-Gruppe, Kohlenwasserstoffzyklen oder Heterozyklen
darstellt,
- Z₁ und Z₄ wie in den Ansprüchen 1 bis 17 definiert sind,
- Z₆, Z₇, Z₈ und Z₉ unabhängig ein Wasserstoffatom, ein Halogenatom, eine Alkyl-, Halogenalkyl-, Alkenyl-, Alkinyl-, Acyl-, Aryl-, Arylalkyl-, Arylalkenyl-, Arylalkinyl-Gruppe oder einen Kohlenwasserstoffzyklus oder einen Heterozyklus, eine Polymerkette, eine -(CH₂)ₘ-OR^{k}, -(CH₂)ₘ-CH(OR^{k})(OR^{l})-, CH(OR^{k})(OR^{l})-, -(CH₂)ₘ-SR^{k}-, -(CH₂)ₘ-SO₃R^{k}-, -(CH₂)ₘ-NO₂-, -(CH₂)ₘ-CN-, -(CH₂)ₘ-R^{k}-, -[(CH₂)ₘ-P(O)(OR^{k})(OR^{l})]-, (CH₂)ₘ-SiR^{k}R^{l}R^{m}-, -(CH₂)ₘ-COOR^{k}-, -(CH₂)ₘ-NCOR^{k}-, -(CH₂)ₘ-NR^{k}R^{l}-Gruppe darstellen, wobei
• R^{k}, R^{l} und Rₘ jeweils unabhängig eine Alkyl-, Acyl-, Aryl-, Alkenyl-, Alkinyl-, Arylalkyl-, Alkaryl-, Alkylsulfonyl-, Arylsulfonyl-Gruppe, einen Kohlenwasserstoffzyklus oder einen Heterozyklus bezeichnen,
• oder aber R^{k} und R^{l} bilden gemeinsam mit dem Atom, an welches sie gebunden sind, einen Kohlenwasserstoffzyklus oder Heterozyklus;
• m eine ganze Zahl größer als oder gleich 1 bezeichnet,
oder aber Z₆, Z₇, Z₈ und Z₉ bilden paarweise einen oder mehrere Kohlenwasserstoffzyklus/en oder Heterozyklus/en, wobei die Gruppen Z₆, Z₇, Z₈ und Z₉ keinen Zyklus bilden, der ausgewählt ist aus den oben genannten Radikalen.

28. Verbindung gemäß Anspruch 27, wobei X eine -NZ₂Z₃-, -OZ₅- oder Hal-Gruppe ausgewählt aus Cl, Br und I darstellt, wobei Z₂, Z₃, Z₅ und Hal wie in den Ansprüchen 1 bis 17 definiert sind.

29. Verbindung gemäß einem der Ansprüche 27 oder 28, **gekennzeichnet dadurch, dass** sie der Formel (IIa) entspricht: 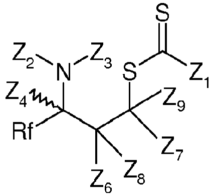 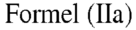 wobei Z₁, Z₂, Z₃, Z₄, Z₆, Z₈, Z₉, Z₇ und Rf wie in einem der Ansprüche 27 oder 28 definiert sind.

30. Verbindung gemäß einem der Ansprüche 27 bis 29, ausgewählt aus den folgenden Verbindungen:
- S-[1-(2-Acetylamino-3,3,3-trifluoropropyl)-4-oxo-pentyl]dithiocarbonsäureester-O-ethylester,
- S-[5-(1-Acetylamino-2,2,2-trifluoroethyl)-2-oxo-[1,3]dioxalan-4-yl] dithiocarbonsäureester-O-ethylester,
- 3-Acetylamino-1-ethoxythiocarbonylsulfanyl-4,4,4-trifluorobutylessigester,
- S-(3-Acetylamino-4,4,4-trifluoro-1-tlimethylsilanylmethylbutyl) dithiocarbonsäureester-O-ethylester,
- S-(3-Acetylamino-1-cyanomethyl-4,4,4-trifluorobutyl)dithiocarbonsäureester-O-ethylester
- S-(3-Acetylamino-1-diethoxymethyl-4,4,4-trifluorobutyl)dithiocarbonsäureester- O -ethylester,
- S-[3-Acetylamino-1-(1,3-dioxo-1,3-dihydroisoindol-2-ylmethyl)-4,4,4-trifluoro-butyl]dithiocarbonsäureester-O-ethylester,
- (4-Acetylamino-2-ethoxythiocarbonylsulfanyl-5,5,5-trifluoropentyl)-phosphonsäurediethylester,
- 4-Acetylamino-2-ethoxythiocarbonylsulfanyl-5,5,5-trifluoropentylessigester,
- S-[3-Acetylamino-4,4,4-tufluoro-1-(2-oxo-pyrrolidin-1-yl)-butyl] dithiocarbonsäureester- O-ethylester,
- S-[3-Acetylamino-l-{[(4-bromophenyl)methansulfonyl-amino]-methyl}-4,4,4-trfluorobutyl)dithiocarbonsäureester-O-ethylester,
- S-[1-(2-Acetylamino-3,3,3-trifluoropropyl)-2-phenylcyclopropan] dithiocarbonsäure-O-ethylester,
- 4-Benzoylamino-2-ethoxythio-carbonylsulfanyl-5,5,5-trifluoro-butyleessigsäureester,
- Essigsäure-4-tert-butyloxycarbamate-2-ethoxythiocarbonyl-sulfanyl-5,5,5-trifluoro-pentylester,
- S-(3-tert-Butyloxycarbamate-1-diethoxymethyl-4,4,4-trifluorobutyl-dithiocarbonsäure-O-ethylester,
- S-(3-tert-Butyl-oxycarbamate-1-diethoxymethyl-4,4,4-trifluoropentyl) dithiocarbonsäureester-O-ethylester,
- S-[3-Acetoxy-l-(l,3-dioxo-l,3-dihydroisoindol-2-ylmethyl)-4,4,4-trifluoro-butyl] dithiocarbonsäureester-O-ethylester,
- S-(3-Acetoxy-4,4,4-trifluoro-1-trimethylsilanylmethylbutyl)dithiocarbonsäureester-O-ethylester,
- Essigsäure-3-acetoxy-1-ethoxythiocarbonylsulfanyl-4,4,4-trifluorobutylester,
- S-(3-Acetoxy-1-diethoxymethyl-4,4,4-trifluoropentyl)dithiocarbonsäureester-O-ethylester,
- S-(3-Acetoxy-1-cyanomethyl-4,4,4-trifluoro)butyldithiocarbonsäureester-O-ethylester,
- S-1-(2-Acetoxy-3,3,3-trifluoropropyl)-4-oxopentyldithiocarbonsäureester-O-ethylester
- Essigsäure-4-[4-bromophenyl)-methansulfonylamino]-3-ethoxy-carbonylsulfanyl-1-trifluoromethylbutylester
- S-3-Chloro-4,4,4-trifluoro-1-trimethylsilanylmethylbutyldithiocarbonsäureester-O-ethylester,
- Essigsäure-4-chloro-2-ethoxythiocarbonylsulfanyl-5,5, 5-trifluoro-pentyl,
- S-3-Chloro-1-(1,3-dioxo-1,3-dihydro-isoindol-2-ylmethyl)-4,4,4-trifluorobutyl dithiocarbonsäureester-O-ethylester,
- S-1-(2-Chloro-3,3,3-trifluoropropyl)-4-oxo-pentyldithiocarbonsäureester-O-ethylester,
- 4-Chloro-2-ethoxythiocarbonyl-sulfanyl-5,5,5-trifluoropentylphosphonsäureester-dimethylester,
- S-3-Chloro-1-cyanomethyl-4,4,4-trifluorobutyldithiocarbonsäureester-O-ethylester,
- S-3-Chloro-1-diethoxymethyl-4,4,4- trifluoropentyldithiocarbonsäureester-O-ethylester,
- S-3-Chloro-1-(4-chloro-phenoxymethyl)-4,4,4-trifluorobutyldithiocarbonsäureester-O-ethylester,
- S-3-Chloro-4,4,4-trifluoro-1-(2-oxo-pyrrolidin-1-yl)-butyldithiocarbonsäureester-O-ethylester.

31. Verfahren zur Herstellung einer Verbindung der Formel (II) wie in Anspruch 27 definiert, wobei das Verfahren das Reagieren-lassen einer Verbindung der Formel (I) wie in Anspruch 1 definiert mit mindestens einem Olefin der Formel (III): 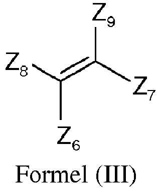 wobei Z₆, Z₇, Z₈ und Z₉ wie in einem der Ansprüche 27 bis 30 definiert sind, in Anwesenheit einer freien-Radikal-Quelle, in einem gegenüber Radikalen inerten, organischen Lösemittel, und die Gewinnung der Verbindung der allgemeinen Formel (II) umfasst.

32. Verfahren gemäß Anspruch 31, **gekennzeichnet dadurch, dass** das umgesetzte Olefin der Formel (III) ausgewählt ist aus: Vinylacetat, Hex-5-en-2-on, Allylacetat, Vinyltrimethylsilan, 3-Butennitril, 3,3-Diethoxypropen, Diethylallylphosphonat.

33. Verbindung der allgemeinen Formel (II), erhältlich gemäß des Verfahrens wie in den Ansprüchen 31 bis 32 definiert.

34. Verfahren zur Herstellung einer Verbindung der allgemeinen Formel (IV): 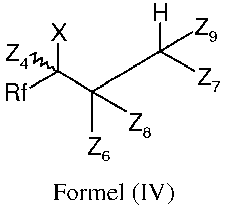 wobei X, Rf, Z₄, Z₆, Z₇, Z₈ und Z₉ wie in einem der Ansprüche 27 bis 30 definiert sind,
wobei das Verfahren das Umsetzen der Verbindung der Formel (II) gemäß einem der Ansprüche 27 bis 30 in einer Reduktionsreaktion umfasst.

35. Verfahren zur Herstellung einer Verbindung der allgemeinen Formel (V): 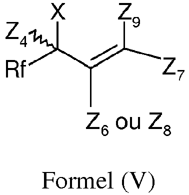 wobei Rf, X, Z₄, Z₆, Z₇, Z₈ und Z₉ wie in den Ansprüchen 27 bis 30 definiert sind, wobei das Verfahren das Umsetzen einer Verbindung der Formel (II), wobei mindestens eine der Gruppen Z₆ und Z₈ ein Wasserstoffatom darstellt, gemäß einem der Ansprüche 27 bis 30, in einer Eliminierungsreaktion umfasst.

36. Verfahren zur Herstellung einer Verbindung der allgemeinen Formel (VI): 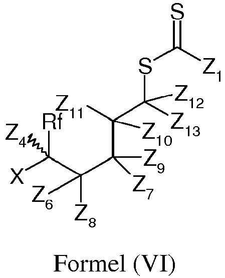 wobei Rf, X, Z₄, Z₆, Z₇, Z₈ und Z₉ wie in den Ansprüche 27 bis 30 definiert sind, Z₁₀, Z₁₁, Z₁₂ und Z₁₃ den oben genannten Definitionen für Z₆, Z₇, Z₈ und Z₉ entsprechen,
wobei das Verfahren das Umsetzen einer Verbindung der Formel (II) gemäß einem der Ansprüche 27 bis 30 in einer radikalischen Additionsreaktion an ein Olefin der Formel: 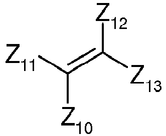 umfasst.

37. Verfahren zur Herstellung einer Verbindung der allgemeinen Formel (VII): 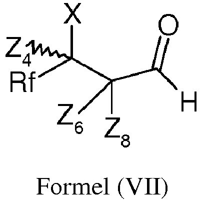 wobei Rf, X, Z₄, Z₆, Z₈ wie in den Ansprüche 27 bis 30 definiert sind,
wobei das Verfahren das Reagieren-lassen einer Verbindung der Formel (II), wobei Z₇ und Z₉ jeweils ein Wasserstoffatom und eine Acyloxy-Gruppe darstellen, in Anwesenheit einer organischen oder mineralischen Säure umfasst.

38. Verbindung ausgewählt aus:
- N-[3-(2-Oxo-pyrrolidin-1-yl)-1-trifluoromethylallyl]acetamid,
- N-[4-(1,3-Dioxo-1,3-dihydroisoindol-2-yl)-1-trifluoromethyl-butyl]acetamid,
- S-{1-[5-(1-Acetylamino-2,2,2-tufluoroethyl)-2-oxo-[1,3]dioxolan-4-ylmethyl]-2,2-diethoxy-ethyl}dithiocarbonsäureester-O-ethylester,
- N-[1-(5-Bromo-1-methansulfonyl-2,3-dihydro-1H-indol-3-yl-methyl)- 2,2,2-trifluoroethyl] acetamid,
- N-(3,3-Dimethoxy-1-trifluoromethylpropyl)-acetamid,
- S-{2-[5-(1-Acetylamino-2,2,2-trifluoroetyl)-2-oxo-[1,3]dioxalan-4-yl]-1-trimethylsilanylmethylethyl}dithiocarbonsäureester-O-ethylester
- N-[1-(5-Ethoxy-2-oxo-[1,3]dithiolan-4-ylmethyl)-2,2,2-trifluoroethyl]-acetamid,
- Essigsäure-4-benzoylamino-5,5-5-trifluoro-butylester,
- 4-Acetoxy-5,5,5-trifluoropent-1-en,
- Essigsäure-1-[5-bromo-1-methansulfonyl-2,3-dihydro-1H-indol-3- ylmethyl)-2,2,2 -trifluoroethyl]ester,
- 2-Benzoyloxy-3,3,3-trifluoro-1-trifluoromethylpropyl-benzoesäureester
- 1-(3-Chloro-4,4,4-trifluorobut-1-enyl)-pyrrolidin-2-on,
- 2-(4-Chloro-5,5,5-trifluoropentyl)-isoindol-1,3-dion.

39. Verbindung der allgemeinen Formel (VIII): 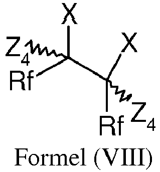 wobei Z₄ wie in den Ansprüche 1 bis 17 definiert ist,
- X eine -NZ₂Z₃-Gruppe darstellt, wobei
- Z₂ und Z₃, unabhängig voneinander, ein Wasserstoffatom, eine Gruppe ausgewählt aus Alkylen, Cycloalkylen, Arylen und Elektronen-ziehenden Gruppen darstellen, wobei mindestens eines der Radikale Z₂ und Z₃ vorzugsweise einen Elektronen-ziehenden Effekt gegenüber der Elektronendichte des Stickstoffatoms, an welches sie gebunden sind, aufweist,
- und Rf
(i) ein Fluoratom;
(ii) ein Fluoroalkyl;
(iii) ein poly- oder perhalogeniertes Aryl-Radikal, oder
(iv) ein Radikal ausgewählt aus R_{A}-CF₂, R_{A}-CF₂-CF₂-, R_{A}-CF₂-CF(CF₃)-, CF₃-C(R_{A})F- mit R_{A} ausgewählt aus einer Alkyl-, Acyl-, Aryl-, Aralkyl-, Alken- oder Alkin-Gruppe, Kohlenwasserstoffzyklen oder Heterozyklen, oder (CF₃)R_{A} mit R_{A} ausgewählt aus einer Alkyl-, Acyl-, Aralkyl-, Alken- oder Alkin-Gruppe, Kohlenwasserstoffzyklen oder Heterozyklen
darstellt.

40. Verfahren zur Herstellung mindestens einer Verbindung der allgemeinen Formel (VIII) wie in Anspruch 39 definiert, umfassend:
- einen Schritt der radikalischen Dimerisation einer Verbindung der allgemeinen Formel (I) 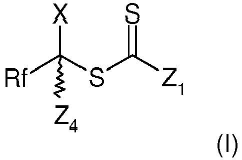 wobei
- X eine -NZ₂Z₃-, -OZ₅-Gruppe oder ein Halogenatom (Hal) ausgewählt aus Cl, Br und I darstellt, wobei
- Z₂ und Z₃, unabhängig voneinander, ein Wasserstoffatom, eine Gruppe ausgewählt aus Alkylen, Cycloalkylen, Arylen und Elektronen-ziehenden Gruppen darstellen, wobei mindestens eines der Radikale Z₂ und Z₃ vorzugsweise einen Elektronen-ziehenden Effekt gegenüber der Elektronendichte des Stickstoffatoms, an welches sie gebunden sind, aufweist,
- Z₅ ein Wasserstoffatom, eine Gruppe ausgewählt aus Alkylen, Cycloalkylen, Arylen oder gegenüber der Elektronendichte des Sauerstoffatoms, an welches sie gebunden sind, Elektronen-ziehenden Gruppen darstellt,
- Z₁ eine Gruppe darstellt ausgewählt aus:
(i) Alkyl-, Acyl-, Aryl-, Aralkyl, Alken- oder Alkin-Gruppen, Kohlenwasserstoffzyklen oder Heterozyklen,
(ii) einer -OR^{a} oder -SR^{a}-Gruppe, wobei R^{a} eine Gruppe ist ausgewählt aus:
- einer Alkyl-, Halogenalkyl-, Alkenyl-, Alkinyl-, Acyl-, Aryl-, Arylalkyl-, Arylalkenyl-, Arylalkinyl-Gruppe, oder einem Kohlenwasserstoffzyklus oder Heterozyklus, oder aber einer Polymerkette;
- einer -CR^{b}R^{c}PO(OR^{d})(OR^{e})-Gruppe, wobei:
• R^{b} und R^{c} jeweils unabhängig voneinander ein Wasserstoffatom, ein Halogenatom, eine Alkyl-, Perfluoroalkyl-Gruppe, einen Kohlenwasserstoffzyklus oder einen Heterozyklus, oder aber eine -NO₂-, -NCO-, CN-Gruppe oder eine Gruppe ausgewählt aus den Gruppen der Sorte R^{f}, -SO₃R^{f}, -OR^{f}, -SR^{f}, -NR^{f}R^{g}, -COOR^{f}, -O₂CR^{f}, -CONR^{f}R^{g},-NCOR^{f}R^{g} darstellen, wobei R^{f} und R^{g} jeweils unabhängig eine Alkyl-, Alkenyl-, Alkinyl-, Cycloalkenyl-, Cycloalkinyl-, Aryl-, optional kondensiert an einen Heterozyklus, Alkaryl, Arylalkyl, Heteroaryl bezeichnen,
• oder aber R^{b} und R^{c} bilden zusammen mit dem Kohlenstoffatom, an welches sie gebunden sind, eine C=O oder C=S-Gruppe oder aber einen Kohlenwasserstoffzyklus oder einen Heterozyklus; und
• R^{d} und R^{e} jeweils unabhängig voneinander ein Radikal entsprechend einer der vorangehend für die R^{f}-Gruppe gegebenen Definitionen darstellen;
• oder aber R^{d} und R^{e} bilden zusammen eine Kohlenwasserstoffkette mit 2 bis 4 Kohlenstoffatomen, optional unterbrochen durch eine Gruppe ausgewählt aus -O-, -S- und -NR^{h}-; wobei R^{h} einer der vorangehend für die R^{f}-Gruppe gegebenen Definitionen entspricht;
(iii) einer -NRⁱR^{j}-Gruppe, wobei:
- Rⁱ und R^{j} unabhängig voneinander ein Radikal ausgewählt aus einer Alkyl-, Halogenalkyl-, Alkenyl-, Alkinyl-, Acyl-, Ester-, Aryl-, Arylalkyl-, Arylalkenyl-, Arylalkinyl-Gruppe oder aber einen Kohlenwasserstoffzyklus oder einen Heterozyklus darstellen; oder
- Rⁱ und R^{j} zusammen eine Kohlenwasserstoffkette mit 2 bis 4 Kohlenstoffatomen bilden, optional unterbrochen durch eine -O-, -S- oder -NR^{H}-Gruppe, wobei R^{H} den vorangehend für die Gruppe R^{f} gegebenen Definitionen entspricht,
- Z₄ ein Wasserstoffatom, eine Alkyl- oder Cycloalkyl-Gruppe darstellt und
- R^{f}
(i) ein Halogenatom, vorzugsweise Fluor;
(ii) Fluoroalkyl;
(iii) ein poly- oder perhalogeniertes Aryl-Radikal, oder
(iv) ein Radikal ausgewählt aus R_{A}-CF₂, R_{A}-CF₂-CF₂-, R_{A}-CF₂-CF(CF₃)-, CF₃-C(R_{A})F- und (CF₃)R_{A}- mit R_{A} ausgewählt aus einer Alkyl-, Acyl-, Aryl-, Aralkyl-, Alken- oder Alkin-Gruppe, Kohlenwasserstoffzyklen oder Heterozyklen
darstellt,
- und die Gewinnung des Produkts der Formel (VIII).

## Claims

1. Compound having the general formula (I): 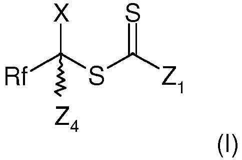 in which
- X represents a group -NZ₂Z₃, OZ₅ or a halogen atom (Hal) chosen from Cl, Br and I, wherein
- Z₂ and Z₃ represent, independently of each other, a hydrogen atom, a group selected from the alkyls, cycloalkyls, aryls and the electroattractive groups, it being understood that at least one of the radicals Z₂ and Z₃ advantageously has an electroattractive effect with respect to the electron density of the nitrogen atom to which they are bonded,
- Z₂ and Z₃ can be bonded in order to form a heterocycle with the nitrogen atom,
- Z₅ represents a hydrogen atom, a group selected from the alkyls, cycloalkyls, aryls or the groups which are electroattractive with respect to the electron density of the oxygen atom to which it is bonded,
- Z₁ represents a group selected from:
(i) the alkyl, acyl, aryl, aralkyl, alkene or alkyne groups, the cyclic hydrocarbons or the heterocycles,
(ii) a -OR^{a} or -SR^{a} group in which R^{a} is a group selected from :
- an alkyl, halogenoalkyl, alkenyl, alkynyl, acyl, aryl, arylalkyl, arylalkenyl, arylalkynyl group, or a cyclic hydrocarbon or a heterocycle, or a polymer chain;
- a -CR^{b}R^{c}PO(OR^{d})(OR^{e}) group in which :
• R^{b} and R^{c} each represent, independently of each other, a hydrogen atom, a halogen atom, an alkyl group, perfluoroalkyl, a cyclic hydrocarbon or a heterocycle, or an -NO₂, -NCO, CN group, or a group selected from groups of the type -R^{f}, -SO₃R^{f}, -OR^{f}, -SR^{f},-NR^{f}R^{g},
-COOR^{f}, -O₂CR^{f}, -CONR^{f}R^{g}, -NCOR^{f}R^{g}, in which R^{f} and R^{g} each independently refer to an alkyl, alkenyl, alkynyl, cycloalkenyl, cycloalkynyl, aryl group which is optionally condensed to a heterocycle, alkaryl, arylalkyl, heteroaryl,
• or R^{b} and R^{c} form, together with the carbon atom to which they are attached, a C=O or C=S group or a cyclic hydrocarbon or a heterocycle; and
• R^{d} and R^{e} each represent, independently of each other, a radical which complies with one of the definitions given above for the group R^{f};
• or R^{d} and R^{e} together form a hydrocarbon chain which comprises from 2 to 4 carbon atoms, and which is optionally interrupted by a group selected from -O-, -S- and -NR^{h}-; in which R^{h} complies with one of the definitions given above for the group R^{f};
(iii) a groupc -NRⁱR^{j}, in which:
- Rⁱ and R^{j} represent, independently of each other, a radical selected from an alkyl, halogenoalkyl, alkenyl, alkynyl, acyl, ester, aryl, arylalkyl, arylalkenyl, arylalkynyl group, or a cyclic hydrocarbon or a heterocycle; or
- Rⁱ and R^{j} together form a hydrocarbon chain which comprises from 2 to 4 carbon atoms and which is optionally interrupted by a O, S, or -NR^{H}, or R^{H} group which complies with one of the definitions given above for the R^{f} group,
- Z₄ represents a hydrogen atom, an alkyl or cycloalkyl group, and
- Rf represents
(i) a halogen atom, preferably fluorine;
(ii) fluoroalkyl;
(iii) a poly- or per-halogenated aryl radical, or
(iv) a radical selected from R_{A}-CF₂-, R_{A}-CF₂-CF₂-, R_{A}-CF₂-CF(CF₃)-, CF₃-C(R_{A})F- and (CF₃)R_{A}-, with R_{A} selected from an alkyl, acyl, aryl, aralkyl, alkene or alkyne group, the cyclic hydrocarbons or the heterocycles,
and the salts of compounds of this type.

2. Compound according to claim 1, **characterised in that** it complies with the general formula (Ia): 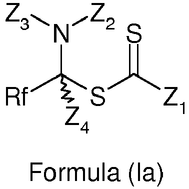 in which Z₁, Z₂, Z₃, Z₄ and R₄ are as defined in claims 1.

3. Compound according to claim 2, in which Z₂ and Z₃ represent, independently of each other, a hydrogen atom, a group selected from the alkyls, cycloalkyls, aryls, and the electroattractive groups, it being understood that at least one of the radicals Z₂ and Z₃ advantageously has an electroattractive effect with respect to the electron density of the nitrogen atom to which they are bonded.

4. Compound according to claim 1, **characterised in that** it complies with the general formula (Ib): 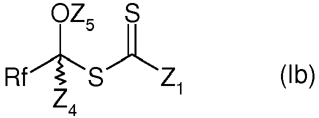 in which Z₁, Z₄, Z₅ and Rf are as defined in claims 1 to 2.

5. Compound according to claim 1, **characterised in that** it complies with the general formula (Ic): 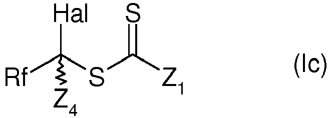 in which Rf, Z₁, Z₄ and Hal are as defined in claims 1.

6. Compound according to any one of the preceding claims, **characterised in that** Z₄ is a hydrogen atom.

7. Compound according to any one of the preceding claims, **characterised in that** Rf is a perfluoroalkyl group or a poly- or per-halogenated aryl radical comprising at least one fluorine atom.

8. Compound according to claim 7, **characterised in that** the perfluoroalkyl group is the trifluoromethyl radical.

9. Compound according to any one of claims 1 to 4 and 6 to 8, **characterised in that** Z₅ or at least one of the groups Z₂ and Z₃ represents an electroattractive group, such as the acyl, aroyl, carboxyl, alkyloxycarbonyl, aryloxycarbonyl, aralkyloxycarbonyl, carbamoyl, alkylcarbamoyl, arylcarbamoyl, cyano-, sulphonyl, alkylsulphonyl, arylsulphonyl groups.

10. Compound according to claim 9, **characterised in that** Z₅ or at least one of the groups Z₂ and Z₃ represents an electroattractive acyl, alkoxycarbonyl or aralkyloxycarbonyl group.

11. Compound according to claim 10, **characterised in that** the electroattractive group is selected from the acetyl, t-butoxycarbonyl and benzyloxycarbonyl groups.

12. Compound according to any one of claims 9 to 11, **characterised in that** the other group Z₂ or Z₃ represents a hydrogen atom.

13. Compound according to any one of the preceding claims, **characterised in that** Z₁ represents a -OR^{a}, R^{a} group as defined in claim 1.

14. Compound according to any one of the preceding claims, **characterised in that** R^{a} represents an alkyl group.

15. Compound according to any one of claims 1, 5 to 8, 13 and 14, **characterised in that** the Hal group is a chlorine atom.

16. Compound according to any one of claims 1 or 4, **characterised in that** Z₅ is a hydrogen atom.

17. Compound according to any one of the preceding claims, **characterised in that** it is:
- *S*-[1-(N-acetylamino)-2,2,2-trifluoroethyl]-*O*-ethyl dithiocarbonate;
- O-ethyl and S-1-benzoylamino-2,2,2-trifluoro-ethyl diester of dithiocarbonic acid;
- O-ethyl and S-(1-hydroxy-2,2,2-trifluoro-ethyl) ester of dithiocarbonic acid;
- O-ethyl and S-(1-acetyl-2,2,2-trifluoro-ethyl) ester of dithiocarbonic acid;
- 1-ethoxythiocarbonylsulphanyl-2,2,2-trifluoro-ethyl ester of benzoic acid;
- O-ethyl and S-1-chloro-2,2,2-trifluoro-ethyl ester of dithiocarbonic acid.

18. Method for preparing a compound having the formula (Ib) in which Z₅ is different from H comprising:
a. the use of a compound as defined in claim 16 and a compound Z₅-Y, in which Z₅ is as defined in claims 1, 4, 9 to 11 and Y refers to a leaving group; and optionally
b. the recovery of the product obtained.

19. Method for preparing a compound having the formula (Ic) comprising:
a. the use of a compound as defined in claim 16 in the presence of a halogenation agent; and optionally
b. the recovery of the product obtained.

20. Method for preparing a compound according to claim 16 comprising:
a) the use of a compound having the formula (A) : 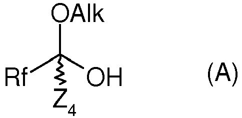 with a mineral acid and a compound MS-(C=S)-Z₁ in which Z₁ is as defined in claims 1 to 17 and M refers to an alkali metal and Alk refers to an alkyl group; and, if necessary
b) the recovery of the product obtained.

21. Method for preparing a compound having the formula (Ia), the method comprising the following successive steps:
a) a nucleophilic substitution of the alkoxyl function of the hemiacetal Rf-C(OAlk)(OH)Z₄ (A) by means of the addition of a Z₂Z₃NH derivative in order to produce a compound having the formula Rf-C(NZ₂Z₃)(OH)Z₄, in which Alk refers to an alkyl group and in which Rf, Z₂, Z₃ are as defined in claims 1 to 17,
b) a halogenation of the hydroxyl function of the compound produced when step (a) is complete,
c) a substitution of the halogen group introduced in step (b) by a thiocarbonylsulphanyl derivative in the form of an alkali metal salt, MS-(CS)-Z₁, in which Z₁ is as defined in claims 1 to 17 and in which M refers to an alkali metal.

22. Use of a compound having the formula (I) in organic radical synthesis, 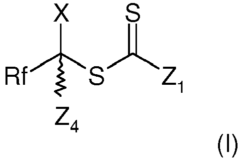 in which
- X represents a group -NZ₂Z₃, OZ₅ or a halogen atom (Hal) chosen from Cl, Br and I, wherein
- Z₂ and Z₃ represent, independently of each other, a hydrogen atom, a group selected from the alkyls, cycloalkyls, aryls and the electroattractive groups, it being understood that at least one of the radicals Z₂ and Z₃ advantageously has an electroattractive effect with respect to the electron density of the nitrogen atom to which they are bonded,
- Z₂ and Z₃ can be bonded in order to form a heterocycle with the nitrogen atom,
- Z₅ represents a hydrogen atom, a group selected from the alkyls, cycloalkyls, aryls or the groups which are electroattractive with respect to the electron density of the oxygen atom to which it is bonded,
- Z₁ represents a group selected from:
(i) the alkyl, acyl, aryl, aralkyl, alkene or alkyne groups, the cyclic hydrocarbons or the heterocycles,
(ii) a -OR^{a} or -SR^{a} group in which R^{a} is a group selected from :
- an alkyl, halogenoalkyl, alkenyl, alkynyl, acyl, aryl, arylalkyl, arylalkenyl, arylalkynyl group, or a cyclic hydrocarbon or a heterocycle, or a polymer chain;
- a -CR^{b}R^{c}PO(OR^{d})(OR^{e}) group in which :
• R^{b} and R^{c} each represent, independently of each other, a hydrogen atom, a halogen atom, an alkyl group, perfluoroalkyl, a cyclic hydrocarbon or a heterocycle, or an -NO₂, -NCO, CN group, or a group selected from groups of the type -R^{f}, -SO₃R^{f}, -OR^{f}, -SR^{f},-NR^{f}R^{g},
-COOR^{f}, -O₂CR^{f}, -CONR^{f}R^{g}, -NCOR^{f}R^{g}, in which R^{f} and R^{g} each independently refer to an alkyl, alkenyl, alkynyl, cycloalkenyl, cycloalkynyl, aryl group which is optionally condensed to a heterocycle, alkaryl, arylalkyl, heteroaryl,
• or R^{b} and R^{c} form, together with the carbon atom to which they are attached, a C=O or C=S group or a cyclic hydrocarbon or a heterocycle; and
• R^{d} and R^{e} each represent, independently of each other, a radical which complies with one of the definitions given above for the group R^{f};
• or R^{d} and R^{e} together form a hydrocarbon chain which comprises from 2 to 4 carbon atoms, and which is optionally interrupted by a group selected from -O-, -S- and -NR^{h}-; in which R^{h} complies with one of the definitions given above for the group R^{f};
(iii) a group -NRⁱR^{j}, in which:
- Rⁱ and R^{j} represent, independently of each other, a radical selected from an alkyl, halogenoalkyl, alkenyl, alkynyl, acyl, ester, aryl, arylalkyl, arylalkenyl, arylalkynyl group, or a cyclic hydrocarbon or a heterocycle; or
- Rⁱ and R^{j} together form a hydrocarbon chain which comprises from 2 to 4 carbon atoms and which is optionally interrupted by a O, S, or -NR^{H}, or R^{H} group which complies with one of the definitions given above for the R^{f} group,
- Z₄ represents a hydrogen atom, an alkyl or cycloalkyl group, and
- Rf represents
(i) a halogen atom, preferably fluorine;
(ii) fluoroalkyl;
(iii) a poly- or per-halogenated aryl radical, or
(iv) a radical selected from R_{A}-CF₂-, R_{A}-CF₂-CF₂-, R_{A}-CF₂-CF(CF₃)-, CF₃-C(R_{A})F- and (CF₃)R_{A}-, with R_{A} selected from an alkyl, acyl, aryl, aralkyl, alkene or alkyne group, the cyclic hydrocarbons or the heterocycles,
and the salts of compounds of this type,
as a source of radicals:

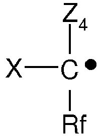

23. Use according to claim 22, **characterised in that** it is the use of a compound having the formula (Ia) as a source of radicals (Z₂Z₃N)(Rf)(Z₄)C.

24. Use according to claim 22 for introducing to an olefin a group:

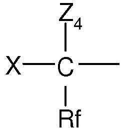

25. Use according to claim 24, for introducing a group (Z₂Z₃N)(Rf)(Z₄)C- to an olefin.

26. Use according to claim 24 for introducing to an olefin one of the following groups: 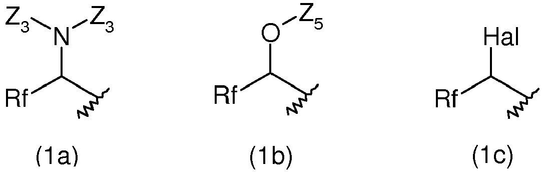 in which R_{f}, Z₂, Z₃, Z₅ and Hal are as defined in claim 22.

27. Compound having the formula (II): 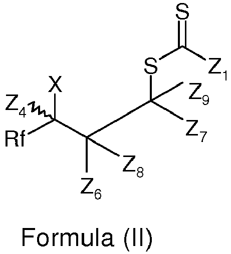 in which :
- X represents a group -NZ₂Z₃, OZ₅ or a halogen atom (Hal) chosen from Cl, Br and I, wherein
- Z₂ and Z₃ represent, independently of each other, a hydrogen atom, a group selected from the alkyls, cycloalkyls, aryls and the electroattractive groups, it being understood that at least one of the radicals Z₂ and Z₃ advantageously has an electroattractive effect with respect to the electron density of the nitrogen atom to which they are bonded,
- Z₂ and Z₃ can be bonded in order to form a heterocycle with the nitrogen atom,
- Z₅ represents a hydrogen atom, a group selected from the alkyls, cycloalkyls, aryls or the groups which are electroattractive with respect to the electron density of the oxygen atom to which it is bonded,
- Rf represents
(i) a halogen atom, preferably fluorine;
(ii) fluoroalkyl;
(iii) a poly- or per-halogenated aryl radical, or
(iv) a radical selected from R_{A}-CF₂, R_{A}-CF₂-CF₂-, R_{A}-CF₂-CF(CF₃)-, CF₃-C(R_{A})F- and (CF₃)R_{A}-, with R_{A} selected from an alkyl, acyl, aryl, aralkyl, alkene or alkyne group, the cyclic hydrocarbons or the heterocycles,
- Z₁ and Z₄ are as defined in claims 1 to 17,
- Z₆, Z₇, Z₈ and Z₉ independently represent a hydrogen atom, a halogen atom, an alkyl, halogenoalkyl, alkenyl, alkynyl, acyl, aryl, arylalkyl, arylalkenyl, arylalkynyl group, or a cyclic hydrocarbon or a heterocycle, a polymer chain, a group -(CH₂)ₘ-OR^{k}, -(CH₂)ₘ-CH(OR^{k})(OR^{l}), CH(OR^{k})(OR^{l})-, -(CH₂)ₘ-SR^{k},-(CH₂)ₘ-SO₃R^{k}, -(CH₂)ₘ-NO₂, -(CH₂)ₘ-CN, -(CH₂)ₘ-R^{k}, -[(CH₂)ₘ-P(O)(OR^{k})(OR^{l})], (CH₂)ₘ-SiR^{k}R^{l}R^{m}, -(CH₂)ₘ-COOR^{k}, -(CH₂)ₘ-NCOR^{k}, -(CH₂)ₘ-NR^{k}R^{l}, in which:
• R^{k}, R^{l} and R^{m} each independently refer to an alkyl, acyl, aryl, alkenyl, alkynyl, aralkyl, alkaryl, alkylsulphonyl, arylsulphonyl group, a cyclic hydrocarbon or a heterocycle,
• or R^{k} and R^{l} form, together with the atom to which they are attached, a cyclic hydrocarbon or a heterocycle;
• m referring to a whole number which is greater than or equal to 1, or Z₆, Z₇, Z₈ and Z₉ form, two by two, one or more cyclic hydrocarbon(s) or heterocycle(s), the groups Z₆, Z₇, Z₈ and Z₉ which do not form a ring being selected from the radicals mentioned above.

28. Compound according to claim 27, in which X represents -NZ₂Z₃, -OZ₅ or Hal group, in which Z₂, Z₃, Z₅ and Hal are as defined in claims 1 to 17.

29. Compound according to either claim 27 or 28, **characterised in that** it complies with the formula (IIa): 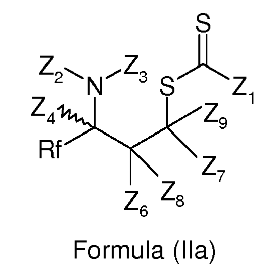 in which Z₁, Z₂, Z₃, Z₄, Z₆, Z₈, Z₉, Z₇ and Rf are as defined in either claim 27 or 28.

30. Compound according to any one of claims 27 to 29, selected from the following compounds:
- ester of *S*-[1-(2-acetylamino-3,3,3-trifluoro-propyl)-4-oxo-pentyl] dithiocarbonic acid O-ethyl ester,
- ester of S-[5-(1-acetylamino-2,2,2-trifluoro-ethyl)-2-oxo-[1,3]dioxolan-4-yl] dithiocarbonic acid O-ethyl ester,
- ester of 3-acetylamino-1-ethoxythiocarbonylsulphanyl-4,4,4-trifluoro-butyl acetic acid,
- ester of *S*-(3-acetylamino-4,4,4-trifluoro-1-trimethyl-silanylmethylbutyl) dithiocarbonic acid O-ethyl ester ,
- ester of *S*-(3-acetylamino-1-cyanomethyl-4,4,4-trifluoro-butyl) dithiocarbonic acid O-ethyl ester,
- ester of *S*-(3-acetylamino-1-diethoxymethyl-4,4,4-trifluoro-butyl) dithiocarbonic acid O-ethyl ester,
- ester of *S*-[3-acetylamino-1-(1,3-dioxo-1,3-dihydro-isoindol-2-ylmethyl)-4,4,4-trifluoro-butyl] dithiocarbonic acid O-ethyl ester,
- ester of (4-acetylamino-2-ethoxythiocarbonylsulphanyl-5,5,5-trifluoro-pentyl) diethyl phosphonic acid,
- ester of 4-acetylamino-2-ethoxythiocarbonylsulphanyl-5,5,5-trifluoro-pentyl acetic acid,
- ester of *S*-[3-acetylamino-4,4,4-trifluoro-1-(2-oxo-pyrrolidin-1yl)-butyl] dithiocarbonic acid *O*-ethyl ester,
- ester of *S*-[3-acetylamino-1-{[(4-bromo-phenyl) methane-sulphonyl-amino]-methyl}-4,4,4-trifluoro-butyl) dithiocarbonic acid O-ethyl ester,
- ester of S-[1-(2-acetylamino-3,3,3-trifluoro-propyl)-2-phenyl-cyclopropane] dithiocarbonic acid O-ethyl
- ester of 4-benzoylamino-2-ethoxythio-carbonyl-sulphanyl-5,5,5-trifluoro-butyl acetic acid,
- 4-tertbutyloxycarbamate-2-ethoxythiocarbonyl-sulphanyl-5,5,5-trifluoro-pentyl ester of acetic acid,
- O-ethyl and *S*-(3-tertbutyloxycarbamate-1-diethoxy-methyl-4,4,4-trifluoro-butyl ester of dithiocarbonic acid,
- O-ethyl and *S*-(3-tertbutyl-oxycarbamate-1-diethoxy-methyl-4,4,4-trifluoro-pentyl) diester of dithiocarbonic acid,
- O-ethyl and S-[3-acetoxy-1-(1,3-dioxo-1,3-dihydro-isoindol-2-ylmethyl)-4,4,4-trifluoro-butyl] diester of dithiocarbonic acid,
- O-ethyl and S-(3-acetoxy-4,4,4-trifluoro-1-trimethyl-silanylmethylbutyl) ester of dithiocarbonic acid,
- 3-acetoxy-1-ethoxythiocarbonylsulphanyl-4,4,4-trifluoro-butyl ester of acetic acid,
- O-ethyl and *S*-(3-acetoxy-1-diethoxymethyl-4,4,4-trifluoro-pentyl) diester of dithiocarbonic acid,
- O-ethyl and *S*-(3-acetoxy-1-cyanomethyl-4,4,4-trifluoro)butyl ester of dithiocarbonic acid,
- O-ethyl and *S*-1-(2-acetoxy-3,3,3-trifluoro-propyl)-4-oxo-pentyl diester of dithiocarbonic acid,
- 4-[4-bromo-phenyl)-methanesulphonyl-amino]-3-ethoxy-carbonylsulphanyl-1-trifluoromethyl-butyl ester of acetic acid,
- O-ethyl and *S*-3-chloro-4,4,4-trifluoro-1-trimethylsilanylmethylbutyl diester of dithiocarbonic acid,
- 4-chloro-2-ethoxythiocarbonylsulphanyl-5,5,5-trifluoro-pentyl ester of acetic acid,
- O-ethyl and *S*-3-chloro-1-(1,3-dioxo-1,3-dihydro-isoindol-2-ylmethyl)-4,4,4-trifluoro-butyl ester of,dithiocarbonic acid
- O-ethyl and *S*-1-(2-chloro-3,3,3-trifluoro-propyl)-4-oxo-pentyl diester of dithiocarbonic acid,
- Dimethyl and 4-chloro-2-ethoxythiocarbonyl-sulphanyl-5,5,5-trifluoro-pentyl ester of phosphonic acid,
- O-ethyl and *S*-3-chloro-1-cyanomethyl-4,4,4-trifluoro-butyl diester of dithiocarbonic acid,
- O-ethyl and *S*-3-chloro-1-diethoxymethyl-4,4,4-trifluoro-pentyl diester of dithiocarbonic acid,
- O-ethyl and *S*-3-chloro-1-(4-chloro-phenoxymethyl)-4,4,4-trifluorobutyl diester of dithiocarbonic acid,
- O-ethyl and *S*-3-chloro-4,4,4-trifluoro-1-(2-oxo-pyrrolidin-1-yl)-butyl diester of dithiocarbonic acid.

31. Method for preparing a compound having the formula (II) as defined in claim 27, the method comprising the reaction of a compound having the formula (I) as defined in claim 1, with at least one olefin having the formula (III): 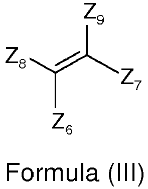 in which Z₆, Z₇, Z₈ and Z₉ are as defined in any one of claims 27 to 30, in the presence of a source of free radicals, in an organic solvent which is inert relative to radicals, and the recovery of the compound having the general formula (II).

32. Method according to claim 31, **characterised in that** the olefin having the formula (III) used is selected from: vinyl acetate, hex-5-en-2-one, allyl acetate, vinyltrimethylsilane, but-3-enenitrile, 3,3-diethoxypropene, diethyl allylphosphonate.

33. Compound having the general formula (II) which is capable of being produced according to the method as defined in claims 31 to 32.

34. Method for preparing a compound having the general formula (IV): 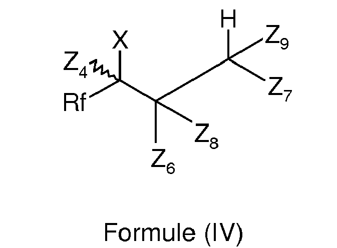 in which X, Rf, Z₄, Z₆, Z₇, Z₈ and Z₉ are as defined in any one of claims 27 to 30,
the method comprising the use of a compound having the formula (II) according to any one of claims 27 to 30 in a reduction reaction.

35. Method for preparing a compound having the general formula (V): 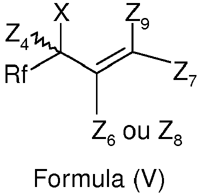 in which Rf, X, Z₄, Z₆, Z₇, Z₈ and Z₉ are as defined in claims 27 to 30,
the method comprising the use of a compound having the formula (II) in which at least one of the groups Z₆ and Z₈ represents a hydrogen atom according to any one of claims 27 to 30 in a removal reaction.

36. Method for preparing a compound having the general formula (VI): 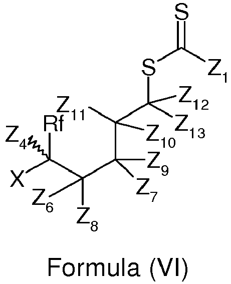 in which Rf, X, Z₄, Z₆, Z₇, Z₈ and Z₉ are as defined in claims 27 to 30, Z₁₀, Z₁₁, Z₁₂ and Z₁₃ complying with the above definitions for Z₆, Z₇, Z₈ and Z₉, the method comprising the use of a compound having the formula (II) according to any one of claims 27 to 30 in a reaction of radical addition to an olefin having the formula:

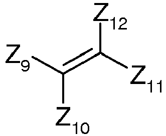

37. Method for preparing a compound having the general formula (VII): 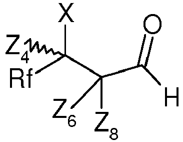 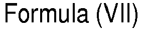 in which Rf, X, Z₄, Z₆, Z₈ are as defined in claims 27 to 30,
the method comprising the reaction of a compound having the formula (II), in which Z₇ and Z₉ each represent a hydrogen atom and an acyloxyl group, in the presence of an organic or mineral acid.

38. Compound selected from:
- *N*-[3-(2-oxo-pyrrolidin-1-yl)-1-trifluoromethyl-allyl] acetamide,
- *N*-[4-(1,3-dioxo-1,3-dihydro-isoindol-2-yl)-1-trifluoromethyl-butyl] acetamide,
- ester of *S*-{1-[5-(1-acetylamino-2,2,2-trifluoro-ethyl)-2-oxo-[1,3]dioxolan-4-ylmethyl]-2,2-diethoxy-ethyl} dithiocarbonic acid *O-*ethyl ester,
- *N*-[1-(5-bromo-1-methanesulphonyl-2,3-dihydro-1H-indol-3-ylmethyl)-2,2,2-trifluoro-ethyl]-acetamide,
- *N*-(3,3-dimethoxy-1-trifluoromethyl-propyl)-acetamide,
- ester of *S*-{2-[5-(1-acetylamino-2,2,2-trifluoro-ethyl)-2-oxo-[1,3]dioxolan-4-yl]-1-trimethylsilanylmethyl-ethyl} dithiocarbonic acid O-ethyl ester,
- *N*-[1-(5-ethoxy-2-oxo-[1,3]dithiolan-4-ylmethyl)-2,2,2-trifluoro-ethyl]-acetamide,
- 4-benzoylamino-5,5-5-trifluoro-butyl ester of acetic acid,
- 4-acetoxy-5,5,5-trifluoro-pent-1-ene,
- ester of 1-[5-bromo-1-methanesulphonyl-2,3-dihydro-1H-indol-3-ylmethyl)-2,2,2-trifluoro-ethyl] acetic acid,
- 2-benzoyloxy-3,3,3-trifluoro-1-trifluoromethyl-propyl ester of benzoic acid,
- 1-(3-chloro-4,4,4-trifluoro-but-1-enyl)-pyrrolidin-2-one,
- 2-(4-chloro-5,5,5-trifluoro-pentyl)-isoindole-1,3-dione.

39. Compound having the general formula (VIII): 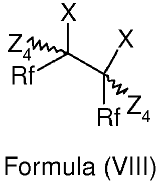 in which Z₄ is as defined in claims 1 to 17 and
- X represents a group -NZ₂Z₃, wherein
- Z₂ and Z₃ represent, independently of each other, a hydrogen atom, a group selected from the alkyls, cycloalkyls, aryls and the electroattractive groups, it being understood that at least one of the radicals Z₂ and Z₃ advantageously has an electroattractive effect with respect to the electron density of the nitrogen atom to which they are bonded,
- and Rf represents
(i) a fluorine atom;
(ii) a fluoroalkyl;
(iii) a poly- or per-halogenated aryl radical, or
(iv) a radical selected from R_{A}-CF₂, R_{A}-CF₂-CF₂-, R_{A}-CF₂-CF(CF₃)-, CF₃-C(R_{A})F- and (CF₃)R_{A}-, with R_{A} selected from an alkyl, acyl, aryl, aralkyl, alkene or alkyne group, the cyclic hydrocarbons or the heterocycles.

40. Method for preparing at least one compound having the general formula (VIII) as defined in claim 39, the method comprising:
- a step for radical dimerisation of a compound having the general formula (I) 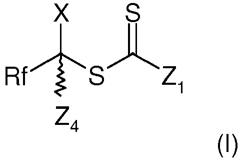 in which
- X represents a group -NZ₂Z₃, OZ₅ or a halogen atom (Hal) chosen from Cl, Br and I, wherein
- Z₂ and Z₃ represent, independently of each other, a hydrogen atom, a group selected from the alkyls, cycloalkyls, aryls and the electroattractive groups, it being understood that at least one of the radicals Z₂ and Z₃ advantageously has an electroattractive effect with respect to the electron density of the nitrogen atom to which they are bonded,
- Z₅ represents a hydrogen atom, a group selected from the alkyls, cycloalkyls, aryls or the groups which are electroattractive with respect to the electron density of the oxygen atom to which it is bonded,
- Z₁ represents a group selected from:
(i) the alkyl, acyl, aryl, aralkyl, alkene or alkyne groups, the cyclic hydrocarbons or the heterocycles,
(ii) a -OR^{a} or -SR^{a} group in which R^{a} is a group selected from :
- an alkyl, halogenoalkyl, alkenyl, alkynyl, acyl, aryl, arylalkyl, arylalkenyl, arylalkynyl group, or a cyclic hydrocarbon or a heterocycle, or a polymer chain;
- a -CR^{b}R^{c}PO(OR^{d})(OR^{e}) group in which :
• R^{b} and R^{c} each represent, independently of each other, a hydrogen atom, a halogen atom, an alkyl group, perfluoroalkyl, a cyclic hydrocarbon or a heterocycle, or an -NO₂, -NCO, CN group, or a group selected from groups of the type -R^{f}, -SO₃R^{f}, -OR^{f}, -SR^{f},-NR^{f}R^{g},
-COOR^{f}, -O₂CR^{f}, -CONR^{f}R^{g}, -NCOR^{f}R^{g}, in which R^{f} and R^{g} each independently refer to an alkyl, alkenyl, alkynyl, cycloalkenyl, cycloalkynyl, aryl group which is optionally condensed to a heterocycle, alkaryl, arylalkyl, heteroaryl,
• or R^{b} and R^{c} form, together with the carbon atom to which they are attached, a C=O or C=S group or a cyclic hydrocarbon or a heterocycle; and
• R^{d} and R^{e} each represent, independently of each other, a radical which complies with one of the definitions given above for the group R^{f};
• or R^{d} and R^{e} together form a hydrocarbon chain which comprises from 2 to 4 carbon atoms, and which is optionally interrupted by a group selected from -O-, -S- and -NR^{h}-; in which R^{h} complies with one of the definitions given above for the group R^{f};
(iii) a group -NRⁱR^{j}, in which:
- Rⁱ and R^{j} represent, independently of each other, a radical selected from an alkyl, halogenoalkyl, alkenyl, alkynyl, acyl, ester, aryl, arylalkyl, arylalkenyl, arylalkynyl group, or a cyclic hydrocarbon or a heterocycle; or
- Rⁱ and R^{j} together form a hydrocarbon chain which comprises from 2 to 4 carbon atoms and which is optionally interrupted by a O, S, or -NR^{H}, or R^{H} group which complies with one of the definitions given above for the R^{f} group,
- Z₄ represents a hydrogen atom, an alkyl or cycloalkyl group, and
- Rf represents
(i) a halogen atom, preferably fluorine;
(ii) fluoroalkyl;
(iii) a poly- or per-halogenated aryl radical, or
(iv) a radical selected from R_{A}-CF₂-, R_{A}-CF₂-CF₂-, R_{A}-CF₂-CF(CF₃)-, CF₃-C(R_{A})F- and (CF₃)R_{A}-, with R_{A} selected from an alkyl, acyl, aryl, aralkyl, alkene or alkyne group, the cyclic hydrocarbons or the heterocycles,
- and the recovery of the compound having the formula (VIII).
